# EUROPEAN PATENT APPLICATION

(11) **EP 4 516 785 A1**
(43) Date of publication of application: **05.03.2025**
(21) Application number: 23794677.7
(22) Date of filing: 02.02.2023
(51) Int. Cl.: C07D 401/04, C07D 401/14, C07D 405/14, C07D 417/14, C07D 409/14, A61K 31/4545, A61K 31/454, A61P 35/00, A61P 35/02

(54) **HALOGEN-SUBSTITUTED ISOINDOLINE COMPOUND AND USE THEREOF**

(30) Priority: 29.04.2022 CN 202210493924
(71) Applicant: Chengdu Fendi Pharmaceutical Co., Ltd., Chengdu, Sichuan 610200 (CN)
(72) Inventor: HU, Wei, Chengdu, Sichuan 610200 (CN); WANG, Liqiang, Chengdu, Sichuan 610200 (CN); CAI, Xin, Chengdu, Sichuan 610200 (CN)
(74) Representative: Gille Hrabal Partnerschaftsgesellschaft mbB Patentanwälte
(86) International application number: PCT/CN2023/074286
(87) International publication number: WO 2023/207241

(57) **Abstract**

The present invention relates to a halogen-substituted isoindoline compound represented by formula I below and use thereof. The compound of the present invention has significantly better anti-tumor activity, and can be prepared into an oral dosage form which is more convenient, safe, and economical. Therefore, the utilization value of the compound is remarkably improved compared with that of an existing compound.

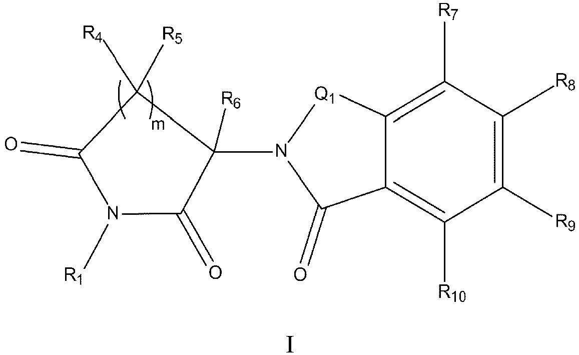

## Description

The present application claims priority to the prior Patent Application No. 2022104939244 entitled "HALOGEN-SUBSTITUTED ISOINDOLINE COMPOUND AND USE THEREOF" filed with China National Intellectual Property Administration on April 29, 2022, the content of which is incorporated herein by reference in its entirety.

### TECHNICAL FIELD

The present disclosure relates to the field of pharmaceutical technology, and particularly, to a halogen-substituted isoindoline compound and use thereof.

### BACKGROUND

The mutation, improper expression, allosterism, and functional abnormality of proteins cause a number of diseases. Protein synthesis and degradation are tightly regulated processes, both spatially and temporally, during cell growth and proliferation. Dysregulation of such processes may lead to uncontrolled growth, proliferation, and migration of cells, leading to the development of diseases such as cancer, aging, viral infections, etc.

The translation termination factor GSPT1 (eRF3a) is closely related to diseases such as cancer, and can mediate the recognition of a stop codon and promote the release of a nascent peptide from a ribosome. In addition to the translation termination functionality, GSPT1 is also involved in several other key cellular processes, such as cell cycle regulation, cytoskeletal organization, and apoptosis. GSPT1 is the carcinogenic driver for a variety of cancers, including hematological tumor, breast cancer, liver caner, gastric cancer, and prostate cancer, among others. In addition, GSPT1 is significantly upregulated in cancer tissues and cell lines. Its high expression is positively correlated with the tumor size, and its overexpression can promote the proliferation and migration of cancer cells. For example, GSPT1 depletion can effectively inhibit proliferation and migration of cancer cell, and induce the apoptosis of invasive colon cancer cells *in vitro,* and inhibit the tumorigenicity of HCT116 colon cancer cells *in vivo.* Moreover, recent studies have shown that the down-regulation of GSPT1 is also therapeutically effective in the treatment of cystic fibrosis and Lassa virus and Ebola virus infections.

The Ikaros zinc finger (IKZF) family of transcription factors are regulators of the development and differentiation of hematopoietic cells and many immune cells including CD4+ T cells. The IKZF family consists of five members: Ikaros (encoded by IKZF1), Helios (IKZF2), Aiolos (IKZF3), Eos (IKZF4), and Pegasus (IKZF5). Evidence that the Ikaros family is associated with cancer progression was primarily found in hematopoietic malignancies, suggesting the association of Ikaros dysfunction with the development of chronic lymphocytic leukemia. In recent years, it has been found that Ikaros was a major tumor suppressor involved in human B-cell acute lymphocytic leukemia, and also involved in the differentiation and function of individual T helper cells. Aiolos plays a critical role in B and T cell maturation. Elevated expression levels of Aiolos can promote cell survival by regulating Bcl2 family members in chronic lymphocytic leukemia, and have been detected in follicular central cell lymphoma. Aiolos has a synergic effect with Blimp-1 in regulating the survival of multiple myeloma cells. Moreover, in solid tumors, the overexpression of Aiolos can also promote the epithelial-mesenchymal transition and cancer stem cell nature of lung cancer cells. Currently, protein regulatory drugs based on GSPT1 or the like have not yet been approved, and it has not been reported that halogen-substituted isoindoline compounds are used as small-molecule regulators for protein such as GSPT1 in the prior art. This kind of protein regulatory drug with the most advanced development progress is CC-90009 developed by Celegen, which is proposed for intravenous administration and is in its Phase I trial. Oral administration is convenient, safe, and cost-efficient as compared with injection, and thus is the most commonly used route of administration. In addition, minor changes in the molecular structure of protein regulators may result in dramatic changes in the metabolic and biological activity of drugs. For example, a change of one atom in a molecular glue or PROTAC molecule may result in the loss of degradative activity against a particular protein and biological activity against tumors. Thus, regulators of different chemical structures may improve the efficacy and safety of drugs.

### SUMMARY

In order to solve the above technical problems, the present disclosure provides a compound of formula I, or a tautomer, a stereoisomer, a hydrate, a solvate, or a pharmaceutically acceptable salt thereof: 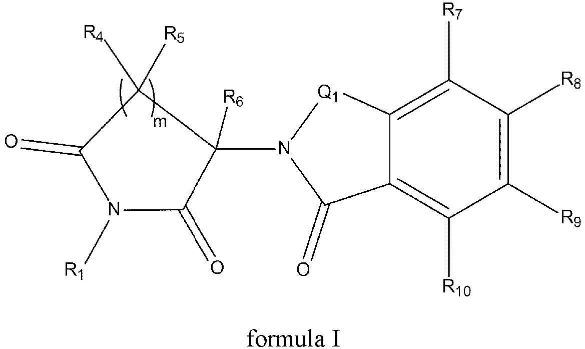 wherein R₁ is selected from hydrogen, deuterium, C₁₋₁₂ alkyl, 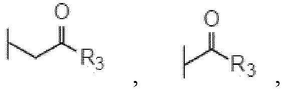 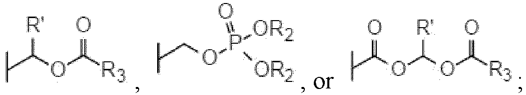
R₂ are identical or different and are each independently selected from hydrogen and the following groups unsubstituted or optionally substituted with one, two, or more Ra: C₁₋₁₂ alkyl, C₆-₂₀ aryl, 5-to 20-membered heteroaryl, C₃₋₂₀ cycloalkyl, and 3- to 20-membered heterocyclyl;
R' and R₃ are identical or different and are each independently selected from hydrogen and the following groups unsubstituted or optionally substituted with one, two, or more Rb: C₁₋₁₂ alkyl, C₁₋₁₂ alkoxy, C₆-₂₀ aryl, 5- to 20-membered heteroaryl, C₃₋₂₀ cycloalkyl, and 3- to 20-membered heterocyclyl;
R₄ and R₅ are identical or different and are each independently selected from hydrogen, deuterium, and halogen;
m is 1, 2, or 3;
R₆ is selected from hydrogen, deuterium, halogen, and the following groups unsubstituted or optionally substituted with one, two, or more Rc: C₁₋₁₂ alkyl and C₁₋₁₂ alkoxy;
Q₁ is -CH₂-, -CD₂-, -C(O)-, or -C(S)-;
R₇, R₈, R₉, and R₁₀ are identical or different and are each independently selected from hydrogen, halogen, deuterium, CN, C₁₋₁₂ alkyl, -CH₂-NHBoc, -CH₂-NH₂, -CH₂-NHCO-CF₂-R₁₁, -CH₂-NHCO-R₁₂, -CH₂-NHCO-Y-R₁₃, -CH₂-NH-Y-R₁₄, and -CH₂-NHCONH-R₁₂, wherein hydrogen(s) on methylene of -CH₂-NHBoc, -CH₂-NH₂, -CH₂-NHCO-CF₂-R₁₁, -CH₂-NHCO-R₁₂, -CH₂-NHCONH-R₁₂, -CH₂-NHCO-Y-R₁₃, and -CH₂-NH-Y-R₁₄ is(are) optionally substituted with 1 or 2 deuteriums;
R₁₁, R₁₂, R₁₃, and R₁₄ are identical or different and are each independently selected from the following groups unsubstituted or optionally substituted with one, two, or more Rs: C₆-₂₀ aryl, 5- to 20-membered heteroaryl, C₃₋₂₀ cycloalkyl, 3- to 20-membered heterocyclyl, C₂₋₁₂ alkenyl, C₂₋₁₂ alkynyl, C₁₋₁₂ alkyl, -P(C₆-₂₀ aryl)₂, -N(C₁₋₁₂ alkyl)₂, -COC₁₋₁₂ alkyl, -C₁₋₁₂ alkyl-N(C₁₋₁₂ alkyl)₂, C₆-₂₀ aryl fused with C₃₋₂₀ cycloalkyl, and spirocyclyl formed by C₃₋₂₀ cycloalkyl and C₃₋₂₀ cycloalkyl;
Y is selected from C₁₋₁₂ alkylene, C₃₋₂₀ cycloalkylene, C₂₋₁₂ alkenylene, C₂₋₁₂ alkynylene, -CH₂NH-, -CH₂NHCO-, -CH₂NHCO-, -CH₂O-, -C₂H₄O-, -CH₂S-, 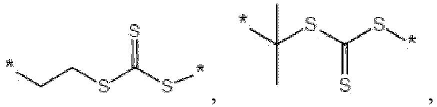 and 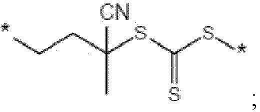
Ra, Rb, Rc, and Rs are identical or different and are each independently selected from halogen, amino, hydroxyl, C₁₋₁₂ alkyl, C₁₋₁₂ alkoxy, halogenated C₁₋₁₂ alkyl, CN, C₃₋₂₀ cycloalkyl, -N(C₁₋₁₂ alkyl)₂, deuterium, C₂₋₁₂ alkenyl, C₂₋₁₂ alkynyl, -COC₁₋₁₂ alkyl, -COC₆-₂₀ aryl, C₁₋₁₂ alkylthio, sulfhydryl, =O, -C₁₋₁₂ alkylhydroxy, 3- to 20-membered heterocyclyl, -SO₂C₁₋₁₂ alkyl, -SO₂NH₂, C₆-₂₀ aryl, -OC₆-₂₀ aryl, -C₁₋₁₂ alkyl-C₆-₂₀ aryl, -C₁₋₁₂ alkylamino, -C₆-₂₀ aryl-C₁₋₁₂ alkylamino, -C₆-₂₀ arylamino, -OC₁₋₁₂ alkyl-C₆-₂₀ aryl, -C₆-₂₀ arylhydroxy, and -C₆-₂₀ aryl-C₁₋₁₂ alkylhydroxy.

According to an embodiment of the present disclosure, R₁ is selected from hydrogen, C₁₋₆ alkyl, -C₁₋₆ alkyl-COOC₁₋₆ alkyl, -COOC₁₋₆ alkyl, and -C₁₋₆ alkyl-OCOOC₁₋₆ alkyl;
R₄, R₅, and R₆ are hydrogen; m is 1, 2, or 3; Q₁ is -CH₂- or -C(O)-;
R₇, R₈, R₉, and R₁₀ are identical or different and are each independently selected from hydrogen, deuterium, halogen, CN, C₁₋₆ alkyl, -CH₂-NHBoc, -CH₂-NH₂, -CH₂-NHCO-CF₂-R₁₁, -CH₂-NHCO-R₁₂, -CH₂-NHCONH-R₁₂, -CH₂-NHCO-Y-R₁₃, and -CH₂-NH-Y-R₁₄, wherein hydrogen(s) on CH₂ of -CH₂-NHBoc, -CH₂-NH₂, -CH₂-NHCO-CF₂-R₁₁, -CH₂-NHCO-R₁₂, -CH₂-NHCONH-R₁₂, -CH₂-NHCO-Y-R₁₃, and -CH₂-NH-Y-R₁₄ is(are) optionally substituted with 1 or 2 deuteriums;
R₁₁, R₁₂, R₁₃, and R₁₄ are identical or different and are each independently selected from the following groups unsubstituted or optionally substituted with one, two, or more Rs: C₆-₁₂ aryl, 5- to 12-membered heteroaryl, C₃₋₁₂ cycloalkyl, 3- to 12-membered heterocyclyl, C₂₋₆ alkenyl, C₂₋₆ alkynyl, C₁₋₆ alkyl, -P(C₆-₂₀ aryl)₂, -N(C₁₋₆ alkyl)₂, -COC₁₋₆ alkyl, -C₁₋₆ alkyl-N(C₁₋₆ alkyl)₂, C₆-₁₂ aryl fused with C₃₋₁₂ cycloalkyl, and spirocyclyl formed by C₃₋₁₂ cycloalkyl and C₃₋₁₂ cycloalkyl;
Y is selected from C₁₋₆ alkylene, C₃₋₁₂ cycloalkylene, C₂₋₆ alkenylene, C₂₋₆ alkynylene, -CH₂NH-, - CH₂NHCO-, -CH₂NHCO-, -CH₂O-, -C₂H₄O-, -CH₂S-, 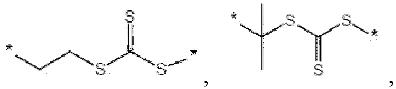 and 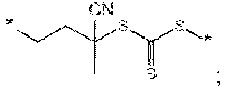
Rs are identical or different and are each independently selected from halogen, amino, hydroxyl, C₁₋₆ alkyl, C₁₋₆ alkoxy, halogenated C₁₋₆ alkyl, CN, C₃₋₁₂ cycloalkyl, -N(C₁₋₆ alkyl)₂, deuterium, C₂₋₆ alkenyl, C₂₋₆ alkynyl, -COC₁₋₆ alkyl, -COC₆-₁₂ aryl, C₁₋₆ alkylthio, sulfhydryl, =O, -C₁₋₆ alkylhydroxy, 3- to 12-membered heterocyclyl, -SO₂C₁₋₆ alkyl, -SO₂NH₂, C₆-₁₂ aryl, -OC₆-₁₂ aryl, - C₁₋₆ alkyl-C₆-₁₂ aryl, -C₁₋₆ alkylamino, -C₆-₁₂ aryl-C₁₋₆ alkylamino, -C₆-₁₂ arylamino, -OC₁₋₆ alkyl-C₆-₁₂ aryl, -C₆-₁₂ arylhydroxy, and -C₆-₁₂ aryl-C₁₋₆ alkylhydroxy.

In one embodiment, the compound of formula I has the structure of formula I-1 below: 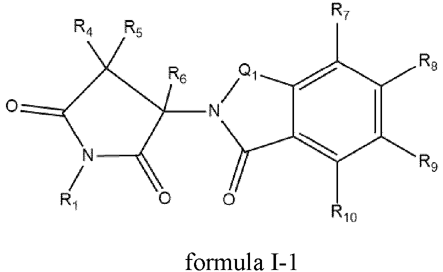 wherein, R₁, R₄, R₅, R₆, R₇, R₈, R₉, R₁₀, and Q₁ have the definitions as described above.

In one embodiment, the compound of formula I has the structure of formula I-2 below: 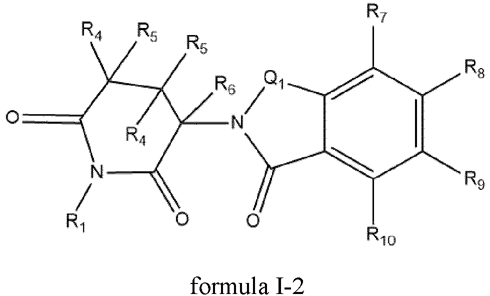 wherein, R₁, R₄, R₅, R₆, R₇, R₈, R₉, R₁₀, and Q₁ have the definitions as described above.

In one embodiment, the compound of formula I has the structure of formula I-3 below: 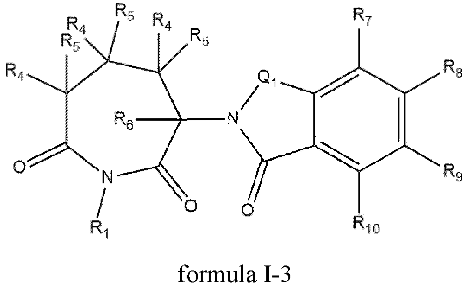 wherein, R₁, R₄, R₅, R₆, R₇, R₈, R₉, R₁₀, and Q₁ have the definitions as described above.

According to an embodiment of the present disclosure, the compound of formula I has the structure of formula Ia below: 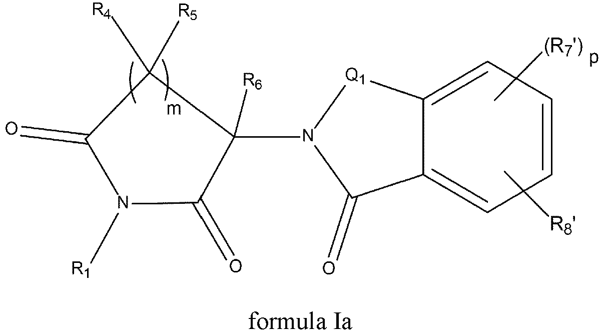 wherein, in formula Ia,
R₁ is selected from hydrogen, -C₁₋₆ alkyl, -C₁₋₆ alkyl-COOC₁₋₆ alkyl, -COOC₁₋₆ alkyl, and -C₁₋₆ alkyl-OCOOC₁₋₆ alkyl;
R₄, R₅, and R₆ are selected from hydrogen and deuterium;
m is 1, 2, or 3;
Q₁ is -CH₂-, -CD₂-, or -C(O)-;
R₇' is selected from hydrogen, deuterium, and halogen; p is 1, 2, or 3, with the proviso that at least one R₇' is halogen;
R₈' is selected from CN, -CH₂-NHBoc, -CH₂-NH₂, -CH₂-NHCO-CF₂-R₁₁, -CH₂-NHCO-R₁₂, -CH₂-NHCONH-R₁₂, -CH₂-NHCO-Y-R₁₃, and -CH₂-NH-Y-R₁₄, wherein H atom(s) on CH₂ of -CH₂-NHBoc, -CH₂-NH₂, -CH₂-NHCO-CF₂-R₁₁, -CH₂-NHCO-R₁₂, -CH₂-NHCONH-R₁₂, -CH₂-NHCO-Y-R₁₃, and -CH₂-NH-Y-R₁₄ is(are) optionally substituted with 1 or 2 deuteriums;
R₁₁, R₁₂, R₁₃, and R₁₄ are identical or different and are each independently selected from the following groups unsubstituted or optionally substituted with one, two, or more Rs: C₆-₁₂ aryl, 5- to 12-membered heteroaryl, C₃₋₁₂ cycloalkyl, 3- to 12-membered heterocyclyl, C₂₋₆ alkenyl, C₂₋₆ alkynyl, C₁₋₆ alkyl, -P(C₆-₂₀ aryl)₂, -N(C₁₋₆ alkyl)₂, -COC₁₋₆ alkyl, -C₁₋₆ alkyl-N(C₁₋₆ alkyl)₂, C₆-₁₂ aryl fused with C₃₋₁₂ cycloalkyl, and spirocyclyl formed by C₃₋₁₂ cycloalkyl and C₃₋₁₂ cycloalkyl;
Y is selected from C₁₋₆ alkylene, C₃₋₁₂ cycloalkylene, C₂₋₆ alkenylene, C₂₋₆ alkynylene, -CH₂NH-, - CH₂NHCO-, -CH₂NHCO-, -CH₂O-, -C₂H₄O-, -CH₂S-, 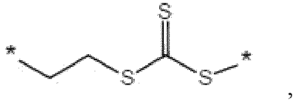 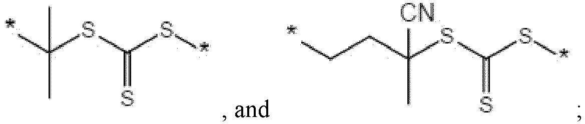
Rs are identical or different and are each independently selected from halogen, amino, hydroxyl, C₁₋₆ alkyl, C₁₋₆ alkoxy, halogenated C₁₋₆ alkyl, CN, C₃₋₁₂ cycloalkyl, -N(C₁₋₆ alkyl)₂, deuterium, C₂₋₆ alkenyl, C₂₋₆ alkynyl, -COC₁₋₆ alkyl, -COC₆-₁₂ aryl, C₁₋₆ alkylthio, sulfhydryl, =O, -C₁₋₆ alkylhydroxy, 3- to 12-membered heterocyclyl, -SO₂C₁₋₆ alkyl, -SO₂NH₂, C₆-₁₂ aryl, -OC₆-₁₂ aryl, - C₁₋₆ alkyl-C₆-₁₂ aryl, -C₁₋₆ alkylamino, -C₆-₁₂ aryl-C₁₋₆ alkylamino, -C₆-₁₂ arylamino, -OC₁₋₆ alkyl-C₆-₁₂ aryl, -C₆-₁₂ arylhydroxy, and -C₆-₁₂ aryl-C₁₋₆ alkylhydroxy.

According to an embodiment of the present disclosure, at least one of R₇, R₈, R₉, and R₁₀ is a halogen. Preferably, one of R₇, R₈, R₉, and R₁₀ is a halogen, or two of R₇, R₈, R₉, and R₁₀ are halogens, or three of R₇, R₈, R₉, and R₁₀ are halogens.

In one embodiment of the present disclosure, one of R₇, R₈, R₉, and R₁₀ is F.

According to a preferred embodiment of the present disclosure, the compound of formula I has the structure of formula Ib below: 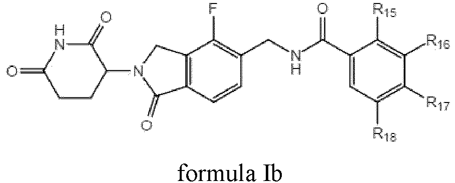 wherein, in formula Ib, R₁₅, R₁₆, R₁₇, and R₁₈ are identical or different and are each independently selected from hydrogen, deuterium, halogen, amino, hydroxyl, cyano, and the following groups unsubstituted or optionally substituted with one, two, or more Rx: C₁₋₆ alkyl, -SO₂C₁₋₆ alkyl, C₁₋₆ haloalkyl, C₆-₁₂ aryl, C₁₋₆ alkoxy, -N(C₁₋₆ alkyl)₂, C₂₋₆ alkenyl, C₂₋₆ alkynyl, C₃₋₁₂ cycloalkyl, C₁₋₆ alkylthio, -COC₁₋₆ alkyl, -COC₆-₁₂ aryl, -OC₆-₁₂ aryl, -C₁₋₆ alkyl-hydroxyl, 3- to 12-membered heterocyclyl, and -SO₂NH₂;
Rx are identical or different and are each independently selected from halogen, amino, hydroxyl, C₁₋₆ alkyl, C₁₋₆ alkoxy, halogenated C₁₋₆ alkyl, CN, C₃₋₁₂ cycloalkyl, -N(C₁₋₆ alkyl)₂, deuterium, C₂₋₆ alkenyl, C₂₋₆ alkynyl, -COC₁₋₆ alkyl, -COC₆-₁₂ aryl, C₁₋₆ alkylthio, sulfhydryl, =O, -C₁₋₆ alkylhydroxy, -C₁₋₆ alkylamino, 3- to 12-membered heterocyclyl, -SO₂C₁₋₆ alkyl, -SO₂NH₂, C₆-₁₂ aryl, -OC₆-₁₂ aryl, and -C₁₋₆ alkyl-C₆-₁₂ aryl.

In one embodiment of the present disclosure, in formula Ib, R₁₅, R₁₆, R₁₇, and R₁₈ are identical or different and are each independently selected from hydrogen, deuterium, F, Cl, methyl, amino, hydroxyl, methoxy, trifluoromethyl, cyano, phenyl, dimethylamino, ethyl, n-propyl, isopropyl, *tert-*butyl, vinyl, ethynyl, cyclopropyl, carbonylmethyl, carbonylphenyl, phenoxy, tert-butoxy, alkylthio, -SO₂NH₂, hydroxymethyl, N-tetrahydropyrrolyl, -SO₂CH₂, p-aminophenyl,

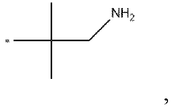

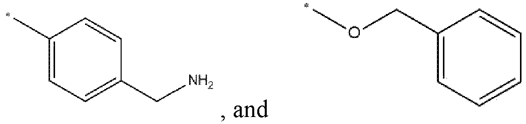

In one embodiment of the present disclosure, the compound of formula Ib is prepared by the following method: subjecting compound Im and compound In to a condensation reaction to give the compound of formula Ib, 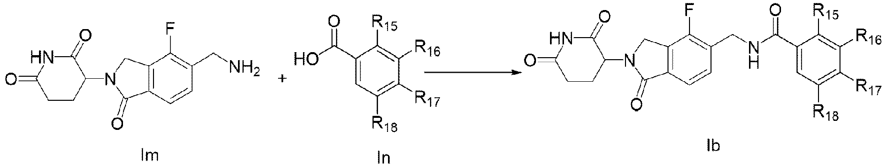 wherein R₁₅, R₁₆, R₁₇, and R₁₈ have the definitions as described above.

In one preferred embodiment, the compound of formula I has the structure of formula Ic: 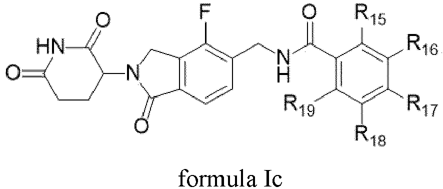
wherein, in formula Ic, R₁₅, R₁₆, R₁₇, R₁₈, and R₁₉ are identical or different and are each independently selected from hydrogen, deuterium, halogen, amino, hydroxyl, cyano, and the following groups unsubstituted or optionally substituted with one, two, or more Rx: C₁₋₆ alkyl, - SO₂C₁₋₆ alkyl, C₁₋₆ haloalkyl, C₆-₁₂ aryl, C₁₋₆ alkoxy, -N(C₁₋₆ alkyl)₂, C₂₋₆ alkenyl, C₂₋₆ alkynyl, C₃₋₁₂ cycloalkyl, C₁₋₆ alkylthio, -COC₁₋₆ alkyl, -COC₆-₁₂ aryl, -OC₆-₁₂ aryl, -C₁₋₆ alkyl-hydroxyl, 3- to 12-membered heterocyclyl, and -SO₂NH₂;
Rx are identical or different and are each independently selected from halogen, amino, hydroxyl, C₁₋₆ alkyl, C₁₋₆ alkoxy, halogenated C₁₋₆ alkyl, CN, C₃₋₁₂ cycloalkyl, -N(C₁₋₆ alkyl)₂, deuterium, C₂₋₆ alkenyl, C₂₋₆ alkynyl, -COC₁₋₆ alkyl, -COC₆-₁₂ aryl, C₁₋₆ alkylthio, sulfhydryl, =O, -C₁₋₆ alkylhydroxy, -C₁₋₆ alkylamino, 3- to 12-membered heterocyclyl, -SO₂C₁₋₆ alkyl, -SO₂NH₂, C₆-₁₂ aryl, -OC₆-₁₂ aryl, and -C₁₋₆ alkyl-C₆-₁₂ aryl, and R₁₉ is not H.

In one embodiment of the present disclosure, the compound of formula Ic is prepared by the following method: subjecting compound Ip and compound Iq to a condensation reaction to give the compound of formula Ic, 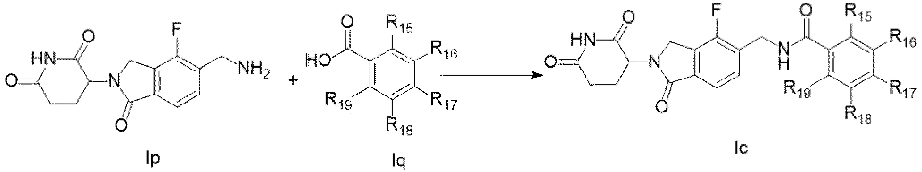 wherein R₁₅, R₁₆, R₁₇, R₁₈, and R₁₉ have the definitions as described above.

In one preferred embodiment, the compound of formula I has the structure of formula Id: 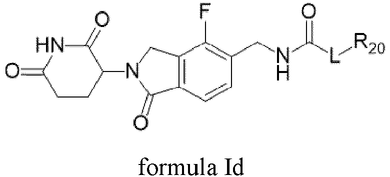
wherein, in formula Id, L is a chemical bond, C₂₋₁₂ alkynylene, C₂₋₁₂ alkenylene, NH, oxygen, sulfur, or C₁₋₁₂ alkylene, and the C₁₋₁₂ alkylene is optionally substituted with the following group: hydroxyl or amino;
R₂₀ is the following groups unsubstituted or optionally substituted with one, two, or more Ry: C₆-₁₂ aryl, 3- to 12-membered cycloalkyl, 5- to 12-membered heterocyclyl, or 5- to 12-membered heteroaryl; and
Ry is selected from deuterium, halogen, hydroxyl, amino, carboxyl, CN, C₁₋₆ alkyl, C₁₋₆ alkoxy, halogenated C₁₋₆ alkyl, C₃₋₁₂ cycloalkyl, -N(C₁₋₆ alkyl)₂, -COC₁₋₆ alkyl, -COC₆-₁₂ aryl, C₁₋₆ alkylthio, sulfhydryl, =O, -C₁₋₆ alkylhydroxy, -C₁₋₆ alkylamino, 3- to 12-membered heterocyclyl, -SO₂C₁₋₆ alkyl, -SO₂NH₂, C₆-₁₂ aryl, -OC₆-₁₂ aryl, -OC₁₋₆ alkyl-C₆-₁₂ aryl, -C₁₋₆ alkyl-C₆-₁₂ aryl, -C₁₋₆ alkyl-COOH, -C₆-₁₂ aryl-C₁₋₆ alkylamino, -C₆-₁₂ aryl-C₁₋₆ alkylhydroxy, -C₆-₁₂ arylamino, and -C₆-₁₂ aryl hydroxy.

In one preferred embodiment, in formula Id, L is a chemical bond, C₂₋₁₂ alkynylene, C₂₋₁₂ alkenylene, NH, or C₁₋₆ alkylene; the C₁₋₆ alkylene is optionally substituted with the following group: hydroxyl or amino;
R₂₀ is the following groups unsubstituted or optionally substituted with one, two, or more Ry: C₆-₁₂ aryl or 5- to 12-membered heteroaryl; and
Ry is selected from hydroxyl, amino, C₁₋₆ alkyl, C₁₋₆ alkoxy, C₆-₁₂ aryl, -OC₁₋₃ alkyl-C₆-₁₂ aryl, -C₁₋₃ alkyl-C₆-₁₂ aryl, -C₁₋₆ alkyl-NH₂, -C₁₋₆ alkylhydroxy, -C₁₋₆ alkyl-COOH, -C₆-₁₂ aryl-C₁₋₃ alkylamino, -C₆-₁₂ aryl-C₁₋₃ alkylhydroxy, -C₆-₁₂ arylamino, and -C₆-₁₂ aryl hydroxy.

In one more preferred embodiment, in formula Id, L is a chemical bond, alkynylene, NH, 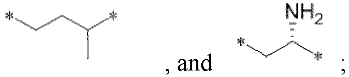
R₂₀ is phenyl; and
Ry is selected from Cl, hydroxyl, amino, tert-butyl, phenyl, p-aminophenyl, p-hydroxyphenyl, ethylamino, hydroxyethyl, *p*-hydroxyethylphenyl, *p*-ethylaminophenyl,

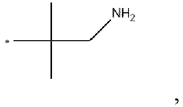

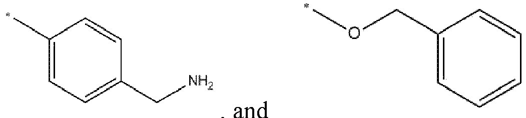

In one embodiment, the compound of formula Id is prepared by the following method: subjecting compound Ix and compound Iy to a condensation reaction to give the compound of formula Id, 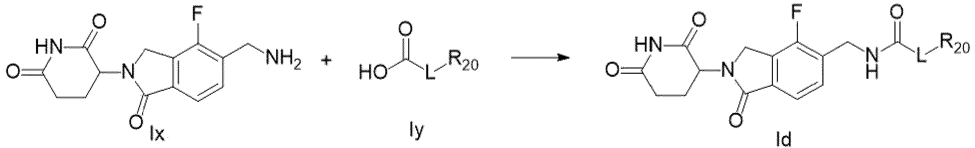 wherein R₂₀ and L have the definitions as described above.

As an example, the compound of formula I is selected from:

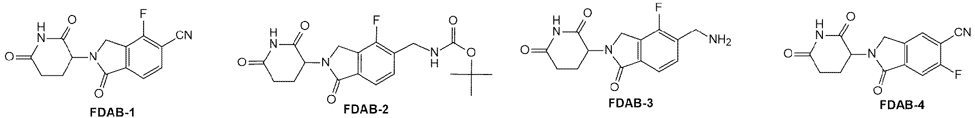

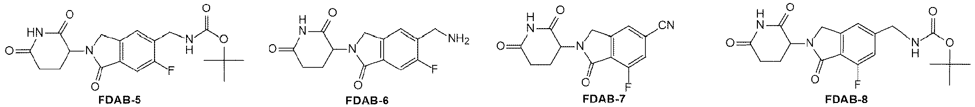

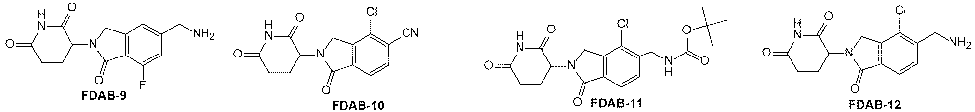

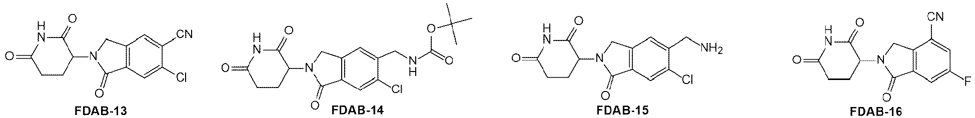

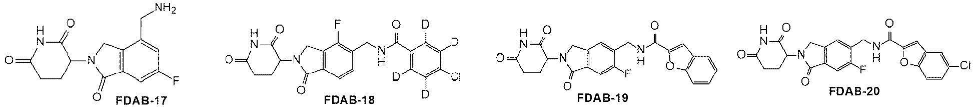

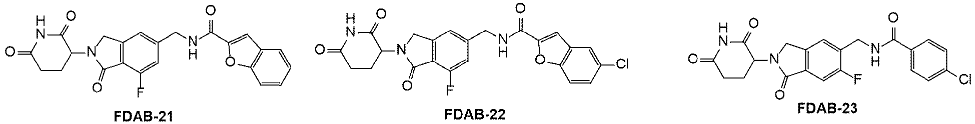

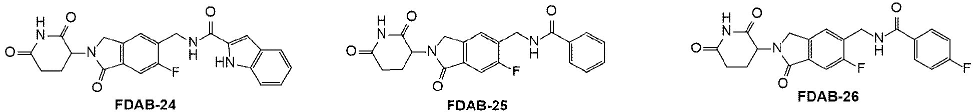

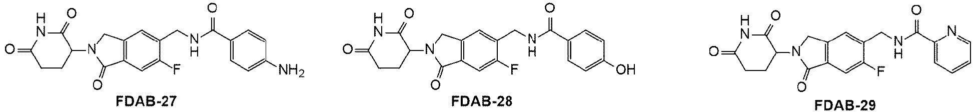

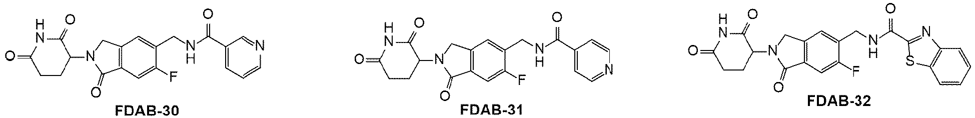

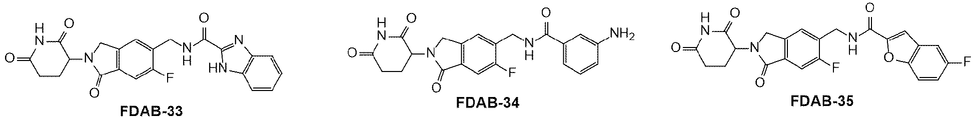

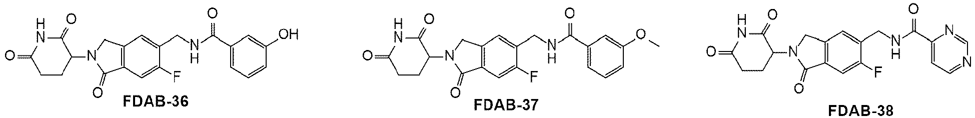

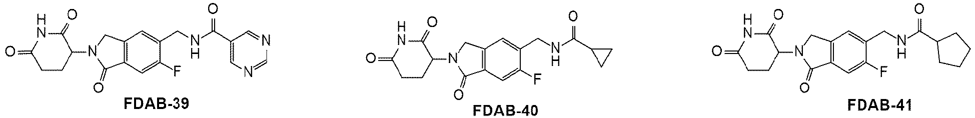

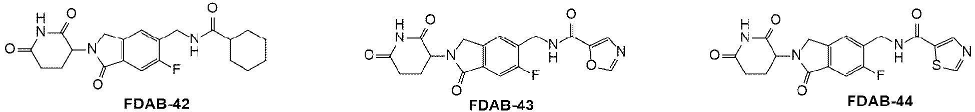

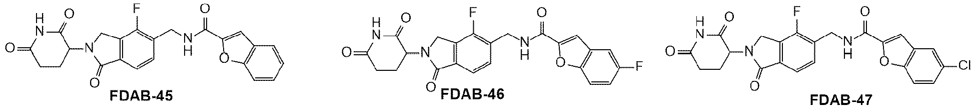

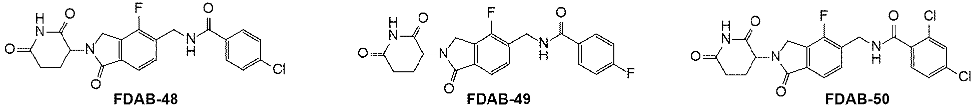

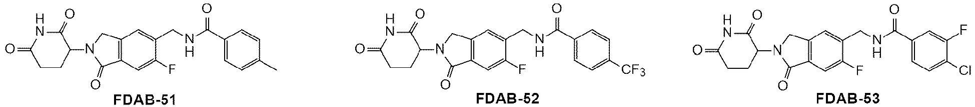

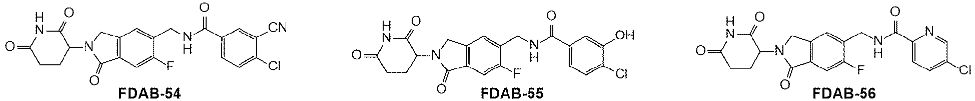

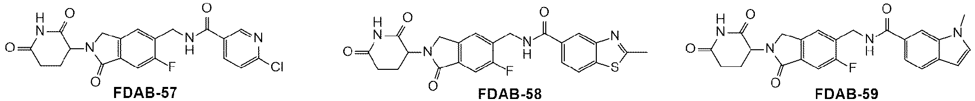

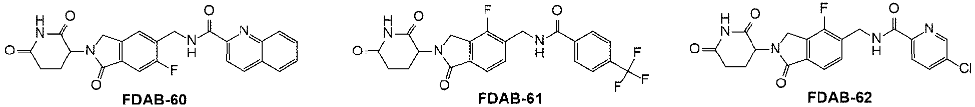

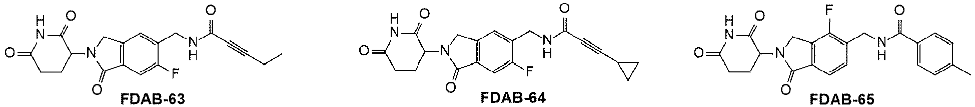

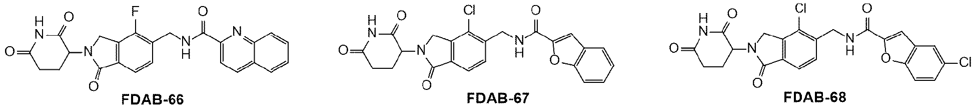

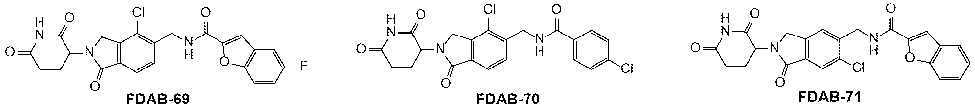

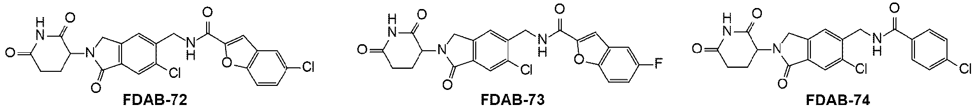

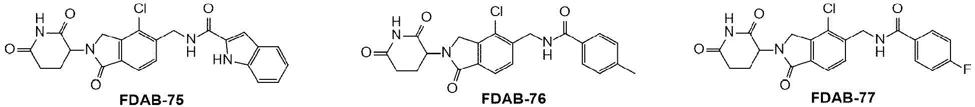

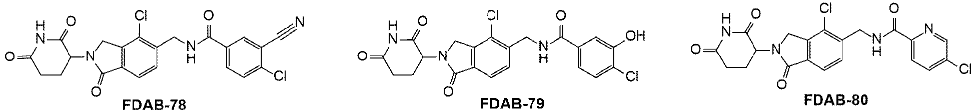

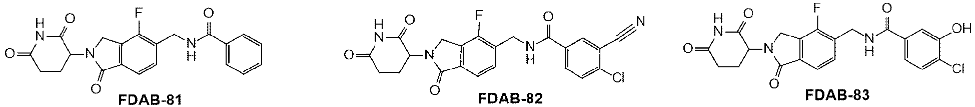

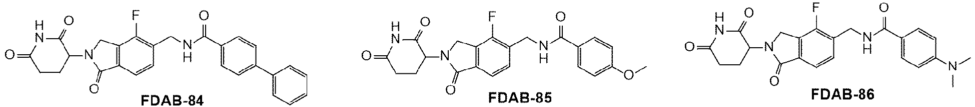

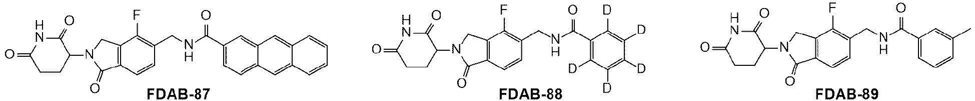

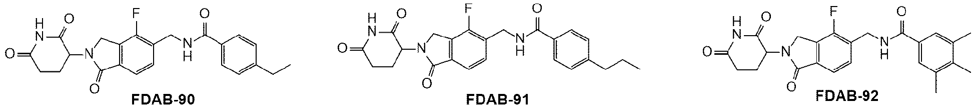

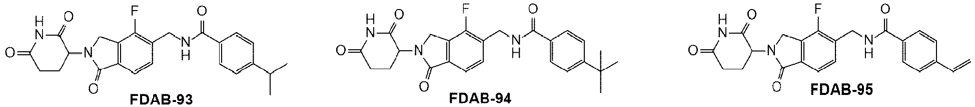

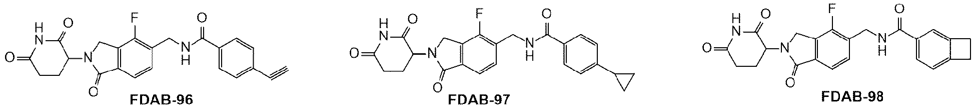

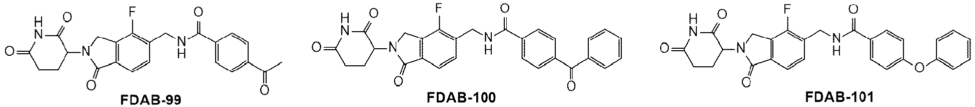

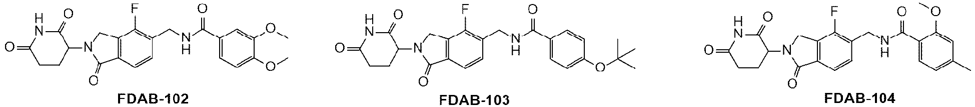

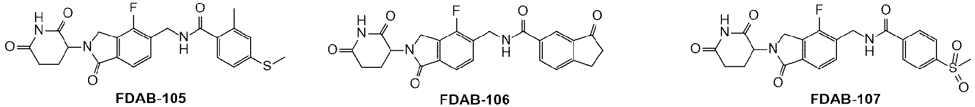

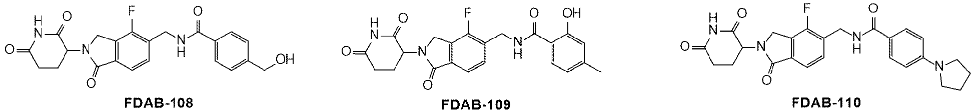

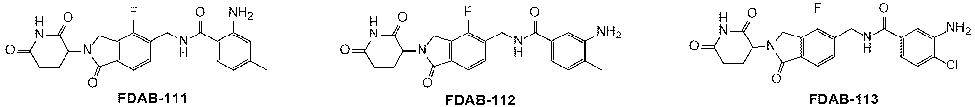

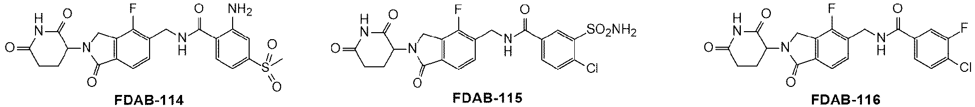

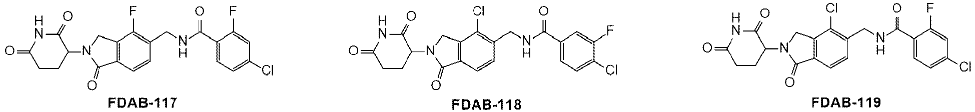

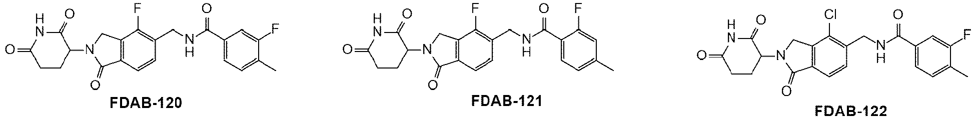

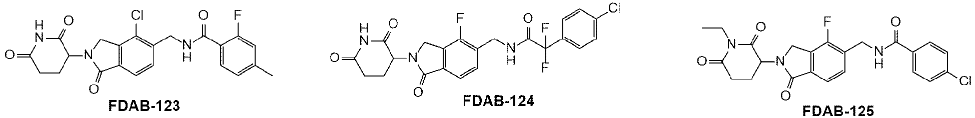

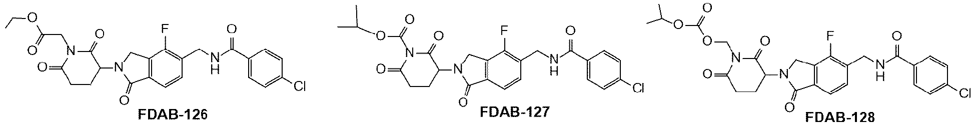

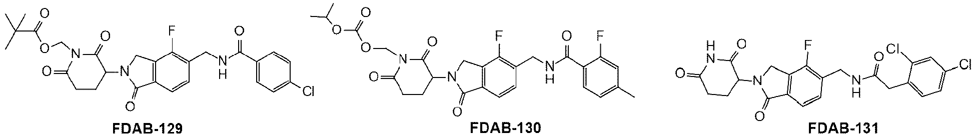

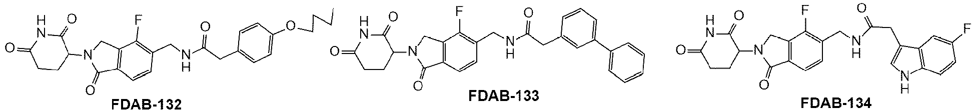

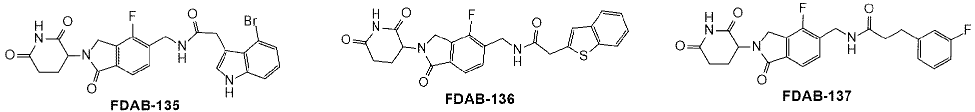

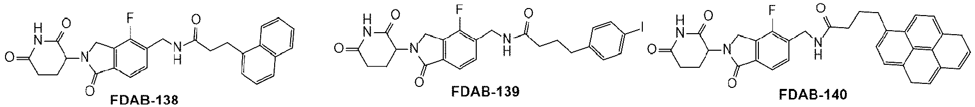

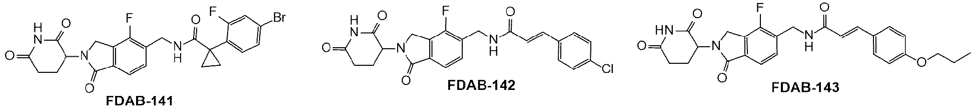

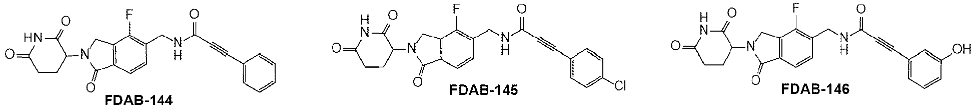

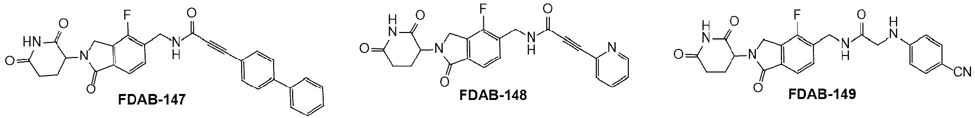

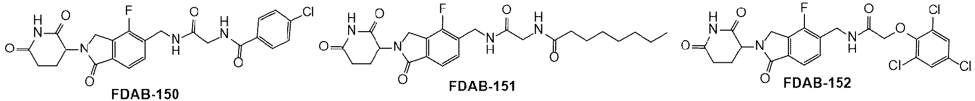

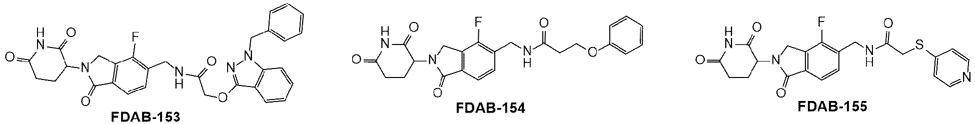

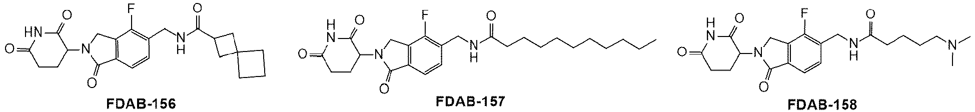

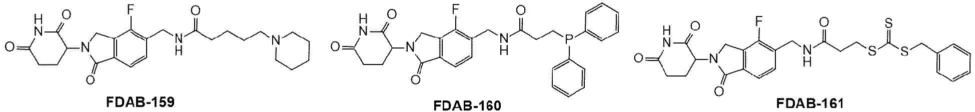

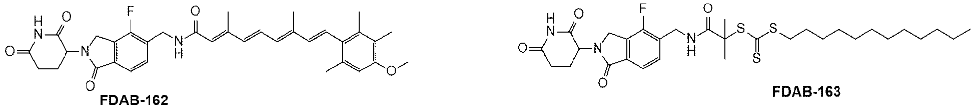

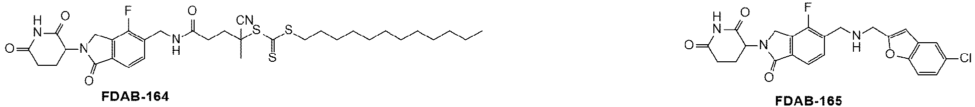

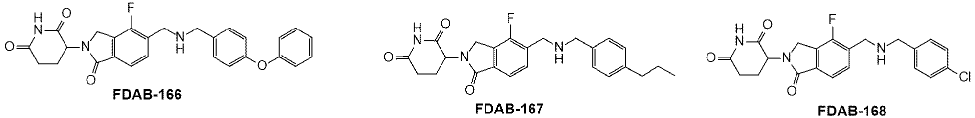

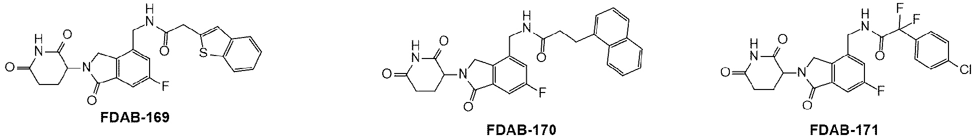

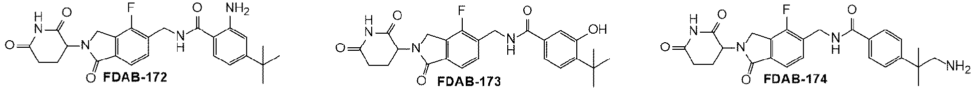

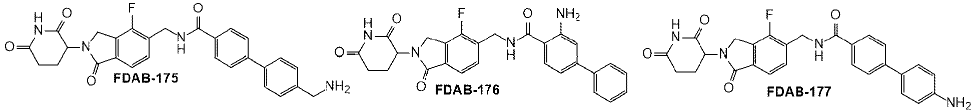

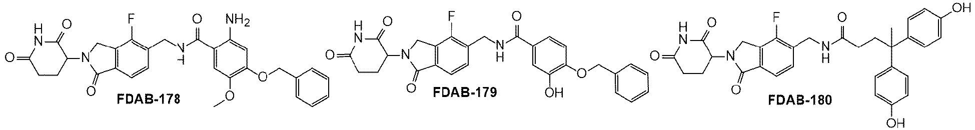

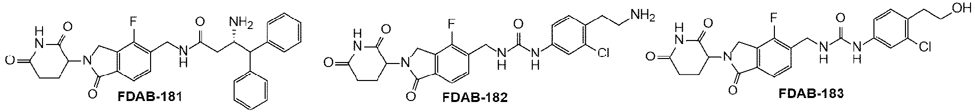

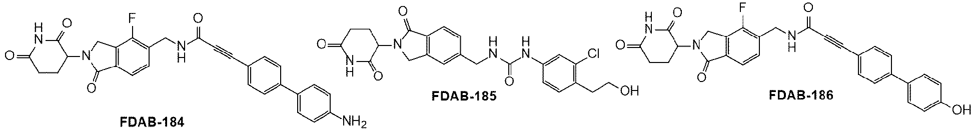

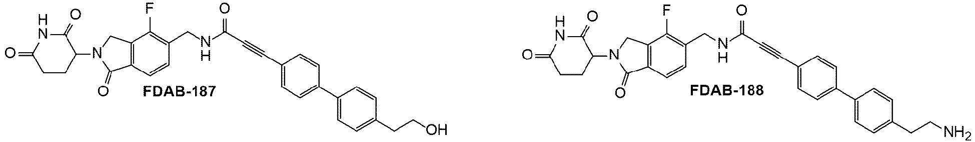

According to an embodiment of the present disclosure, the pharmaceutically acceptable salt is selected from a salt as defined below. For example, the salt includes, but is not limited to, hydrochloride, sulfate, nitrate, bisulfate, hydrobromide, acetate, oxalate, citrate, mesylate, formate, or meglumine, preferably hydrochloride.

The present disclosure further provides use of the compound of formula I, or the tautomer, the stereoisomer, the hydrate, the solvate, or the pharmaceutically acceptable salt thereof described above in preparing a medicament for treating or preventing a disease, disorder, or condition associated with a mutation, improper expression, allosterism, and functional abnormality of a protein such as GSPT1, IKZF1, IKZF2, IKZF3, CK1α, N-MYC, or C-MYC.

According to an embodiment of the present disclosure, the disease, disorder, or condition includes: myelodysplastic syndrome, multiple myeloma, mantle cell lymphoma, non-Hodgkin's lymphoma, papillary and follicular thyroid carcinoma, breast cancer, prostate cancer, chronic lymphocytic leukemia, amyloidosis, complex regional pain syndrome type I, malignant melanoma, radiculopathy, myelofibrosis, glioblastoma, gliosarcoma, malignant glioma, refractory plasmacytoma, chronic myelomonocytic leukemia, follicular lymphoma, ciliary body and chronic melanoma, iris melanoma, recurrent bilateral melanoma, extraocular extension melanoma, solid tumor, T-cell lymphoma, erythroid lymphoma, monoblastic and monocytic leukemia, myeloid leukemia (e.g., acute myeloid leukemia), central nervous system lymphoma, brain tumor, meningioma, spinal cord tumor, thyroid cancer, non-small cell lung cancer, ovarian cancer, skin cancer, renal cell carcinoma, Burkitt's lymphoma, Hodgkin's lymphoma, large cell lymphoma, diffuse large B-cell lymphoma, astrocytoma, hepatocellular carcinoma, primary macroglobulinemia, and viral infection.

The present disclosure further provides a pharmaceutical composition, comprising the compound of formula I, or the tautomer, the stereoisomer, the hydrate, the solvate, or the pharmaceutically acceptable salt thereof described above.

According to an embodiment of the present disclosure, in addition to the active ingredient compound of formula I, the pharmaceutical composition further comprises an additional therapeutic agent including but not limited to at least one of PD-1 inhibitor (e.g., nivolumab, pembrolizumab, or cemiplimab), PD-L1 inhibitor (e.g., atezolizumab, avelumab, or durvalumab), rituximab, trastuzumab, elotuzumab, ublituximab, daratumumab, atezolizumab, ibritumomab, alemtuzumab, brentuximab, cytarabine, azacitidine, anthracycline, prednisone, dexamethasone, melphalan, cladribine, fludarabine, mitoxantrone, etoposide, methotrexate, pemetrexed, topotecan, doxorubicin, cyclophosphamide, gemcitabine, dacarbazine, clarithromycin, vincristine, docetaxel, clofarabine injection, HDAC inhibitor (e.g., panobinostat, romidepsin, vorinostat, belinostat, or chidamide), FLT3 inhibitor (e.g., midostaurin, gilteritinib, or quizartinib), IDH1/2 inhibitor (e.g., ivosidenib, or enasidenib), BCL-2 inhibitor (e.g., venetoclax), proteasome inhibitor (e.g., bortezomib, carfilzomib, or ixazomib), PI3K inhibitor, BTK inhibitor (e.g., zanubrutinib, acalabrutinib, ibrutinib, tirabrutinib, or orelabrutinib), palbociclib, erythrocyte growth hormone, eltrombopag, minocycline, and CAR-T. According to a preferred embodiment of the present disclosure, in addition to the active ingredient compound of formula I, the pharmaceutical composition further comprises azacitidine or dexamethasone.

According to an embodiment of the present disclosure, the pharmaceutical composition is for use in treating or preventing a disease, disorder, or condition associated with a mutation, improper expression, allosterism, and functional abnormality of a protein such as GSPT1, IKZF1, IKZF2, IKZF3, CK1α, N-MYC, or C-MYC.

According to an embodiment of the present disclosure, the disease, disorder, or condition includes: myelodysplastic syndrome, multiple myeloma, mantle cell lymphoma, non-Hodgkin's lymphoma, papillary and follicular thyroid carcinoma, breast cancer, prostate cancer, chronic lymphocytic leukemia, amyloidosis, complex regional pain syndrome type I, malignant melanoma, radiculopathy, myelofibrosis, glioblastoma, gliosarcoma, malignant glioma, refractory plasmacytoma, chronic myelomonocytic leukemia, follicular lymphoma, ciliary body and chronic melanoma, iris melanoma, recurrent bilateral melanoma, extraocular extension melanoma, solid tumor, T-cell lymphoma, erythroid lymphoma, monoblastic and monocytic leukemia, myeloid leukemia (e.g., acute myeloid leukemia), central nervous system lymphoma, brain tumor, meningioma, spinal cord tumor, thyroid cancer, non-small cell lung cancer, ovarian cancer, skin cancer, renal cell carcinoma, Burkitt's lymphoma, Hodgkin's lymphoma, large cell lymphoma, diffuse large B-cell lymphoma, astrocytoma, hepatocellular carcinoma, primary macroglobulinemia, and viral infection.

According to an embodiment of the present disclosure, the compound of formula I or the pharmaceutical composition may be administered by oral, rectal, topical, buccal, parenteral, intramuscular, intradermal, intravenous, or transdermal administration. In one embodiment, the pharmaceutical composition is administered orally.

### Beneficial Effects:

The compound of the present disclosure has regulatory effects on proteins such as GSPT1, IKZF1, IKZF2, IKZF3, CK1α, N-MYC, or C-MYC and an anti-tumor and anti-cancer activity, particularly, a strong inhibitory activity against acute myeloid leukemia and myeloma cells. The activity advantages of the compound of the present disclosure are mainly reflected in that:
1. The inventors surprisingly found that, compared with the unsubstituted compound, the halogen-substituted compound of the present disclosure has greatly improved the activity against tumor cell proliferation, and has significantly improved the degradative effect on proteins such as GSPT1. For example, the halogen-substituted compound FDAB-48 of the present disclosure demonstrated an IC₅₀ of 10.8 nM against MV-4-11 cell proliferation and an 88% degradation of GSPT1 protein when co-incubated for 6 h with NB-4 cells, while the unsubstituted compound 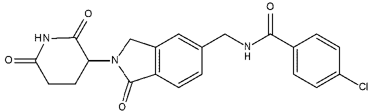 demonstrated an IC₅₀ of 102.6 nM against MV-4-11 cell proliferation and only a 65% degradation of GSPT1 protein when co-incubated for 6 h with NB-4 cells.
2. The compound of the present disclosure has almost unobservable inhibitory activity against the hERG channel (9 to 25% inhibition) at a concentration of 3 µM, while the compound CC-90009 in a clinical trial, has a significant hERG channel-inhibitory activity (66% inhibition).
3. The compound CC-90009 in a clinical trial is in an injection. The compound of the present disclosure has a significant tumor inhibitory activity in a mouse xenograft tumor model, and the compound CC-90009 has no tumor inhibitory activity when it is orally administered at the same dosage. It is demonstrated that the compound of the present disclosure can be formulated into an oral dosage form with higher convenience, better safety, and higher cost-efficiency than the compound CC-90009 (an injection).

In conclusion, the compound of the present disclosure has significantly improved anti-tumor activity, and can be formulated into an oral dosage form with higher convenience, better safety, and higher cost-efficiency, thus possessing significantly greater value compared with existing compounds.

### Definitions and Description

Unless otherwise stated, the definitions of groups and terms described in the specification and claims of the present application, including definitions thereof as examples, exemplary definitions, preferred definitions, definitions documented in tables, definitions of specific compounds in the examples, and the like, may be arbitrarily combined and incorporated with each other. The definitions of groups and the structures of the compounds in such combinations and incorporations should fall within the scope of the specification of the present application.

As used herein, the terms "comprise", "include", and/or "contain" or any variations of these terms are open-ended, i.e., include the elements recited the present disclosure, but do not exclude other unrecited elements.

As used herein, when referring to one, two, or more, the "more" shall refer to an instance greater than 2, for example, an integer of 3 or more, e.g., 3, 4, 5, 6, 7, 8, 9, or 10.

As used herein, the term "optional" or "optionally" indicates both the presence and absence of the stated feature, which means that the subsequently described event may, but does not necessarily, occur and thus includes both instances where the event occurs and where it does not. For example, the "heterocyclyl group optionally substituted with alkyl" means that the alkyl may be, but not necessarily, present, and includes instances where the heterocyclyl group is substituted with alkyl and where the heterocyclyl group is not substituted with alkyl.

In some of the substituents in the present application, the " |" and "*" are connection sites.

As used herein, the term "halogen" refers to fluorine, chlorine, bromine, and/or iodine. Accordingly, the term "halo" refers to fluoro, chloro, bromo, and/or iodo. Within the scope of the present disclosure, when an atom, a residue, a group, or a moiety is halogenated, the atom at the halogenated position may be monosubstituted, disubstituted, or polysubstituted with halogen atom(s) up to being totally substituted.

The term "C₁₋₁₂ alkyl" preferably refers to a linear or branched saturated monovalent hydrocarbyl group having 1 to 12 carbon atoms, preferably C₁₋₆ alkyl. The "C₁₋₆ alkyl" preferably refers to a linear or branched saturated monovalent hydrocarbyl group having 1, 2, 3, 4, 5, or 6 carbon atoms. The alkyl is, for example, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, etc., or an isomer thereof. Particularly, the group has 1, 2, or 3 carbon atoms ("C₁₋₃ alkyl"), such as methyl, ethyl, *n*-propyl, or isopropyl.

The term "C₂₋₁₂ alkenyl" refers to a linear or branched monovalent hydrocarbyl comprising one or more double bonds and having 2 to 12 carbon atoms, preferably "C₂₋₆ alkenyl". The "C₂₋₆ alkenyl" preferably refers to a linear or branched monovalent hydrocarbyl group comprising one or more double bonds and having 2, 3, 4, 5, or 6 carbon atoms, in particular, 2 or 3 carbon atoms ("C₂₋₃ alkenyl"). It will be appreciated that in the case where the alkenyl comprises more than one double bond, the double bonds can be separated from one another or conjugated. The alkenyl is, for example, ethenyl, allyl, (*E*)-2-methylethenyl, (*Z*)-2-methylethenyl, (*E*)-but-2-enyl, (*Z*)-but-2-enyl, (*E*)-but-1-enyl, (*Z*)-but-1-enyl, pent-4-enyl, (*E*)-pent-3-enyl, (*Z*)-pent-3-enyl, (*E*)-pent-2-enyl, (*Z*)-pent-2-enyl, (*E*)-pent-1-enyl, (*Z*)-pent-1-enyl, hex-5-enyl, (*E*)-hex-4-enyl, (*Z*)-hex-4-enyl, (*E*)-hex-3-enyl, (*Z*)-hex-3-enyl, (*E*)-hex-2-enyl, (*Z*)-hex-2-enyl, (*E*)-hex-1-enyl, (*Z*)-hex-1-enyl, isopropenyl, 2-methylprop-2-enyl, 1-methylprop-2-enyl, 2-methylprop-1-enyl, (*E*)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethylprop-1-enyl, 1-propylvinyl, or 1-isopropylvinyl.

The term "C₂₋₁₂ alkynyl" refers to a linear or branched monovalent hydrocarbyl group comprising one or more triple bonds and having 2 to 12 carbon atoms, and is preferably "C₂-C₆ alkynyl". The term "C₂-C₆ alkynyl" preferably refers to a linear or branched monovalent hydrocarbyl group comprising one or more triple bonds and having 2, 3, 4, 5, or 6 carbon atoms, in particular 2 or 3 carbon atoms ("C₂-C₃-alkynyl"). The alkynyl is, for example, ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl, or 3,3-dimethylbut-1-ynyl. In particular, the alkynyl is ethynyl, prop-1-ynyl, or prop-2-ynyl.

The term "C₃₋₂₀ cycloalkyl" refers to a saturated monovalent monocyclic or bicyclic hydrocarbon ring having 3 to 20 carbon atoms, preferably "C₃₋₁₂ cycloalkyl". The term "C₃₋₁₂ cycloalkyl" refers to a saturated monovalent monocyclic or bicyclic hydrocarbon ring having 3, 4, 5, 6, 7, 8, 9, 10, 11, or 12 carbon atoms. The C₃₋₁₂ cycloalkyl may be a monocyclic hydrocarbon group, such as cyclopropyl, cyclobutyl, cyclopentyl, cyclohexyl, cycloheptyl, cyclooctyl, cyclononyl or cyclodecyl, or a bicyclic hydrocarbon group, such as a decaline ring.

The term "3- to 20-membered heterocyclyl" refers to a saturated monovalent monocyclic or bicyclic hydrocarbon ring containing 1-5 heteroatoms independently selected from N, O, and S, preferably "3- to 12-membered heterocyclyl". The term "3- to 12-membered heterocyclyl" refers to a saturated monovalent monocyclic or bicyclic hydrocarbon ring containing 1-5, preferably 1-3, heteroatoms selected from N, O, and S. The heterocyclyl may be attached to the rest of the molecule through any one of the carbon atoms or a nitrogen atom (if present). In particular, the heterocyclyl may include, but is not limited to, 4-membered rings such as azetidinyl and oxetanyl; 5-membered rings such as tetrahydrofuranyl, dioxolyl, pyrrolidinyl, imidazolidinyl, pyrazolidinyl and pyrrolinyl; 6-membered rings such as tetrahydropyranyl, piperidyl, morpholinyl, dithianyl, thiomorpholinyl, piperazinyl and trithianyl; or 7-membered rings such as diazepanyl. Optionally, the heterocyclyl may be benzo-fused. The heterocyclyl may be bicyclic, for example, but not limited to, a 5,5-membered ring such as a hexahydrocyclopenta[c]pyrrol-2(1*H*)-yl ring, or a 5,6-membered bicyclic ring such as a hexahydropyrrolo[1,2-*a*]pyrazin-2(1*H*)-yl ring. The ring containing a nitrogen atom may be partially unsaturated, i.e., it may contain one or more double bonds, for example, but not limited to, 2,5-dihydro-1*H*-pyrrolyl, 4*H*-[1,3,4]thiadiazinyl, 4,5-dihydrooxazolyl, or 4*H*-[1,4]thiazinyl, or it may be benzo-fused, for example, but not limited to, dihydroisoquinolyl. According to the present disclosure, the heterocyclyl is non-aromatic.

The term "C₆₋₂₀ aryl" refers to an aromatic or partially aromatic monovalent monocyclic, bicyclic, or tricyclic hydrocarbon ring having 6 to 20 carbon atoms, preferably "C₆₋₁₂ aryl". The term "C₆₋₁₂ aryl" preferably refers to an aromatic or partially aromatic monovalent monocyclic, bicyclic, or tricyclic hydrocarbon ring having 6, 7, 8, 9, 10, 11, or 12 carbon atoms ("C₆₋₁₂ aryl"), in particular a ring having 6 carbon atoms ("C₆ aryl"), such as phenyl or biphenyl, a ring having 9 carbon atoms ("C₉ aryl"), such as indanyl or indenyl, a ring having 10 carbon atoms ("C₁₀ aryl"), such as tetrahydronaphthyl, dihydronaphthyl or naphthyl, a ring having 13 carbon atoms ("C₁₃ aryl"), such as fluorenyl, or a ring having 14 carbon atoms ("C₁₄ aryl"), such as anthryl.

The term "5- to 20-membered heteroaryl" refers to a monovalent aromatic monocyclic, bicyclic, or tricyclic ring system that has 5 to 20 ring atoms and contains 1-5 heteroatoms independently selected from N, O, and S, such as "5- to 12-membered heteroaryl". The term "5- to 12-membered heteroaryl" refers to a monovalent aromatic monocyclic, bicyclic, or tricyclic ring system that has 5, 6, 7, 8, 9, 10, 11, or 12 ring atoms, in particular 5, or 6, or 9, or 10 carbon atoms, contains 1-5, preferably 1-3, heteroatoms independently selected from N, O, and S, and may be benzo-fused in each case. In particular, the heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, thia-4H-pyrazolyl and the like and benzo derivatives thereof, such as benzofuranyl, benzothienyl, benzoxazolyl, benzoisoxazolyl, benzimidazolyl, benzotriazolyl, indazolyl, indolyl, isoindolyl, and the like; or pyridinyl, pyridazinyl, pyrimidinyl, pyrazinyl, triazinyl and the like and benzo derivatives thereof, such as quinolyl, quinazolinyl, isoquinolyl, and the like; or azocinyl, indolizinyl, purinyl and the like and benzo derivatives thereof, or cinnolinyl, phthalazinyl, quinazolinyl, quinoxalinyl, naphthyridinyl, pteridinyl, carbazolyl, acridinyl, phenazinyl, phenothiazinyl, phenoxazinyl, and the like.

Unless otherwise stated, the heterocyclyl, heteroaryl, or heteroarylene includes all possible isomeric forms thereof, e.g., position isomers thereof. Accordingly, for some illustrative, non-limiting examples, pyridinyl or pyridinylene includes pyridin-2-yl, pyridin-2-ylene, pyridin-3-yl, pyridin-3-ylene, pyridin-4-yl, and pyridin-4-ylene; thienyl or thienylene includes thien-2-yl, thien-2-ylene, thien-3-yl, and thien-3-ylene.

The above definition of the term "C₁₋₁₂ alkyl" applies similarly to other terms containing "C₁₋₁₂ alkyl", such as the terms "C₁₋₁₂ alkoxy", "halogenated C₁₋₁₂ alkyl", "-C₁₋₁₂ alkyl-COOC₁₋₁₂ alkyl", "-COOC₁₋₁₂ alkyl" or "-C₁₋₁₂ alkyl-OCOOC₁₋₁₂", "-C₁₋₆ alkyl-C₆-₁₂ aryl", "-C₆-₁₂ aryl-C₁₋₆ alkylamino", "-C₆-₁₂ aryl-C₁₋₆ alkylhydroxy", "-N(C₁₋₆ alkyl)₂", "-C₁₋₆ alkylhydroxy", etc. In other words, in the present application, when referring to a group containing a "C₁₋₆ alkyl" substituent, the C₁₋₆ alkyl has the definition described above. Similarly, the "C₆₋₁₂ aryl", "C₃₋₂₀ cycloalkyl", "5- to 20-membered heteroaryl", "3- to 20-membered heterocyclyl", and the like have the same definitions as used herein.

The term "C₁₋₁₂ haloalkyl" has the same meaning as the "halogenated C₁₋₁₂ alkyl", both referring to the substituent formed by substituting the "C₁₋₁₂ alkyl" described above with a halogen.

The term "C₆₋₂₀ aryl fused with C₃₋₂₀ cycloalkyl" refers to a group constituting of the C₆-₂₀ aryl and the ₃₋₂₀ cycloalkyl described above by sharing at least 2 carbon atoms.

The term "spirocyclyl formed by C₃₋₂₀ cycloalkyl and C₃₋₂₀ cycloalkyl" refers to a spirocyclyl formed by two C₃₋₂₀ cycloalkyl groups by sharing 1 carbon atom, in which the C₃₋₂₀ cycloalkyl is defined above.

The term "C₁₋₁₂ alkylthio" refers to a -SC₁₋₁₂ alkyl, wherein the C₁₋₁₂ alkyl is defined above.

The term "Boc" is *tert*-butoxycarbonyl.

As used herein, the "pharmaceutically acceptable salt" refers to a salt of the compound of the present disclosure that is safe and effective for use in the body of a mammal and possesses requisite biological activity.

The pharmaceutically acceptable salt includes acid addition salts of the compound of the present disclosure having a nitrogen atom in the chain or ring and sufficient basicity. In addition, the basic nitrogen-containing groups may be quaternized with the following agents: lower alkyl halides such as methyl, ethyl, propyl and butyl chlorides, bromides, and iodides; dialkyl sulfates such as dimethyl sulfate, diethyl sulfate, dibutyl sulfate, and dipentyl sulfate; long chain halides such as decyl, lauryl, myristyl and stearyl chlorides, bromides, and iodides; and aralkyl halides such as benzyl and phenethyl bromides. As an example, the physiologically/pharmaceutically acceptable salt includes, but is limited to, hydrochloride, sulfate, nitrate, bisulfate, hydrobromide, acetate, oxalate, citrate, mesylate, formate, meglumine, and the like.

Since the compound of the present disclosure may have a plurality of salt-forming sites, the physiologically/pharmaceutically acceptable salt includes not only a salt formed at 1 salt-forming site of the compound of the present disclosure but also a salt formed at 2, 3 or all of the salt-forming sites thereof. For this purpose, in the physiologically/pharmaceutically acceptable salt, the molar ratio of the compound of formula (I) to a radical ion of an acid (anion) or a cation of a base required for salt formation may vary within a wide range, and may be, for example, 4:1-1:4, such as 3:1, 2:1, 1:1, 1:2, 1:3, or the like.

### DETAILED DESCRIPTION

The technical solutions of the present disclosure will be further described in detail with reference to the following specific examples. It will be appreciated that the following examples are merely exemplary illustration and explanation of the present disclosure and should not be construed as limiting the claimed scope of the present disclosure. All techniques implemented based on the content described above of the present disclosure are encompassed within the claimed scope of the present disclosure.

Unless otherwise stated, the starting materials and reagents used in the following examples are all commercially available products or can be prepared by known methods. The structures of the compounds were determined by nuclear magnetic resonance (NMR) on the Bruker Avance-400 system or by mass spectrometry (MS) on the Agilent Technologies 6110 liquid chromatography-mass spectrometry (LC-MS) system using an ESI ion source.

### Example 1: Synthesis of compound FDAB-1

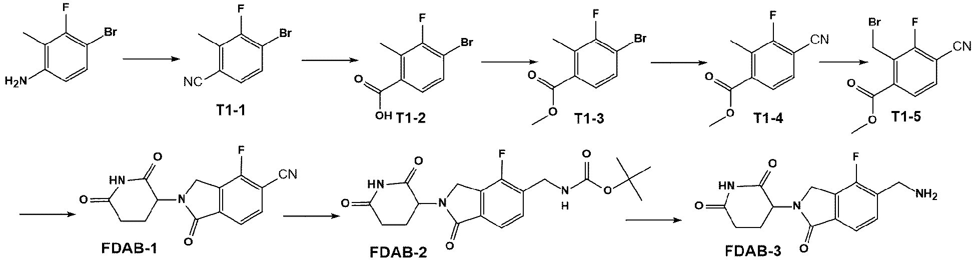

Synthesis of **T1-1:** Cuprous cyanide (5.28 g, 59.0 mmol) and tert-butyl nitrite (14 g, 136.1 mmol) were dissolved in 100 mL of dimethyl sulfoxide. The reaction system was warmed to 60 °C and reacted for about 30 min at 60 °C while stirring. A solution of 4-bromo-3-fluoro-2-methylaniline (10 g, 49.0 mmol) in dimethyl sulfoxide (20 mL) was slowly added dropwise over about 30 min. The reaction was then stirred at 60 °C for about 1 h, until LCMS indicated the depletion of the starting materials. The reaction solution was cooled to room temperature. The reaction was quenched by dropwise adding 6 M hydrochloric acid solution, then extracted with ethyl acetate (2 × 100 mL) and phase separated. The organic phases were combined, backwashed with saturated brine, dried over anhydrous sodium sulfate, filtered, and concentrated at reduced pressure to give a crude product. The crude product was subjected to column chromatography (ethyl acetate/petroleum ether = 1/20-1/10) to give a pale yellow oily liquid **T1-1** (4.5 g, 43% yield).

Synthesis of **T1-2:** Intermediate **T1-1** (4.5 g, 21.0 mmol) was mixed with aqueous sodium hydroxide solution (5 M, 50 mL). The mixed solution system was warmed to 100 °C and stirred for about 16 h, until LCMS indicated the depletion of **T1-1.** The reaction solution was cooled to room temperature. 6 M hydrochloric acid solution was dropwise added to the reaction solution in an ice bath to adjust the reaction solution to an acidic pH of about 1. During this period, a large amount of solid was precipitated in the reaction solution and filtered. The filter cake was washed twice with distilled water and then dried to give a pale yellow solid **T1-2** (2.4 g, 50% yield).

Synthesis of **T1-3:** Intermediate **T1-2** (4.5 g, 19.3 mmol) and potassium carbonate (5.3 g, 38.6 mmol) were dissolved in 50 mL of *N,N-*dimethylformamide, and iodomethane (4.1 g, 29.0 mmol) was added slowly at room temperature. The reaction system was then stirred at room temperature for about 1 h, until LCMS indicated the depletion of the starting materials **T1-2**. The reaction solution was quenched by the addition of ice water. The organic phase was extracted with ethyl acetate (2 × 100 mL) and separated. The organic phases were combined, backwashed with saturated brine, dried over anhydrous sodium sulfate, filtered, and concentrated at reduced pressure to give a crude product. The crude product was subjected to column chromatography (ethyl acetate/petroleum ether = 1/50-1/20) to give a pale yellow oily liquid **T1-3** (4.3 g, 90% yield).

Synthesis of **T1-4:** Intermediate **T1-3** (9.8 g, 39.7 mmol) and cuprous cyanide (5.34 g, 59.7 mmol) were dissolved in 60 mL of *N*-methylpyrrolidinone. The mixed system was warmed to 180 °C and stirred at this temperature for about 2 h, until LCMS indicated the depletion of the starting materials. The mixed system was cooled to room temperature and filtered. The filtrate was diluted with 100 mL of water, extracted with ethyl acetate (2 × 100 mL) and phase separated. The organic phases were combined, backwashed with saturated brine, dried over anhydrous sodium sulfate, filtered, and concentrated at reduced pressure to give a crude product. The crude product was subjected to column chromatography (ethyl acetate/petroleum ether = 1/20-1/10) to give an off-white solid **T1-4** (4.6 g, 60% yield).

Synthesis of **T1-5:** Intermediate **T1-4** (3.9 g, 20.0 mmol) and N-bromosuccinimide (5.4 g, 30.0 mmol) were dissolved in 50 mL chloroform, and warmed to 65 °C. A catalytic amount of azobisisobutyronitrile (0.1 g) was added, and the reaction system was stirred at this temperature for about 4 h, until LCMS indicated the depletion of the starting materials. The reaction solution was cooled to room temperature and filtered at reduced pressure. The filter cake was washed with 30 mL of dichloromethane, and the filtrate was diluted with 30 mL of water and extracted with dichloromethane (2 × 30 mL) and phase separated. The organic phases were combined, backwashed with saturated brine, dried over anhydrous sodium sulfate, filtered, and concentrated at reduced pressure to give a crude product. The crude product was subjected to column chromatography (ethyl acetate/petroleum ether = 1/20-1/10) to give a pale yellow liquid **T1-5** (3.3 g, 60% yield).

Intermediate **T1-5** (5.4 g, 20.0 mmol) and 3-aminopiperidine-2,6-dione hydrochloride (3.3 g, 20.0 mmol) were dissolved in 60 mL of *N,N-*dimethylformamide, and diisopropylethylamine (7.7 g, 60.0 mmol) was added at room temperature. The mixed system was warmed to 75 °C and stirred at this temperature for about 4 h, until LCMS indicated the depletion of **T1-5.** The reaction solution was diluted with 100 mL of water, and extracted with a mixture of methanol/dichloromethane = 1:10 (3 × 100 mL) and phase separated. The organic phases were combined, dried over anhydrous sodium sulfate, filtered, and concentrated at reduced pressure to give a crude product. The crude product was subjected to column chromatography (methanol/dichloromethane = 1/100-1/30) to give an off-white solid **FDAB-1** (3.5 g, 60% yield).

### Example 2: Synthesis of compound FDAB-2

**FDAB-1** (5.7 g, 20.0 mmol) and di-*tert*-butyl dicarbonate (6.5 g, 30.0 mmol) were dissolved in 60 mL of a mixed solvent of tetrahydrofuran/*N,N*-dimethylformamide = 10:1, and Raney nickel (0.6 g) was added at room temperature. The mixed system was purged with hydrogen for three times, maintained in a hydrogen atmosphere at a certain pressure, warmed to 40 °C and stirred at this temperature for about 4 h, until LCMS indicated the depletion of FDAB-1. The reaction solution was cooled and filtered at reduced pressure. The filter cake was washed three times with 50 mL of dichloromethane/methanol (10/1), and the filtrates were combined and concentrated at reduced pressure. Then, the concentrated solution was added with 100 mL of water, extracted with ethyl acetate (3 × 100 mL) and phase separated. The organic phases were combined, backwashed with saturated brine, dried over anhydrous sodium sulfate, filtered, and concentrated at reduced pressure to give a crude product. The crude product was subjected to column chromatography (methanol/dichloromethane = 1/100-1/50) to give a white solid FDAB-2 (6.3 g, 81% yield).

### Example 3: Synthesis of compound FDAB-3

Intermediate **FDAB-2** (3.9 g, 10 mmol) was dissolved in 30 mL of dichloromethane, a solution of hydrochloric acid in 1,4-dioxane (1 M, 5 mL) was added at room temperature. The reaction system was warmed to 40 °C for reaction. After about 1 h of reaction, LCMS indicated the depletion of the starting material **FDAB-2.** The reaction solution was concentrated at reduced pressure to remove the excess hydrochloric acid solution and solvent. The concentrate was diluted with dichloromethane and adjusted to about pH 7 with aqueous ammonia, concentrated again, and subjected to column chromatography (methanol:dichloromethane = 1/100-1/10) to give an off-white solid product FDAB-3 (about 2.8 g, 86% yield).

### Example 4: Synthesis of compounds FDAB-4, FDAB-5, and FDAB-6

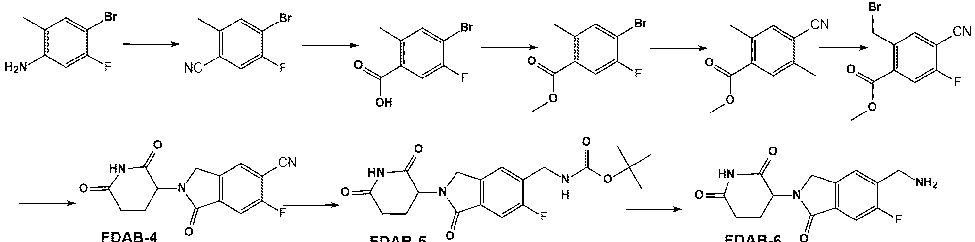

The synthesis of **FDAB-4, FDAB-5,** and **FDAB-6** was similar to that of **FDAB-1, FDAB-2, and FDAB-3** in Examples 1 to 3 above, with the starting material being 4-bromo-5-fluoro-2-methylaniline. The characterization data of the resultant compounds **FDAB-4, FDAB-5,** and **FDAB-6** are shown in Table 1 below.

### Example 5: Synthesis of FDAB-7, FDAB-8, and FDAB-9

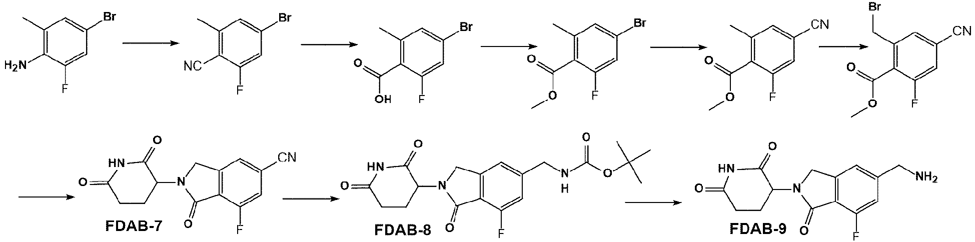

The synthesis of compounds **FDAB-7, FDAB-8,** and **FDAB-9** was similar to that of **FDAB-1, FDAB-2, and FDAB-3** in Examples 1 to 3 above, with the starting material being 4-bromo-2-fluoro-6-methylaniline. The characterization data of the resultant compounds **FDAB-7, FDAB-8,** and FDAB-9 are shown in Table 1 below.

### Example 6: Synthesis of FDAB-10, FDAB-11, and FDAB-12

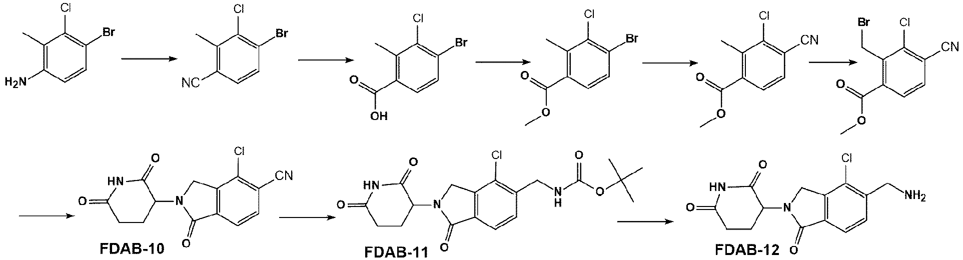

The synthesis of compounds **FDAB-10, FDAB-11,** and **FDAB-12** was similar to that of **FDAB-1, FDAB-2,** and **FDAB-3** in Examples 1 to 3 above, with the starting material being 4-bromo-3-chloro-2-methylaniline. The characterization data of the resultant compounds **FDAB-10, FDAB-11,** and **FDAB-12** are shown in Table 1 below.

### Example 7: Synthesis of compounds FDAB-13, FDAB-14, and FDAB-15

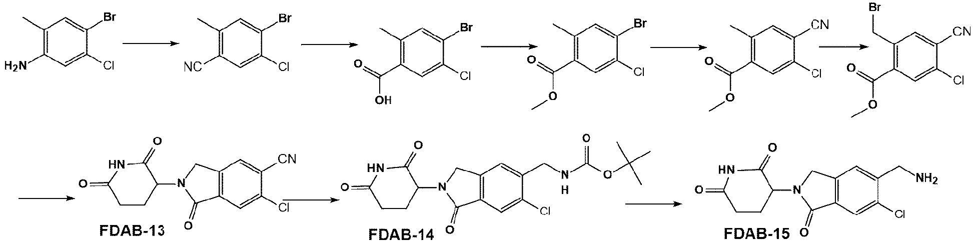

The synthesis of compounds **FDAB-13, FDAB-14,** and **FDAB-15** was similar to that of **FDAB-1, FDAB-2,** and **FDAB-3** in Examples 1 to 3 above, with the starting material being 4-bromo-5-chloro-2-methylaniline. The characterization data of the resultant compounds **FDAB-13, FDAB-14,** and **FDAB-15** are shown in Table 1 below.

### Example 8: Compounds FDAB-16 and FDAB-17

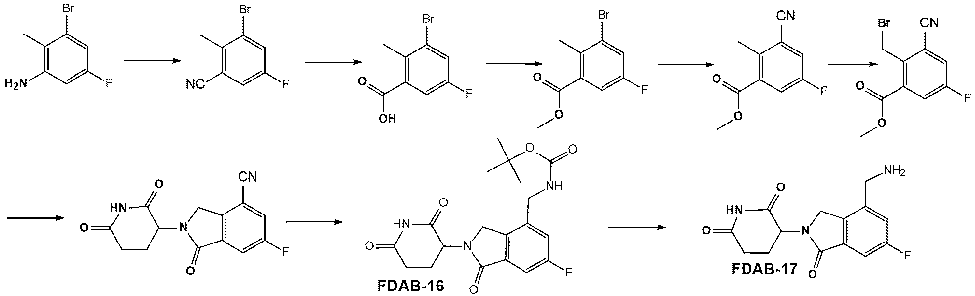

The synthesis of compounds **FDAB-16** and **FDAB-17** was similar to that of **FDAB-2 and FDAB-3** in Examples 2 and 3 above, with the starting material being 3-bromo-5-fluoro-2-methylaniline. The characterization data of the resultant compounds **FDAB-16** and **FDAB-17** are shown in Table 1 below.

### Example 9: Synthesis of compound FDAB-48

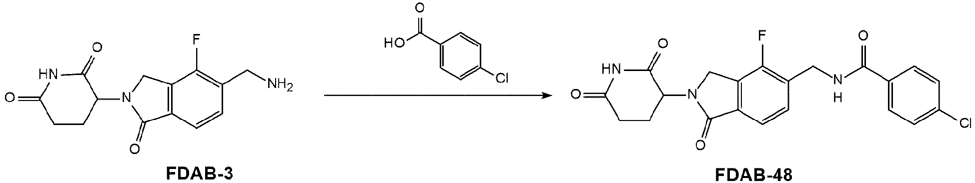

**FDAB-3** (226 mg, 0.69 mmol) andp-chlorobenzoic acid (107 mg, 0.69 mmol) were dissolved in 10 mL of *N,N-*dimethylformamide, and triethylamine (139 mg, 1.37 mmol) was added with stirring. *N,N,N*'*,N*'*-*tetramethyl-O-(7-azabenzotriazol-1-yl)uronium hexafluorophosphate (310 mg, 0.83 mmol) was then added slowly in portions. After about 2 h of reaction, LCMS indicated the depletion of the starting material **FDAB-3.** The reaction solution was quenched by adding 50 mL of water, extracted with ethyl acetate and phase separated. The extraction was repeated three times until the extraction was complete. The organic phases were combined, backwashed with saturated brine, dried over anhydrous sodium sulfate, and concentrated to give a pale yellow concentrate crude product. The crude product was subjected to column chromatography (methanol/dichloromethane = 1/100-1/30) to give a white solid product **FDAB-48** (about 210 mg, 71% yield).

### Example 10: Synthesis of compound FDAB-121

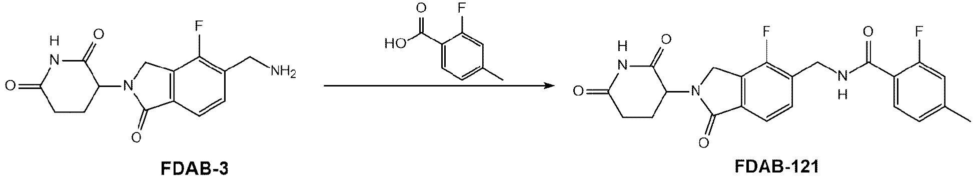

**FDAB-3** (226 mg, 0.69 mmol) and 2-fluoro-4-methylbenzoic acid (106 mg, 0.69 mmol) were dissolved in 10 mL of *N*,*N-*dimethylformamide, and triethylamine (139 mg, 1.38 mmol) was added with stirring. *N*,*N*,*N'*,*N'*-tetramethyl-*O*-(7-azabenzotriazol-1-yl)uronium hexafluorophosphate (310 mg, 0.83 mmol) was then added slowly in portions. After about 2 h of reaction, LCMS indicated the depletion of the starting material **FDAB-3.** The reaction solution was quenched by adding 50 mL of water, extracted with ethyl acetate and phase separated. The extraction was repeated three times until the extraction was complete. The organic phases were combined, backwashed with saturated brine, dried over anhydrous sodium sulfate, and concentrated to give a pale yellow concentrate crude product. The crude product was subjected to column chromatography (methanol/dichloromethane = 1/100-1/30) to give a white solid product **FDAB-121** (about 191 mg, 65% yield).

**FDAB-120** was prepared by replacing the starting material 2-fluoro-4-methylbenzoic acid with 3-fluoro-4-methylbenzoic acid.

Some of the compounds of the present disclosure containing acylamido such as **FDAB-48** and **FDAB-121,** as previously exemplified above, were prepared with reference to the synthesis of **FDAB-48** and **FDAB-121** as described above, which will not be described in detail herein for brevity. The characterization data for these compounds are shown in Table 1 below.

### Example 11: Synthesis of compound FDAB-128

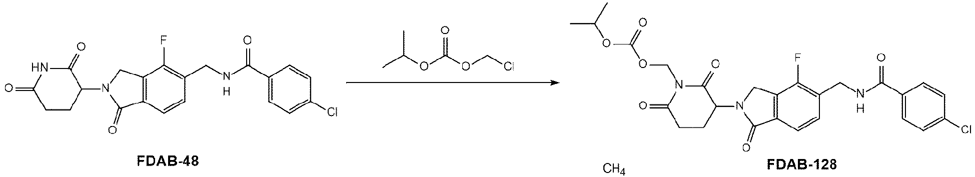

Chloromethyl isopropyl carbonate (34 mg, 0.22 mmol) was added slowly and dropwise to a solution of **FDAB-48** (86 mg, 0.2 mmol) and potassium carbonate (41 mg, 0.3 mmol) in *N,N-*dimethylformamide (10 mL) at 0 °C. The reaction system was slowly warmed to room temperature and stirred for about 3 h, until LCMS indicated the depletion of the starting material **FDAB-48.** The reaction solution was quenched by adding 20 mL of water, extracted with ethyl acetate and phase separated. The extraction was repeated three times until the extraction was complete. The organic phases were combined, backwashed with saturated brine, dried over anhydrous sodium sulfate, and concentrated to give a pale yellow concentrate crude product. The crude product was subjected to column chromatography (methanol/dichloromethane = 1/100-1/50) to give a white solid product **FDAB-128** (about 49 mg, 45% yield).

### Example 12: Synthesis of compound FDAB-130

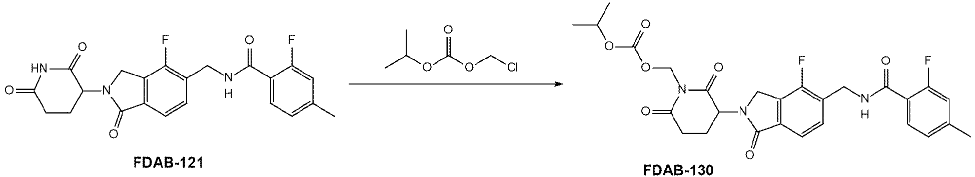

Chloromethyl isopropyl carbonate (34 mg, 0.22 mmol) was added slowly and dropwise to a solution of **FDAB-121** (85 mg, 0.2 mmol) and potassium carbonate (41 mg, 0.3 mmol) in *N,N-*dimethylformamide (10 mL) at 0 °C. The reaction system was slowly warmed to room temperature and stirred for about 3 h, until LCMS indicated the depletion of the starting material **FDAB-121.** The reaction solution was quenched by adding 20 mL of water, extracted with ethyl acetate and phase separated. The extraction was repeated three times until the extraction was complete. The organic phases were combined, backwashed with saturated brine, dried over anhydrous sodium sulfate, and concentrated to give a pale yellow concentrate crude product. The crude product was subjected to column chromatography (methanol/dichloromethane = 1/100-1/50) to give a white solid product **FDAB-130** (about 38 mg, 35% yield).

Some of the compounds of the present disclosure with the N on the glutarimide ring being substituted such as **FDAB-128** and **FDAB-130,** as previously exemplified above, were prepared with reference to the synthesis of **FDAB-128** and **FDAB-130** as described above, which will not be described in detail herein for brevity. The characterization data for these compounds are shown in Table 1 below.

### Example 13: Synthesis of compound FDAB-166

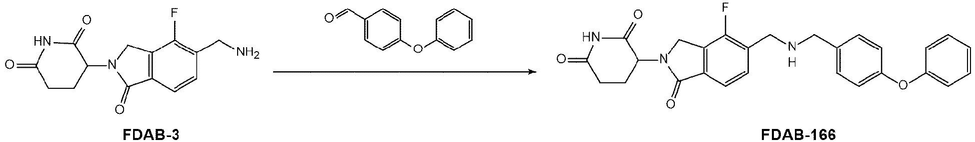

**FDAB-3** (20 mg, 0.06 mmol) and 4-phenoxybenzaldehyde (14 mg, 0.07 mmol) were dissolved in 5 mL of tetrahydrofuran, and sodium bicarbonate (10 mg, 0.12 mmol) was added with stirring. The reaction system was warmed to 50 °C and reacted for about 1 h. The reaction system was added with sodium cyanoborohydride (5.8 mg, 0.09 mmol), and stirred at 50 °C for about 1 h. The reaction solution was quenched by adding 20 mL of water, extracted with ethyl acetate (10 mL) and phase separated. The extraction was repeated three times until the extraction was complete. The organic phases were combined, backwashed with saturated brine, dried over anhydrous sodium sulfate, and concentrated to give a brown concentrate crude product. The crude product was subjected to column chromatography (methanol/dichloromethane = 1/100-1/30) to give a white solid product **FDAB-166** (about 15 mg, 52% yield).

Referring to the synthesis of **FDAB-166,** the compounds **FDAB-165, FDAB-167,** and **FDAB-168** were prepared by reacting **FDAB-3** with corresponding aldehydes. For brevity, the processes are not described in detail herein. The characterization data for these compounds are shown in Table 1 below.

The characterization data for some of the compounds described above are shown in Table 1 below.

**Table 1**

| No. | ¹H NMR (400 MHz, DMSO-*d₆*), MS (m/e) |
|---|---|
| FDAB-1 | 11.07 (s, 1H), 8.11 (dd, *J =* 7.8, 5.5 Hz, 1H), 7.78 (d, *J =* 7.8 Hz, 1H), 5.16 (dd, *J =* 13.3, 5.1 Hz, 1H), 4.68 (d, *J =* 18.0 Hz, 1H), 4.52 (d, *J =* 18.0 Hz, 1H), 2.96-2.87 (m, 1H), 2.66-2.56 (m, 1H), 2.46-2.41 (m, 1H), 2.05-1.99 (m, 1H). Molecular formula: C14H10FN3O3, calculated molecular weight: 287.25; found (LC-MS): 288.25 [M+1]⁺. |
| FDAB-2 | 11.02 (s, 1H), 7.60-7.52 (m, 2H), 7.47 (t, *J =* 7.0 Hz, 1H), 5.11 (dd, *J =* 13.3, 5.1 Hz, 1H), 4.55 (d, *J =* 17.4 Hz, 1H), 4.37 (d, *J =* 17.4 Hz, 1H), 4.26 (d, *J =* 6.1 Hz, 2H), 2.96-2.87 (m, 1H), 2.63-2.57 (m, 1H), 2.45-2.41 (m, 1H), 2.04-1.97 (m, 1H), 1.39 (s, 9H). Molecular formula: C19H22FN3O5, calculated molecular weight: 391.40; found (LC-MS): 392.40 [M+1]⁺. |
| FDAB-3 | 11.04 (s, 1H), 8.54 (s, 2H), 7.79-7.71 (m, 1H), 7.68 (d, *J =* 7.7 Hz, 1H), 5.14 (dd, *J =* 13.3, 5.1 Hz, 1H), 4.60 (d, *J =* 17.7 Hz, 1H), 4.42 (d, *J =* 17.6 Hz, 1H), 4.19 (s, 2H), 2.96-2.86 (m, 1H), 2.65-2.56 (m, 1H), 2.46-2.39 (m, 1H), 2.08-1.98 (m, 1H). Molecular formula: C14H14FN3O3, calculated molecular weight: 291.28; found (LC-MS): 292.28 [M+1]⁺. |
| FDAB-4 | 11.04 (s, 1H), 8.25 (d, *J =* 5.4 Hz, 1H), 7.89 (d, *J =* 8.2 Hz, 1H), 5.16 (dd, *J =* 13.3, 5.1 Hz, 1H), 4.52 (d, *J =* 17.7 Hz, 1H), 4.40 (d, *J =* 17.7 Hz, 1H), 2.97-2.86 (m, 1H), 2.65-2.57 (m, 1H), 2.46-2.39 (m, 1H), 2.10-1.98 (m, 1H). Molecular formula: C14H10FN3O3, calculated molecular weight: 287.25; found (LC-MS): 288.25 [M+1]⁺. |
| FDAB-5 | 11.01 (s, 1H), 7.52 (s, 1H), 7.49 (d, *J =* 8.8 Hz, 2H), 5.11 (dd, *J =* 13.3, 5.1 Hz, 1H), 4.45 (d, *J =* 17.3 Hz, 1H), 4.30 (d, *J =* 17.3 Hz, 1H), 4.26 (d, *J =* 4.8 Hz, 2H), 2.97-2.86 (m, 1H), 2.63-2.57 (m, 1H), 2.45-2.41 (m, 1H), 2.04-1.98 (m, 1H), 1.39 (s, 9H). Molecular formula: C19H22FN3O5, calculated molecular weight: 391.40; found (LC-MS): 392.40 [M+1]⁺. |
| FDAB-6 | 11.03 (s, 1H), 8.51 (s, 2H), 7.81 (d, *J* = 6.2 Hz, 1H), 7.65 (d, *J* = 8.7 Hz, 1H), 5.14 (dd, *J =* 13.3, 5.0 Hz, 1H), 4.48 (d, *J =* 17.4 Hz, 1H), 4.34 (d, *J =* 17.4 Hz, 1H), 4.17 (s, 2H), 2.99-2.86 (m, 1H), 2.65-2.56 (m, 1H), 2.44-2.37 (m, 1H), 2.07-1.99 (m, 1H). Molecular formula: C14H14FN3O3, calculated molecular weight: 291.28; found (LC-MS): 292.28 [M+1]⁺. |
| FDAB-7 | 11.05 (s, 1H), 8.01 (d, *J =* 1.0 Hz, 1H), 7.98 (d, *J =* 9.2 Hz, 1H), 5.12 (dd, *J =* 13.3, 5.1 Hz, 1H), 4.56 (d, *J =* 18.5 Hz, 1H), 4.43 (d, *J =* 18.4 Hz, 1H), 2.97-2.87 (m, 1H), 2.65-2.54 (m, 1H), 2.44-2.35 (m, 1H), 2.05-1.97 (m, 1H). Molecular formula: C14H10FN3O3, calculated molecular weight: 287.25; found (LC-MS): 288.25 [M+1]⁺. |
| FDAB-8 | 11.01 (s, 1H), 7.55 (t, *J* = 6.2 Hz, 1H), 7.28 (s, 1H), 7.12 (d, *J =* 10.4 Hz, 1H), 5.07 (dd, *J =* 13.3, 5.1 Hz, 1H), 4.47 (d, *J =* 17.8 Hz, 1H), 4.32 (d, *J =* 17.7 Hz, 1H), 4.22 (d, *J =* 5.1 Hz, 2H), 2.97-2.86 (m, 1H), 2.64-2.55 (m, 1H), 2.40-2.33 (m, 1H), 2.07-1.96 (m, 1H), 1.40 (s, 9H). Molecular formula: C19H22FN3O5, calculated molecular weight: 391.40; found (LC-MS): 392.40 [M+1]⁺. |
| FDAB-9 | 11.03 (s, 1H), 8.45 (s, 2H), 7.53 (s, 1H), 7.46 (d, *J* = 10.4 Hz, 1H), 5.10 (dd, *J* = 13.3, 5.1 Hz, 1H), 4.51 (d, *J* = 18.0 Hz, 1H), 4.36 (d, *J* = 18.0 Hz, 1H), 4.24-4.08 (m, 2H), 2.98-2.88 (m, 1H), 2.65-2.56 (m, 1H), 2.45-2.37 (m, 1H), 2.06-1.96 (m, 1H). Molecular formula: C14H14FN3O3, calculated molecular weight: 291.28; found (LC-MS): 292.28 [M+1]⁺. |
| FDAB-10 | 11.06 (s, 1H), 8.16 (d, *J =* 7.8 Hz, 1H), 7.91 (d, *J =* 7.8 Hz, 1H), 5.18 (dd, *J* = 13.3, 5.1 Hz, 1H), 4.60 (d, *J* = 18.2 Hz, 1H), 4.44 (d, *J* = 18.2 Hz, 1H), 2.97-2.86 (m, 1H), 2.65-2.56 (m, 1H), 2.48-2.40 (m, 1H), 2.07-1.96 (m, 1H). Molecular formula: C14H10ClN3O3, calculated molecular weight: 303.70; found (LC-MS): 304.70 [M+1]⁺. |
| FDAB-11 | 11.02 (s, 1H), 7.73 (d, *J =* 7.7 Hz, 1H), 7.58 (t, *J =* 6.0 Hz, 1H), 7.49 (d, *J =* 7.8 Hz, 1H), 5.13 (dd, *J =* 13.3, 5.1 Hz, 1H), 4.48 (d, *J =* 17.6 Hz, 1H), 4.31 (dd, *J =* 11.5, 6.1 Hz, 3H), 2.97-2.86 (m, 1H), 2.64-2.55 (m, 1H), 2.45-2.41 (m, 1H), 2.05-1.97 (m, 1H), 1.40 (s, 9H). Molecular formula: C19H22ClN3O5, calculated molecular weight: 407.85; found (LC-MS): 408.85 [M+1]⁺. |
| FDAB-12 | 11.04 (s, 1H), 8.60 (s, 2H), 7.84 (d, *J* = 7.8 Hz, 1H), 7.77 (d, *J* = 7.9 Hz, 1H), 5.16 (dd, *J =* 13.3, 5.1 Hz, 1H), 4.53 (d, *J =* 17.9 Hz, 1H), 4.36 (d, *J =* 17.8 Hz, 1H), 4.27 (s, 2H), 2.97-2.86 (m, 1H), 2.65-2.56 (m, 1H), 2.45-2.41 (m, 1H), 2.08-1.99 (m, 1H). Molecular formula: C14H14ClN3O3, calculated molecular weight: 307.73; found (LC-MS): 308.73 [M+1]⁺. |
| FDAB-13 | 11.05 (s, 1H), 8.31 (s, 1H), 8.08 (s, 1H), 5.15 (dd, *J* = 13.3, 5.1 Hz, 1H), 4.54 (d, *J* = 18.2 Hz, 1H), 4.42 (d, *J* = 18.2 Hz, 1H), 2.98-2.88 (m, 1H), 2.65-2.56 (m, 1H), 2.45-2.37 (m, 1H), 2.06-1.98 (m, 1H). Molecular formula: C14H10ClN3O3, calculated molecular weight: 303.70; found (LC-MS): 304.70 [M+1]⁺. |
| FDAB-14 | 11.02 (s, 1H), 7.74 (s, 1H), 7.56 (d, *J* = 6.1 Hz, 1H), 7.53 (d, *J* = 3.8 Hz, 1H), 5.11 (dd, *J* = 13.3, 5.1 Hz, 1H), 4.48 (d, *J =* 17.7 Hz, 1H), 4.33 (d, *J =* 17.7 Hz, 1H), 4.29 (d, *J =* 5.4 Hz, 2H), 2.96-2.87 (m, 1H), 2.65-2.56 (m, 1H), 2.42-2.35 (m, 1H), 2.05-1.97 (m, 1H), 1.41 (s, 9H). Molecular formula: C19H22ClN3O5, calculated molecular weight: 407.85; found (LC-MS): 408.85 [M+1]⁺. |
| FDAB-15 | 11.04 (s, 1H), 8.57 (s, 2H), 7.88 (s, 1H), 7.84 (s, 1H), 5.14 (dd, *J* = 13.3, 5.1 Hz, 1H), 4.51 (d, *J =* 17.8 Hz, 1H), 4.37 (d, *J =* 17.8 Hz, 1H), 4.25 (s, 2H), 2.99-2.86 (m, 1H), 2.66-2.56 (m, 1H), 2.45-2.37 (m, 1H), 2.07-1.99 (m, 1H). Molecular formula: C14H14ClN3O3, calculated molecular weight: 307.73; found (LC-MS): 308.73 [M+1]⁺. |
| FDAB-16 | 11.03 (s, 1H), 7.42 (dd, *J =* 7.6, 2.4 Hz, 1H), 7.29 (dd, *J =* 10.3, 2.4 Hz, 1H), 5.15 (dd, *J =* 13.3, 5.1 Hz, 1H), 4.47 (d, *J =* 17.3 Hz, 1H), 4.34 (d, *J =* 17.2 Hz, 1H), 4.21 (s, 2H), 2.99-2.86 (m, 1H), 2.67-2.60 (m, 1H), 2.42-2.33 (m, 1H), 2.09-2.00 (m, 1H), 1.39 (s, 9H). Molecular formula: C19H22FN3O5, calculated molecular weight: 391.40; found (LC-MS): 392.40 [M+1]⁺. |
| FDAB-17 | 11.06 (s, 1H), 8.60 (s, 2H), 7.72-7.67 (m, 1H), 7.60-7.56 (m, 1H), 5.18 (dd, *J* = 13.2, 5.1 Hz, 1H), 4.65 (d, *J* = 17.4 Hz, 1H), 4.46 (d, *J* = 17.3 Hz, 1H), 4.10 (s, 2H), 2.97-2.86 (m, 1H), 2.67-2.60 (m, 1H), 2.42-2.33 (m, 1H), 2.08-1.94 (m, 1H). Molecular formula: C14H15ClFN3O3, calculated molecular weight: 327.74; found (LC-MS): 292.28 [M+1]⁺. |
| FDAB-18 | 11.01 (s, 1H), 9.72 (t, *J =* 6.1 Hz, 1H), 7.69 (d, *J =* 7.8 Hz, 1H), 7.43-7.36 (m, 1H), 5.11 (dd, *J =* 13.3, 5.1 Hz, 1H), 4.51-4.35 (m, 3H), 4.25 (d, *J =* 17.3 Hz, 1H), 2.94-2.90 (m, 1H), 2.65-2.58 (m, 1H), 2.46-2.36 (m, 1H), 2.04-1.97 (m, 1H). Molecular formula: C21H13D4ClFN3O4, calculated molecular weight: 433.86; found (LC-MS): 434.86 [M+1]⁺. |
| FDAB-19 | 10.98 (s, 1H), 9.37 (t, *J =* 5.9 Hz, 1H), 7.79 (d, *J =* 7.8 Hz, 1H), 7.67 (d, *J =* 8.4 Hz, 1H), 7.65-7.55 (m, 2H), 7.56-7.44 (m, 2H), 7.35 (t, *J =* 7.5 Hz, 1H), 5.10 (dd, *J =* 13.3, 5.1 Hz, 1H), 4.62 (d, *J =* 5.9 Hz, 2H), 4.42 (d, *J =* 17.3 Hz, 1H), 4.29 (d, *J =* 17.4 Hz, 1H), 2.96-2.85 (m, 1H), 2.63-2.56 (m, 1H), 2.42-2.32 (m, 1H), 2.03-1.95 (m, 1H). Molecular formula: C23H18FN3O5, calculated molecular weight: 435.41; found (LC-MS): 436.41 [M+1]⁺. |
| FDAB-20 | 10.98 (s, 1H), 9.44 (t, *J =* 5.9 Hz, 1H), 7.89 (d, *J =* 2.2 Hz, 1H), 7.71 (d, *J =* 8.8 Hz, 1H), 7.63 (d, *J =* 6.3 Hz, 1H), 7.58 (d, *J =* 1.0 Hz, 1H), 7.56-7.46 (m, 2H), 5.10 (dd, *J =* 13.3, 5.1 Hz, 1H), 4.61 (d, *J =* 5.9 Hz, 2H), 4.42 (d, *J =* 17.4 Hz, 1H), 4.29 (d, *J =* 17.4 Hz, 1H), 2.96-2.85 (m, 1H), 2.63-2.56 (m, 1H), 2.43-2.29 (m, 1H), 2.04-1.96 (m, 1H). Molecular formula: C23H17ClFN3O5, calculated molecular weight: 469.85; found (LC-MS): 470.85 [M+1]⁺. |
| FDAB-21 | 10.99 (s, 1H), 9.41 (t, *J =* 6.2 Hz, 1H), 7.79 (d, *J =* 7.8 Hz, 1H), 7.67 (d, *J =* 8.4 Hz, 1H), 7.60 (s, 1H), 7.50-7.46 (m, 1H), 7.39 (s, 1H), 7.35 (t, *J =* 7.5 Hz, 1H), 7.25 (d, *J =* 10.4 Hz, 1H), 5.06 (dd, *J =* 13.3, 5.1 Hz, 1H), 4.59 (d*, J =* 6.1 Hz, 2H), 4.47 (d, *J =* 17.8 Hz, 1H), 4.33 (d, *J =* 17.7 Hz, 1H), 2.96-2.85 (m, 1H), 2.61-2.57 (m, 1H), 2.40-2.29 (m, 1H), 2.04-1.95 (m, 1H). Molecular formula: C23H18FN3O5, calculated molecular weight: 435.41; found (LC-MS): 436.41 [M+1]⁺. |
| FDAB-22 | 10.99 (s, 1H), 9.48 (t, *J =* 6.1 Hz, 1H), 7.89 (d, *J =* 2.2 Hz, 1H), 7.72 (d, *J =* 8.8 Hz, 1H), 7.58 (s, 1H), 7.50 (dd, *J =* 8.9, 2.3 Hz, 1H), 7.39 (s, 1H), 7.25 (d, *J =* 10.4 Hz, 1H), 5.06 (dd, *J =* 13.3, 5.1 Hz, 1H), 4.58 (d, *J =* 6.1 Hz, 2H), 4.46 (d, *J =* 17.8 Hz, 1H), 4.32 (d, *J =* 17.8 Hz, 1H), 2.95-2.85 (m, 1H), 2.61-2.57 (m, 1H), 2.40-2.31 (m, 1H), 2.02-1.9 (m, 1H). Molecular formula: C23H17ClFN3O5, calculated molecular weight: 469.85; found (LC-MS): 470.85 [M+1]⁺. |
| FDAB-23 | 10.98 (s, 1H), 9.20 (t, *J =* 5.8 Hz, 1H), 7.93 (d, *J =* 8.4 Hz, 2H), 7.65-7.38 (m, 4H), 5.10 (dd, *J =* 13.3, 5.1 Hz, 1H), 4.59 (d, *J* = 5.6 Hz, 2H), 4.42 (d, *J* = 17.3 Hz, 1H), 4.29 (d, *J* = 17.4 Hz, 1H), 2.97-2.86 (m, 1H), 2.61-2.56 (m, 1H), 2.38-2.31 (m, 1H), 2.07-1.98 (m, 1H). Molecular formula: C21H17ClFN3O4, calculated molecular weight: 429.83; found (LC-MS): 430.83 [M+1]⁺. |
| FDAB-24 | 11.61 (s, 1H), 10.98 (s, 1H), 9.10 (t, *J =* 5.9 Hz, 1H), 7.62 (d, *J =* 7.2 Hz, 2H), 7.54 (d, *J =* 8.8 Hz, 1H), 7.43 (d, *J =* 8.3 Hz, 1H), 7.29-7.14 (m, 2H), 7.04 (t, *J =* 7.5 Hz, 1H), 5.10 (dd, *J =* 13.2, 5.1 Hz, 1H), 4.64 (d, *J =* 5.8 Hz, 2H), 4.43 (d, *J =* 17.4 Hz, 1H), 4.29 (d, *J =* 17.4 Hz, 1H), 2.97-2.86 (m, 1H), 2.61-2.56 (m, 1H), 2.42-2.31 (m, 1H), 2.07-1.98 (m, 1H). Molecular formula: C23H19FN4O4, calculated molecular weight: 434.43; found (LC-MS): 435.43 [M+1]⁺. |
| FDAB-25 | 9.12 (t, *J =* 5.8 Hz, 1H), 7.97-7.86 (m, 2H), 7.60 (d, *J =* 6.2 Hz, 1H), 7.59-7.39 (m, 4H), 5.10 (dd, *J =* 13.3, 5.1 Hz, 1H), 4.60 (d, *J =* 5.7 Hz, 2H), 4.43 (d, *J =* 17.3 Hz, 1H), 4.29 (d, *J =* 17.3 Hz, 1H), 2.95-2.85 (m, 1H), 2.61-2.56 (m, 1H), 2.38-2.31 (m, 1H), 2.07-1.98 (m, 1H). Molecular formula: C21H18FN3O4, calculated molecular weight: 395.39; found (LC-MS): 396.39 [M+1]⁺. |
| FDAB-26 | 9.14 (t, *J =* 5.8 Hz, 1H), 8.06-7.89 (m, 2H), 7.60 (d, *J =* 6.3 Hz, 1H), 7.52 (d, *J =* 8.8 Hz, 1H), 7.39-7.23 (m, 2H), 5.10 (dd, *J =* 13.2, 5.1 Hz, 1H), 4.59 (d, *J =* 5.7 Hz, 2H), 4.42 (d, *J =* 17.3 Hz, 1H), 4.29 (d, *J =* 17.4 Hz, 1H), 2.95-2.86 (m, 1H), 2.62-2.57 (m, 1H), 2.37-2.31 (m, 1H), 2.07-1.98 (m, 1H). Molecular formula: C21H17F2N3O4, calculated molecular weight: 413.38; found (LC-MS): 414.38 [M+1]⁺. |
| FDAB-27 | 11.00 (s, 1H), 8.65 (s, 1H), 7.62 (d, *J =* 8.6 Hz, 2H), 7.52 (dd, *J =* 12.6, 7.6 Hz, 2H), 7.42-7.37(m, 1H), 6.91 (d, *J =* 11.3 Hz, 1H), 6.54 (s, 2H), 5.10 (dd, *J =* 13.2, 5.0 Hz, 1H), 4.59-4.53 (m, 3H), 2.95-2.86 (m, 1H), 2.62-2.57 (m, 1H), 2.37-2.31 (m, 1H), 2.07-1.98 (m, 1H). Molecular formula: C21H19FN4O4, calculated molecular weight: 410.41; found (LC-MS): 411.41 [M+1]⁺. |
| FDAB-28 | 11.00 (s, 1H), 10.20 (s, 1H), 8.65 (s, 1H), 7.91-7.85 (m, 2H), 7.71 (dd, *J =* 12.6, 7.6 Hz, 2H), 7.54-7.32 (m, 2H), 5.10 (dd, *J =* 13.2, 5.0 Hz, 1H), 4.59-4.53 (m, 3H), 2.95-2.86 (m, 1H), 2.62-2.57 (m, 1H), 2.37-2.31 (m, 1H), 2.07-1.98 (m, 1H). Molecular formula: C21H18FN3O5, calculated molecular weight: 411.39; found (LC-MS): 412.39 [M+1]⁺. |
| FDAB-29 | 11.00 (s, 1H), 9.45 (t, *J =* 6.3 Hz, 1H), 8.71-8.65 (m, 1H), 8.09-7.97 (m, 2H), 7.65-7.62 (m, 1H), 7.54 (dd, *J* = 14.9, 7.6 Hz, 2H), 5.10 (dd, *J =* 13.3, 5.1 Hz, 1H), 4.64 (d, *J =* 5.4 Hz, 2H), 4.41 (d, *J =* 17.3 Hz, 1H), 4.26 (d, *J =* 17.3 Hz, 1H), 2.95-2.85 (m, 1H), 2.62-2.58 (m, 1H), 2.36-2.32 (m, 1H), 2.03-1.97 (m, 1H). Molecular formula: C20H17FN4O4, calculated molecular weight: 396.38; found (LC-MS): 397.38 [M+1]⁺. |
| FDAB-30 | 10.98 (s, 1H), 9.32 (t, *J =* 5.8 Hz, 1H), 9.07 (d, *J =* 2.3 Hz, 1H), 8.73 (dd, *J =* 4.8, 1.6 Hz, 1H), 8.24 (dt, *J* = 7.8, 2.1 Hz, 1H), 7.64 (d, *J* = 6.3 Hz, 1H), 7.53 (dd, *J* = 8.5, 3.7 Hz, 2H), 5.10 (dd, *J =* 13.3, 5.1 Hz, 1H), 4.62 (d, *J =* 5.7 Hz, 2H), 4.43 (d, *J =* 17.3 Hz, 1H), 4.29 (d, *J =* 17.3 Hz, 1H), 2.92-2.85 (m, 1H), 2.61-2.57 (m, 1H), 2.41-2.33 (m, 1H), 2.04-1.96 (m, 1H). Molecular formula: C20H17FN4O4, calculated molecular weight: 396.38; found (LC-MS): 397.38 [M+1]⁺. |
| FDAB-31 | 10.99 (s, 1H), 9.41 (t, *J =* 5.7 Hz, 1H), 8.81-8.70 (m, 2H), 7.84-7.78 (m, 2H), 7.63 (d, *J =* 6.2 Hz, 1H), 7.53 (d, *J =* 8.9 Hz, 1H), 5.10 (dd, *J =* 13.3, 5.1 Hz, 1H), 4.62 (d, *J* = 5.7 Hz, 2H), 4.43 (d, *J =* 17.4 Hz, 1H), 4.29 (d, *J =* 17.4 Hz, 1H), 2.95-2.86 (m, 1H), 2.61-2.57 (m, 1H), 2.41-2.33 (m, 1H), 2.03-1.98 (m, 1H). Molecular formula: C20H17FN4O4, calculated molecular weight: 396.38; found (LC-MS): 397.38 [M+1]⁺. |
| FDAB-32 | 10.98 (s, 1H), 9.83 (t, *J =* 6.1 Hz, 1H), 8.25 (d, *J =* 8.1 Hz, 1H), 8.16 (d, *J =* 8.0 Hz, 1H), 7.65 (t, *J =* 6.9 Hz, 2H), 7.60 (t, *J =* 7.5 Hz, 1H), 7.53 (d, *J =* 8.8 Hz, 1H), 5.10 (dd, *J =* 13.3, 5.1 Hz, 1H), 4.65 (d, *J =* 6.1 Hz, 2H), 4.43 (d, *J =* 17.3 Hz, 1H), 4.29 (d, *J =* 17.4 Hz, 1H), 2.94-2.85 (m, 1H), 2.61-2.58 (m, 1H), 2.42-2.34 (m, 1H), 2.03-1.95 (m, 1H). Molecular formula: C22H17FN4O4S, calculated molecular weight: 452.46; found (LC-MS): 453.46 [M+1]⁺. |
| FDAB-33 | 13.31 (s, 1H), 11.00 (s, 1H), 9.63 (t, *J =* 6.3 Hz, 1H), 7.74 (s, 1H), 7.61 (d, *J =* 6.2 Hz, 1H), 7.59-7.44 (m, 2H), 7.39-7.28 (m, 2H), 5.10 (dd, *J =* 13.4, 5.1 Hz, 1H), 4.65 (d, J= 4.7 Hz, 2H), 4.41 (d,*J* = 17.4 Hz, 1H), 4.28 (d, *J* = 17.4 Hz, 1H), 2.96-2.83 (m, 1H), 2.58-2.51 (m, 1H), 2.42-2.36 (m, 1H), 2.03-1.97 (m, 1H). Molecular formula: C22H18FN5O4, calculated molecular weight: 435.42; found (LC-MS): 436.42 [M+1]⁺. |
| FDAB-34 | 11.00 (s, 1H), 8.85 (s, 1H), 7.72 (d, *J* = 8.6 Hz, 2H), 7.62 (dd, *J* = 12.6, 7.6 Hz, 2H), 7.52-7.45(m, 1H), 6.91-6.82 (m, 1H), 6.54 (s, 2H), 5.10 (dd, *J* = 13.2, 5.0 Hz, 1H), 4.59-4.53 (m, 3H), 2.95-2.86 (m, 1H), 2.62-2.57 (m, 1H), 2.37-2.31 (m, 1H), 2.07-1.98 (m, 1H). Molecular formula: C21H19FN4O4, calculated molecular weight: 410.41; found (LC-MS): 411.41 [M+1]⁺. |
| FDAB-35 | 10.97 (s, 1H), 9.42 (t, *J* = 5.9 Hz, 1H), 7.71 (dd, *J* = 9.1, 4.1 Hz, 1H), 7.67-7.56 (m, 3H), 7.53 (d, J= 8.8 Hz, 1H), 7.33 (td, *J* = 9.2, 2.8 Hz, 1H), 5.10 (dd, *J* = 13.3, 5.1 Hz, 1H), 4.61 (d, *J* = 5.9 Hz, 2H), 4.42 (d, *J =* 17.3 Hz, 1H), 4.29 (d, *J =* 17.3 Hz, 1H), 2.96-2.85 (m, 1H), 2.61-2.58 (m, 1H), 2.42-2.37 (m, 1H), 2.07-1.98 (m, 1H). Molecular formula: C23H17F2N3O5, calculated molecular weight: 453.40; found (LC-MS): 454.40 [M+1]⁺. |
| FDAB-36 | 11.00 (s, 1H), 10.20 (s, 1H), 8.65 (s, 1H), 7.95-7.88 (m, 1H), 7.82-7.78 (m, 2H), 7.54-7.32 (m, 3H), 5.10 (dd, *J* = 13.2, 5.0 Hz, 1H), 4.59-4.53 (m, 3H), 2.97-2.86 (m, 1H), 2.62-2.57 (m, 1H), 2.37-2.31 (m, 1H), 2.07-1.98 (m, 1H). Molecular formula: C21H18FN3O5, calculated molecular weight: 411.39; found (LC-MS): 412.39 [M+1]⁺. |
| FDAB-37 | 9.11 (t, *J* = 5.8 Hz, 1H), 7.59 (d, *J =* 6.2 Hz, 1H), 7.56-7.43 (m, 3H), 7.39 (t, *J* = 7.9 Hz, 1H), 7.11 (dd, *J* = 8.1, 2.6 Hz, 1H), 5.10 (dd, *J* = 13.3, 5.1 Hz, 1H), 4.59 (d, *J* = 5.7 Hz, 2H), 4.43 (d, *J* = 17.3 Hz, 1H), 4.29 (d, *J* = 17.3 Hz, 1H), 3.80 (s, 3H), 2.96-2.86 (m, 1H), 2.70-2.61 (m, 1H), 2.41-2.35 (m, 1H), 2.04-1.96 (m, 1H). Molecular formula: C22H20FN3O5, calculated molecular weight: 425.42; found (LC-MS): 426.42 [M+1]⁺. |
| FDAB-38 | 11.00 (s, 1H), 9.72 (t, *J* = 6.2 Hz, 1H), 9.37 (d, *J* = 1.4 Hz, 1H), 9.09 (d, *J* = 5.0 Hz, 1H), 8.04 (dd, *J* = 5.0, 1.5 Hz, 1H), 7.58 (d, *J* = 6.3 Hz, 1H), 7.52 (d, *J =* 8.8 Hz, 1H), 5.10 (dd, *J =* 13.3, 5.0 Hz, 1H), 4.64 (d, *J* = 4.1 Hz, 2H), 4.40 (d, *J =* 17.3 Hz, 1H), 4.26 (d, *J =* 17.3 Hz, 1H), 2.95-2.85 (m, 1H), 2.62-2.56 (m, 1H), 2.41-2.36 (m, 1H), 2.03-1.94 (m, 1H). Molecular formula: C19H16FN5O4, calculated molecular weight: 397.37; found (LC-MS): 398.37 [M+1]⁺. |
| FDAB-39 | 11.00 (s, 1H), 9.72 (t, *J* = 6.2 Hz, 1H), 9.28 (d, *J* = 1.4 Hz, 1H), 8.97 (d, *J* = 5.0 Hz, 1H), 8.04 (dd, *J* = 5.0, 1.5 Hz, 1H), 7.58 (d, *J* = 6.3 Hz, 1H), 7.52 (d, *J =* 8.8 Hz, 1H), 5.10 (dd, *J =* 13.3, 5.0 Hz, 1H), 4.64 (d, *J* = 4.1 Hz, 2H), 4.40 (d, *J =* 17.3 Hz, 1H), 4.26 (d, *J =* 17.3 Hz, 1H), 2.95-2.85 (m, 1H), 2.62-2.56 (m, 1H), 2.41-2.36 (m, 1H), 2.03-1.94 (m, 1H). Molecular formula: C19H16FN5O4, calculated molecular weight: 397.37; found (LC-MS): 398.37 [M+1]⁺. |
| FDAB-40 | 11.01 (s, 1H), 8.68 (t, *J =* 5.8 Hz, 1H), 7.52 (t, *J* = 7.4 Hz, 2H), 5.11 (dd, *J =* 13.4, 5.1 Hz, 1H), 4.45 (d, *J =* 17.3 Hz, 1H), 4.39 (d, *J* = 4.8 Hz, 2H), 4.30 (d, *J* = 17.2 Hz, 1H), 2.96-2.87 (m, 1H), 2.64-2.56 (m, 1H), 2.40-2.37 (m, 1H), 2.03-1.96 (m, 1H), 1.68-1.57 (m, 1H), 0.74-0.63 (m, 4H). Molecular formula: C18H18FN3O4, calculated molecular weight: 359.36; found (LC-MS): 360.36 [M+1]⁺. |
| FDAB-41 | 8.37 (t, *J* = 5.8 Hz, 1H), 7.55-7.43 (m, 2H), 5.11 (dd, *J* = 13.3, 5.1 Hz, 1H), 4.44 (d, *J* = 17.3 Hz, 1H), 4.37 (d, *J =* 5.8 Hz, 2H), 4.30 (d, *J* = 17.2 Hz, 1H), 2.96-2.87(m, 1H), 2.69-2.65 (m, 1H), 2.64-2.60 (m, 1H), 2.45-2.38 (m, 1H), 2.05-1.97 (m, 1H), 1.81-1.74 (m, 2H), 1.68-1.59 (m, 4H), 1.55-1.43 (m, 2H). Molecular formula: C20H22FN3O4, calculated molecular weight: 387.41; found (LC-MS): 388.41 [M+1]⁺. |
| FDAB-42 | 11.01 (s, 1H), 8.33 (t, *J =* 5.9 Hz, 1H), 7.49 (t, *J* = 8.0 Hz, 2H), 5.11 (dd, *J* = 13.3, 5.0 Hz, 1H), 4.43 (d, *J =* 17.3 Hz, 1H), 4.35 (d, *J =* 4.6 Hz, 2H), 4.29 (d, *J* = 17.2 Hz, 1H), 2.96-2.87 (m, 1H), 2.64-2.54 (m, 1H), 2.40-2.36 (m, 1H), 2.22-2.14 (m, 1H), 2.05-1.96 (m, 1H), 1.77-1.66 (m, 4H), 1.39-1.29 (m, 2H), 1.29-1.03 (m, 4H). Molecular formula: C21H24FN3O4, calculated molecular weight: 401.44; found (LC-MS): 402.44 [M+1]⁺. |
| FDAB-43 | 11.01 (s, 1H), 9.28 (t, *J* = 5.9 Hz, 1H), 8.59 (s, 1H), 7.83 (s, 1H), 7.60 (d, *J* = 6.3 Hz, 1H), 7.53 (d, *J* = 8.8 Hz, 1H), 5.11 (dd, *J* = 13.4, 5.1 Hz, 1H), 4.57 (d, *J* = 4.6 Hz, 2H), 4.42 (d, *J* = 17.4 Hz, 1H), 4.29 (d, *J* = 17.3 Hz, 1H), 2.95-2.86 (m, 1H), 2.59-2.51 (m, 1H), 2.42-2.38 (m, 1H), 2.03-1.95 (m, 1H). Molecular formula: C18H15FN4O5, calculated molecular weight: 386.34; found (LC-MS): 387.34 [M+1]⁺. |
| FDAB-44 | 11.01 (s, 1H), 9.48 (t, *J* = 5.9 Hz, 1H), 8.99 (s, 1H), 8.03 (s, 1H), 7.60 (d, *J* = 6.3 Hz, 1H), 7.53 (d, *J* = 8.8 Hz, 1H), 5.11 (dd, *J* = 13.4, 5.1 Hz, 1H), 4.57 (d, *J* = 4.6 Hz, 2H), 4.42 (d, *J* = 17.4 Hz, 1H), 4.29 (d, *J* = 17.3 Hz, 1H), 2.95-2.86 (m, 1H), 2.59-2.51 (m, 1H), 2.42-2.38 (m, 1H), 2.03-1.95 (m, 1H). Molecular formula: C18H15FN4O4S, calculated molecular weight: 402.40; found (LC-MS): 403.40 [M+1]⁺. |
| FDAB-45 | 11.00 (s, 1H), 9.38 (t, *J =* 6.0 Hz, 1H), 7.79 (d, *J =* 7.8 Hz, 1H), 7.67 (d, *J =* 8.4 Hz, 1H), 7.57 (dd, *J =* 13.0, 8.4 Hz, 3H), 7.48 (t, *J =* 7.8 Hz, 1H), 7.35 (t, *J =* 7.5 Hz, 1H), 5.12 (dd, *J =* 13.3, 5.1 Hz, 1H), 4.63 (*d, J* = 5.4 Hz, 2H), 4.58 (d, *J =* 17.4 Hz, 1H), 4.40 (d, *J =* 17.4 Hz, 1H), 2.96-2.86 (m, 1H), 2.64-2.57 (m, 1H), 2.45-2.39 (m, 1H), 2.07-1.96 (m, 1H). Molecular formula: C23H18FN3O5, calculated molecular weight: 435.41; found (LC-MS): 436.41 [M+1]⁺. |
| FDAB-46 | 11.00 (s, 1H), 9.43 (t, *J* = 5.9 Hz, 1H), 7.71 (dd, *J* = 9.1, 4.1 Hz, 1H), 7.66-7.46 (m, 4H), 7.33 (td, *J* = 9.2, 2.8 Hz, 1H), 5.12 (dd, *J =* 13.3, 5.1 Hz, 1H), 4.63 (d, *J =* 5.4 Hz, 2H), 4.58 (d, *J =* 17.5 Hz, 1H), 4.40 (d, *J* = 17.4 Hz, 1H), 2.96-2.87 (m, 1H), 2.62-2.55 (m, 1H), 2.46-2.38 (m, 1H), 2.04-1.94 (m, 1H). Molecular formula: C23H17F2N3O5, calculated molecular weight: 453.40; found (LC-MS): 454.40 [M+1]⁺. |
| FDAB-47 | 11.00 (s, 1H), 9.45 (t, *J* = 5.9 Hz, 1H), 7.89 (d, *J* = 2.2 Hz, 1H), 7.71 (d, *J* = 8.9 Hz, 1H), 7.57 (t, *J* = 3.6 Hz, 3H), 7.50 (dd, *J =* 8.8, 2.3 Hz, 1H), 5.12 (dd, *J =* 13.3, 5.0 Hz, 1H), 4.63 (d, *J =* 5.1 Hz, 2H), 4.58 (d, *J* = 17.4 Hz, 1H), 4.40 (d, *J* = 17.4 Hz, 1H), 2.96-2.87 (m, 1H), 2.62-2.58 (m, 1H), 2.46-2.42 (m, 1H), 2.04-1.94 (m, 1H). Molecular formula: C23H17C1FN3O5, calculated molecular weight: 469.85; found (LC-MS): 470.85 [M+1]⁺. |
| FDAB-48 | 11.02 (s, 1H), 9.23 (t, *J =* 5.8 Hz, 1H), 7.96-7.88 (m, 2H), 7.61-7.50 (m, 4H), 5.12 (dd, *J =* 13.3, 5.1 Hz, 1H), 4.61 (d, *J* = 6.2 Hz, 2H), 4.55 (s, 1H), 4.39 (d, *J* = 17.4 Hz, 1H), 2.96-2.87 (m, 1H), 2.63-2.56 (m, 1H), 2.44-2.39 (m, 1H), 2.04-1.95 (m, 1H). Molecular formula: C21H17ClFN3O4, calculated molecular weight: 429.83; found (LC-MS): 430.83 [M+1]⁺. |
| FDAB-49 | 11.02 (s, 1H), 9.17 (t, *J =* 5.8 Hz, 1H), 8.03-7.91 (m, 2H), 7.61-7.49 (m, 2H), 7.38-7.26 (m, 2H), 5.12 (dd, *J =* 13.3, 5.0 Hz, 1H), 4.66-4.53 (m, 3H), 4.39 (d, *J =* 17.4 Hz, 1H), 2.96-2.87 (m, 1H), 2.65-2.55 (m, 1H), 2.44-2.39 (m, 1H), 2.04-1.96 (m, 1H). Molecular formula: C21H17F2N3O4, calculated molecular weight: 413.38; found (LC-MS): 414.38 [M+1]⁺. |
| FDAB-50 | 11.04 (s, 1H), 9.25 (t, J = 5.8 Hz, 1H), 7.97-7.89 (m, 2H), 7.60-7.55 (m, 3H), 5.15 (dd, J = 13.3, 5.1 Hz, 1H), 4.63 (d, J = 6.2 Hz, 2H), 4.57 (s, 1H), 4.37 (d, J = 17.4 Hz, 1H), 2.96-2.90 (m, 1H), 2.63-2.57 (m, 1H), 2.45-2.39 (m, 1H), 2.07-1.95 (m, 1H). Molecular formula: C21H16C12FN3O4, calculated molecular weight: 464.27; found (LC-MS): 465.27 [M+1] ⁺. |
| FDAB-51 | 10.98 (s, 1H), 9.03 (t, *J =* 5.9 Hz, 1H), 7.81 (d, *J =* 7.9 Hz, 2H), 7.58 (d, *J =* 6.3 Hz, 1H), 7.52 (d, *J =* 8.9 Hz, 1H), 7.29 (d, *J =* 7.8 Hz, 2H), 5.10 (dd, *J =* 13.3, 5.1 Hz, 1H), 4.59 (d, *J =* 5.1 Hz, 2H), 4.42 (d, *J =* 17.3 Hz, 1H), 4.29 (d, *J* = 17.4 Hz, 1H), 2.95-2.86 (m, 1H), 2.63-2.56 (m, 1H), 2.40-2.30 (m, 4H), 2.05-1.95 (m, 1H). Molecular formula: C22H20FN3O4, calculated molecular weight: 409.42; found (LC-MS): 410.42 [M+1]⁺. |
| FDAB-52 | 10.99 (s, 1H), 9.38 (t, *J* = 5.8 Hz, 1H), 8.10 (d, *J* = 8.1 Hz, 2H), 7.88 (d, *J* = 8.2 Hz, 2H), 7.62 (d, *J* = 6.3 Hz, 1H), 7.54 (d, *J =* 8.9 Hz, 1H), 5.11 (dd, *J =* 13.3, 5.1 Hz, 1H), 4.62 (d, *J =* 5.7 Hz, 2H), 4.42 (d, *J =* 17.3 Hz, 1H), 4.29 (d, *J* = 17.3 Hz, 1H), 2.96-2.86 (m, 1H), 2.63-2.56 (m, 1H), 2.42-2.35 (m, 1H), 2.04-1.96 (m, 1H). Molecular formula: C22H17F4N3O4, calculated molecular weight: 463.39; found (LC-MS): 464.39 [M+1]⁺. |
| FDAB-53 | 10.99 (s, 1H), 9.28 (t, *J* = 5.7 Hz, 1H), 7.91 (dd, *J* = 10.4, 1.8 Hz, 1H), 7.82-7.70 (m, 2H), 7.62 (d, *J* = 6.3 Hz, 1H), 7.53 (d, *J =* 8.8 Hz, 1H), 5.10 (dd, *J =* 13.2, 5.1 Hz, 1H), 4.60 (d, *J =* 5.7 Hz, 2H), 4.42 (d, *J* = 17.3 Hz, 1H), 4.29 (d, *J* = 17.3 Hz, 1H), 2.96-2.86 (m, 1H), 2.61-2.57 (m, 1H), 2.41-2.34 (m, 1H), 2.04-1.97 (m, 1H). Molecular formula: C21H16C1F2N3O4, calculated molecular weight: 447.82; found (LC-MS): 448.82 [M+1]⁺. |
| FDAB-54 | 11.01 (s, 1H), 9.38 (t, *J* = 5.5 Hz, 1H), 8.46 (d, *J* = 2.2 Hz, 1H), 8.19 (dd, *J* = 8.5, 2.2 Hz, 1H), 7.90 (d, *J* = 8.5 Hz, 1H), 7.64 (d, *J =* 6.3 Hz, 1H), 7.54 (d, *J* = 8.9 Hz, 1H), 5.11 (dd, *J* = 13.3, 5.1 Hz, 1H), 4.61 (d, *J* = 4.3 Hz, 2H), 4.42 (d, *J =* 17.3 Hz, 1H), 4.29 (d, *J =* 17.2 Hz, 1H), 2.98-2.85 (m, 1H), 2.62-2.57 (m, 1H), 2.45-2.41 (m, 1H), 2.04-1.96 (m, 1H). Molecular formula: C22H16C1FN4O4, calculated molecular weight: 454.84; found (LC-MS): 455.84 [M+1]⁺. |
| FDAB-55 | 11.02 (s, 1H), 10.4 (s, 1H), 9.11 (d, *J* = 7.6 Hz, 1H), 7.65-7.39 (m, 4H), 7.32 (d, *J* = 7.8 Hz, 1H), 5.12 (dd, *J* = 13.0, 5.0 Hz, 1H), 4.65-4.52 (m, 3H), 4.39 (d, *J =* 17.3 Hz, 1H), 2.97-2.87 (m, 1H), 2.60-2.57 (m, 1H), 2.46-2.42 (m, 1H), 2.04-1.97 (m, 1H). Molecular formula: C21H17ClFN3O5, calculated molecular weight: 445.83; found (LC-MS): 446.83 [M+1]⁺. |
| FDAB-56 | 11.00 (s, 1H), 9.49 (s, 1H), 8.74 (s, 1H), 8.15 (dd, *J* = 8.5, 2.3 Hz, 1H), 8.06 (d, *J* = 8.6 Hz, 1H), 7.55 (d, *J =* 6.3 Hz, 1H), 7.52 (d, *J* = 8.9 Hz, 1H), 5.10 (dd, *J =* 13.4, 5.1 Hz, 1H), 4.62 (d, *J =* 4.6 Hz, 2H), 4.40 (d, *J =* 17.4 Hz, 1H), 4.26 (d, *J* = 17.3 Hz, 1H), 2.96-2.87 (m, 1H), 2.63-2.58 (m, 1H), 2.47-2.37 (m, 1H), 2.04-1.97 (m, 1H). Molecular formula: C20H16ClFN4O4, calculated molecular weight: 430.82; found (LC-MS): 431.82 [M+1]⁺. |
| FDAB-57 | 11.00 (s, 1H), 9.49 (t, *J* = 6.2 Hz, 1H), 8.53 (d, *J* = 2.4 Hz, 1H), 8.14 (dd, *J* = 8.4, 2.4 Hz, 1H), 8.05 (d, *J* = 8.4 Hz, 1H), 7.42-7.37 (m, 2H), 5.11 (dd, *J =* 13.3, 5.1 Hz, 1H), 4.64 *(d, J =* 6.2 Hz, 2H), 4.57 (d, *J =* 17.4 Hz, 1H), 4.39 (d, *J* = 17.4 Hz, 1H), 2.96-2.87 (m, 1H), 2.63-2.58 (m, 1H), 2.47-2.37 (m, 1H), 2.04-1.97 (m, 1H). Molecular formula: C20H16ClFN4O4, calculated molecular weight: 430.82; found (LC-MS): 431.82 [M+1]⁺. |
| FDAB-58 | 11.00 (s, 1H), 9.30 (t, *J* = 5.8 Hz, 1H), 8.46 (d, *J* = 1.7 Hz, 1H), 8.14 (d, *J* = 8.4 Hz, 1H), 7.93 (dd, *J* = 8.4, 1.7 Hz, 1H), 7.64 (d, *J* = 6.3 Hz, 1H), 7.54 (d, *J* = 8.9 Hz, 1H), 5.10 (dd, *J =* 13.3, 5.1 Hz, 1H), 4.64 *(d, J =* 5.7 Hz, 2H), 4.43 (d, *J =* 17.4 Hz, 1H), 4.29 (d, *J =* 17.3 Hz, 1H), 2.96-2.86 (m, 1H), 2.83 (s, 3H), 2.61-2.58 (m, 1H), 2.37-2.42 (m, 1H), 2.05-1.95 (m, 1H). Molecular formula: C23H19FN4O4S, calculated molecular weight: 466.49; found (LC-MS): 467.49 [M+1]⁺. |
| FDAB-59 | 11.00 (s, 1H), 9.30 (t, *J* = 5.8 Hz, 1H), 8.46 (d, *J =* 1.7 Hz, 1H), 8.14 (d, *J =* 8.4 Hz, 1H), 7.72-7.65 (m, 2H), 7.54 (d, *J =* 6.3 Hz, 1H), 7.54 (d, *J =* 8.9 Hz, 1H), 6.25 (s, 1H), 5.10 (dd, *J =* 13.3, 5.1 Hz, 1H), 4.64 (d, *J =* 5.7 Hz, 2H), 4.43 (d, *J =* 17.4 Hz, 1H), 4.29 (d, *J* = 17.3 Hz, 1H), 3.25 (s, 3H), 2.96-2.86 (m, 1H), 2.61-2.58 (m, 1H), 2.37-2.42 (m, 1H), 2.05-1.95 (m, 1H). Molecular formula: C24H21FN4O4, calculated molecular weight: 448.45; found (LC-MS): 449.45 [M+1]⁺. |
| FDAB-60 | 10.97 (s, 1H), 9.57 (t, *J* = 6.3 Hz, 1H), 8.59 (d, *J* = 8.5 Hz, 1H), 8.17 (dd, *J* = 8.5, 6.3 Hz, 2H), 8.10 (d, *J* = 8.2 Hz, 1H), 7.91-2.87(m, 1H), 7.74 (t, *J =* 7.5 Hz, 1H), 7.63 (d, *J =* 6.4 Hz, 1H), 7.54 (d, *J =* 8.9 Hz, 1H), 5.09 (dd, *J =* 13.3, 5.1 Hz, 1H), 4.71 (d, *J =* 5.7 Hz, 2H), 4.41 (d, *J =* 17.3 Hz, 1H), 4.27 (d, *J =* 17.4 Hz, 1H), 2.94-2.84 (m, 1H), 2.62-2.58 (m, 1H), 2.36-2.30 (m, 1H), 2.03-1.95 (m, 1H). Molecular formula: C24H19FN4O4, calculated molecular weight: 446.44; found (LC-MS): 447.44 [M+1]⁺. |
| FDAB-61 | 11.00 (s, 1H), 9.37 (t, *J* = 5.8 Hz, 1H), 8.09 (d, *J* = 8.1 Hz, 2H), 7.88 (d, *J* = 8.1 Hz, 2H), 7.57 (d, *J* = 4.8 Hz, 2H), 5.12 (dd, *J* = 13.3, 5.0 Hz, 1H), 4.64 (d, *J* = 5.6 Hz, 2H), 4.58 (d, *J* = 17.4 Hz, 1H), 4.40 (d, *J =* 17.4 Hz, 1H), 2.96-2.87 (m, 1H), 2.62-2.58 (m, 1H), 2.49-2.42 (m, 1H), 2.04-1.98 (m, 1H). Molecular formula: C22H17F4N3O4, calculated molecular weight: 463.39; found (LC-MS): 464.39 [M+1]⁺. |
| FDAB-62 | 11.00 (s, 1H), 9.49 (t, *J* = 6.2 Hz, 1H), 8.73 (d, *J* = 2.4 Hz, 1H), 8.14 (dd, *J* = 8.4, 2.4 Hz, 1H), 8.05 (d, *J* = 8.4 Hz, 1H), 7.58-7.48 (m, 2H), 5.11 (dd, *J =* 13.3, 5.1 Hz, 1H), 4.64 (d, *J =* 6.2 Hz, 2H), 4.57 (d, *J =* 17.4 Hz, 1H), 4.39 (d, *J* = 17.4 Hz, 1H), 2.96-2.87 (m, 1H), 2.63-2.58 (m, 1H), 2.47-2.37 (m, 1H), 2.04-1.97 (m, 1H). Molecular formula: C20H16ClFN4O4, calculated molecular weight: 430.82; found (LC-MS): 431.82 [M+1]⁺. |
| FDAB-63 | 11.01 (s, 1H), 9.12 (s, 1H), 7.52 (d, *J =* 2.3 Hz, 1H), 7.50 (d, *J =* 4.7 Hz, 1H), 5.11 (dd, *J =* 13.3, 5.1 Hz, 1H), 4.44 (d, *J* = 17.3 Hz, 1H), 4.39 (d, *J* = 3.9 Hz, 2H), 4.30 (d, *J* = 17.3 Hz, 1H), 2.95-2.86 (m, 1H), 2.63-2.56 (m, 1H), 2.40-2.32 (m, 3H), 2.04-1.97 (m, 1H), 1.11 (t, *J =* 7.5 Hz, 3H). Molecular formula: C19H18FN3O4, calculated molecular weight: 371.37; found (LC-MS): 372.37 [M+1]⁺. |
| FDAB-64 | 11.01 (s, 1H), 9.06 (t, *J* = 5.9 Hz, 1H), 7.52 (s, 1H), 7.50 (d, *J =* 3.1 Hz, 1H), 5.11 (dd, *J* = 13.3, 5.1 Hz, 1H), 4.44 (d, *J* = 17.3 Hz, 1H), 4.37 (d, *J* = 4.2 Hz, 2H), 4.30 (d, *J* = 17.3 Hz, 1H), 2.95-2.86 (m, 1H), 2.63-2.56 (m, 1H), 2.40-2.32 (m, 3H), 2.04-1.97 (m, 1H), 1.51-1.46 (m, 1H), 0.94-0.83 (m, 2H), 0.79-0.74 (m, 2H). Molecular formula: C20H18FN3O4, calculated molecular weight: 383.38; found (LC-MS): 384.38 [M+1]⁺. |
| FDAB-65 | 11.00 (s, 1H), 9.04 (t, *J* = 5.8 Hz, 1H), 7.80 (d, *J* = 7.9 Hz, 2H), 7.55 (h, *J* = 7.4, 6.9 Hz, 2H), 7.28 (d, *J* = 7.9 Hz, 2H), 5.11 (dd, *J =* 13.3, 5.0 Hz, 1H), 4.63-4.53 (m, 3H), 4.40 (d, *J =* 17.4 Hz, 1H), 2.96-2.87 (m, 1H), 2.63-2.58 (m, 1H), 2.47-2.37 (m, 1H), 2.36 (s, 3H), 2.05-1.97 (m, 1H). Molecular formula: C22H20FN304, calculated molecular weight: 409.42; found (LC-MS): 410.42 [M+1]⁺. |
| FDAB-66 | 11.00 (s, 1H), 9.60 (t, *J* = 6.2 Hz, 1H), 8.59 (d, *J* = 8.5 Hz, 1H), 8.17 (dd, *J* = 8.5, 4.8 Hz, 2H), 8.10 (d, *J* = 8.2 Hz, 1H), 7.89 (t, *J =* 7.7 Hz, 1H), 7.74 (t, *J* = 7.5 Hz, 1H), 7.61-7.53 (m, 2H), 5.12 (dd, *J* = 13.3, 5.1 Hz, 1H), 4.73 *(d, J =* 5.5 Hz, 2H), 4.59 (d, *J =* 17.4 Hz, 1H), 4.41 (d, *J* = 17.4 Hz, 1H), 2.96-2.87 (m, 1H), 2.62-2.58 (m, 1H), 2.47-2.37 (m, 1H), 2.04-1.97 (m, 1H). Molecular formula: C24H19FN4O4, calculated molecular weight: 446.44; found (LC-MS): 447.44 [M+1]⁺. |
| FDAB-67 | 11.03 (s, 1H), 9.50 (t, *J =* 5.9 Hz, 1H), 7.90 *(d, J=* 2.2 Hz, 1H), 7.73 (dd, *J =* 8.3, 2.9 Hz, 2H), 7.73-7.65 (m, 2H), 7.56 (d, *J* = 7.8 Hz, 1H), 7.51 (*dd, J =* 8.9, 2.2 Hz, 1H), 5.14 (dd, *J =* 13.2, 5.1 Hz, 1H), 4.67 (d, *J =* 5.0 Hz, 2H), 4.51 (d, *J =* 17.7 Hz, 1H), 4.34 (d, *J* = 17.6 Hz, 1H), 2.96-2.87 (m, 1H), 2.62-2.57 (m, 1H), 2.46-2.42 (m, 1H), 2.05-1.98 (m, 1H). Molecular formula: C23H18ClN3O5, calculated molecular weight: 451.86; found (LC-MS): 452.86 [M+1]⁺. |
| FDAB-68 | 11.03 (s, 1H), 9.50 (t, *J* = 5.9 Hz, 1H), 7.90 (d, *J* = 2.2 Hz, 1H), 7.73 (dd, *J* = 8.3, 2.9 Hz, 2H), 7.61 (s, 1H), 7.56 (d, *J* = 7.8 Hz, 1H), 7.51 (dd, *J* = 8.9, 2.2 Hz, 1H), 5.14 (dd, *J =* 13.2, 5.1 Hz, 1H), 4.67 (d, *J* = 5.0 Hz, 2H), 4.51 (d, *J* = 17.7 Hz, 1H), 4.34 (d, *J* = 17.6 Hz, 1H), 2.96-2.87 (m, 1H), 2.62-2.57 (m, 1H), 2.46-2.42 (m, 1H), 2.05-1.98 (m, 1H). Molecular formula: C23H17C12N3O5, calculated molecular weight: 486.31; found (LC-MS): 487.31 [M+1]⁺. |
| FDAB-69 | 10.95 (s, 1H), 9.51 (t, *J* = 5.9 Hz, 1H), 7.79-7.68 (m, 2H), 7.62 (d, *J* = 7.3 Hz, 2H), 7.56 (d, *J* = 7.9 Hz, 1H), 7.39-7.32 (m, 1H), 5.12 (dd, *J =* 12.9, 5.0 Hz, 1H), 4.66 (d, *J =* 5.0 Hz, 2H), 4.50 (d, *J =* 17.8 Hz, 1H), 4.34 (d, *J* = 17.8 Hz, 1H), 2.92-2.83 (m, 1H), 2.62-2.57 (m, 1H), 2.46-2.42 (m, 1H), 2.05-1.97 (m, 1H). Molecular formula: C23H17C1FN3O5, calculated molecular weight: 469.85; found (LC-MS): 470.85 [M+1]⁺. |
| FDAB-70 | 11.02 (s, 1H), 9.26 (t, *J* = 5.8 Hz, 1H), 7.97-7.92 (m, 2H), 7.71 (d, *J =* 7.8 Hz, 1H), 7.61-7.56 (m, 2H), 7.54 (d, *J* = 7.7 Hz, 1H), 5.14 (dd, *J =* 13.4, 5.1 Hz, 1H), 4.65 (d, *J =* 5.7 Hz, 2H), 4.51 (d, *J =* 17.7 Hz, 1H), 4.33 (d, *J* = 17.7 Hz, 1H), 2.96-2.87 (m, 1H), 2.60-2.57 (m, 1H), 2.46-2.42 (m, 1H), 2.05-1.97 (m, 1H). Molecular formula: C21H17C12N3O4, calculated molecular weight: 446.28; found (LC-MS): 447.28 [M+1]⁺. |
| FDAB-71 | 11.00 (s, 1H), 9.42 (t, *J=* 6.0 Hz, 1H), 7.80 (d, *J* = 10.2 Hz, 2H), 7.69 (d, *J* = 8.3 Hz, 1H), 7.66-7.61 (m, 2H), 7.53-7.47 (m, 1H), 7.40-7.32 (m, 1H), 5.10 (dd, *J =* 13.3, 5.1 Hz, 1H), 4.64 (d, *J =* 5.9 Hz, 2H), 4.44 (d, *J* = 17.7 Hz, 1H), 4.30 (d, *J =* 17.7 Hz, 1H), 2.97-2.86 (m, 1H), 2.61-2.55 (m, 1H), 2.47-2.41 (m, 1H), 2.03-1.96 (m, 1H). Molecular formula: C23H18ClN3O5, calculated molecular weight: 451.86; found (LC-MS): 452.86 [M+1]⁺. |
| FDAB-72 | 11.00 (s, 1H), 9.48 (t, *J* = 5.9 Hz, 1H), 7.91 (d, *J* = 2.2 Hz, 1H), 7.79 (s, 1H), 7.73 (d, *J* = 8.8 Hz, 1H), 7.63 (d, *J* = 12.6 Hz, 2H), 7.51 *(dd, J =* 8.9, 2.2 Hz, 1H), 5.10 (dd, *J = 13.3,* 5.1 Hz, 1H), 4.64 (d, *J* = 5.8 Hz, 2H), 4.44 (d, *J =* 17.8 Hz, 1H), 4.30 (d, *J =* 17.7 Hz, 1H), 2.95-2.84 (m, 1H), 2.60-2.58 (m, 1H), 2.41-2.36 (m, 1H), 2.03-1.96 (m, 1H). Molecular formula: C23H17C12N3O5, calculated molecular weight: 486.31; found (LC-MS): 487.31 [M+1]⁺. |
| FDAB-73 | 11.00 (s, 1H), 9.45 (t, *J* = 6.0 Hz, 1H), 7.78 (s, 1H), 7.73 (dd, *J* = 9.1, 4.1 Hz, 1H), 7.67-7.58 (m, 3H), 7.35 (td, *J =* 9.2, 2.8 Hz, 1H), 5.10 (dd, *J* = 13.2, 5.1 Hz, 1H), 4.64 (d, *J* = 4.3 Hz, 2H), 4.44 (d, *J* = 17.8 Hz, 1H), 4.30 (d, *J =* 17.8 Hz, 1H), 2.96-2.85 (m, 1H), 2.64-2.54 (m, 1H), 2.40-2.32 (m, 1H), 2.04-1.96 (m, 1H). Molecular formula: C23H17C1FN3O5, calculated molecular weight: 469.85; found (LC-MS): 470.85 [M+1]⁺. |
| FDAB-74 | 11.01 (s, 1H), 9.24 (t, *J* = 5.8 Hz, 1H), 7.99-7.91 (m, 2H), 7.78 (s, 1H), 7.64-7.55 (m, 3H), 5.10 (dd, *J* = 13.3, 5.1 Hz, 1H), 4.62 (d, *J* = 5.7 Hz, 2H), 4.44 (d, *J* = 17.7 Hz, 1H), 4.30 (d, *J* = 17.7 Hz, 1H), 2.96-2.87 (m, 1H), 2.60-2.57 (m, 1H), 2.42-2.36 (m, 1H), 2.03-1.96 (m, 1H). Molecular formula: C21H17C12N3O4, calculated molecular weight: 446.28; found (LC-MS): 447.28 [M+1]⁺. |
| FDAB-75 | 11.61 (s, 1H), 10.98 (s, 1H), 9.10 (t, *J* = 5.9 Hz, 1H), 7.79-7.62 (m, 2H), 7.64 (d, *J* = 8.8 Hz, 1H), 7.43 (d, *J* = 8.3 Hz, 1H), 7.29-7.14 (m, 2H), 7.04 (*t, J =* 7.5 Hz, 1H), 5.10 (dd, *J =* 13.2, 5.1 Hz, 1H), 4.64 (d, *J =* 5.8 Hz, 2H), 4.43 (d, *J =* 17.4 Hz, 1H), 4.29 (d, *J* = 17.4 Hz, 1H), 2.97-2.86 (m, 1H), 2.61-2.56 (m, 1H), 2.42-2.31 (m, 1H), 2.07-1.98 (m, 1H). Molecular formula: C23H19ClN4O4, calculated molecular weight: 450.88; found (LC-MS): 451.88 [M+1]⁺. |
| FDAB-76 | 11.02 (s, 1H), 9.10 (t, *J =* 5.8 Hz, 1H), 7.87-7.80 (m, 2H), 7.71 (d, *J =* 7.8 Hz, 1H), 7.52 (d, *J =* 7.8 Hz, 1H), 7.30 (d, *J =* 7.9 Hz, 2H), 5.14 (dd, *J =* 13.3, 5.0 Hz, 1H), 4.64 (d, *J =* 5.8 Hz, 2H), 4.51 (d, *J* = 17.7 Hz, 1H), 4.33 (d, *J =* 17.6 Hz, 1H), 2.97-2.87 (m, 1H), 2.60-2.57 (m, 1H), 2.46-2.42 (m, 1H), 2.37 (s, 3H), 2.05-1.98 (m, 1H). Molecular formula: C22H20ClN304, calculated molecular weight: 425.87; found (LC-MS): 426.87 [M+1]⁺. |
| FDAB-77 | 11.03 (s, 1H), 9.20 (t, *J* = 5.7 Hz, 1H), 8.04-7.95 (m, 2H), 7.72 (d, *J* = 7.8 Hz, 1H), 7.54 (d, *J* = 7.8 Hz, 1H), 7.34 (t, *J =* 8.8 Hz, 2H), 5.14 (dd, *J =* 13.3, 5.1 Hz, 1H), 4.65 (d, *J* = 4.5 Hz, 2H), 4.51 *(d, J =* 17.6 Hz, 1H), 4.33 (d, *J =* 17.6 Hz, 1H), 2.97-2.87 (m, 1H), 2.63-2.57 (m, 1H), 2.46-2.42 (m, 1H), 2.05-1.97 (m, 1H). Molecular formula: C21H17ClFN3O4, calculated molecular weight: 429.83; found (LC-MS): 430.83 [M+1]⁺. |
| FDAB-78 | 11.02 (s, 1H), 9.39 (d, *J* = 5.8 Hz, 1H), 8.60-8.52 (m, 1H), 8.19 (dd, *J* = 8.5, 2.2 Hz, 1H), 7.90 (d, *J =* 8.5 Hz, 1H), 7.58 (d, *J =* 3.0 Hz, 2H), 5.12 (dd, *J =* 13.3, 5.1 Hz, 1H), 4.63 (d, *J =* 5.6 Hz, 2H), 4.58 (d, *J* = 17.4 Hz, 1H), 4.40 (d, *J* = 17.4 Hz, 1H), 2.98-2.85 (m, 1H), 2.62-2.57 (m, 1H), 2.45-2.41 (m, 1H), 2.04-1.96 (m, 1H). Molecular formula: C22H16C12N4O4, calculated molecular weight: 471.29; found (LC-MS): 472.29 [M+1]⁺. |
| FDAB-79 | 11.02 (s, 1H), 10.2 (s, 1H), 9.11 (d, *J* = 7.6 Hz, 1H), 7.78-7.62 (m, 2H), 7.58-7.32 (m, 2H), 7.32 (d, *J* = 7.8 Hz, 1H), 5.12 (dd, *J* = 13.0, 5.0 Hz, 1H), 4.65-4.52 (m, 3H), 4.39 (d, *J* = 17.3 Hz, 1H), 2.97-2.87 (m, 1H), 2.60-2.57 (m, 1H), 2.46-2.42 (m, 1H), 2.04-1.97 (m, 1H). Molecular formula: C21H17C12N3O5, calculated molecular weight: 462.28; found (LC-MS): 463.28 [M+1]⁺. |
| FDAB-80 | 11.00 (s, 1H), 9.49 (t, *J* = 6.2 Hz, 1H), 8.89-8.80 (m, 1H), 8.02 (dd, *J* = 8.4, 2.4 Hz, 1H), 8.05 (d, *J* = 8.4 Hz, 1H), 7.58-7.48 (m, 2H), 5.11 (dd, *J* = 13.3, 5.1 Hz, 1H), 4.64 (d, *J* = 6.2 Hz, 2H), 4.53 (d, *J* = 17.4 Hz, 1H), 4.33 (d, *J =* 17.4 Hz, 1H), 2.96-2.87 (m, 1H), 2.63-2.58 (m, 1H), 2.47-2.37 (m, 1H), 2.04-1.97 (m, 1H). Molecular formula: C20H16C12N4O4, calculated molecular weight: 447.27; found (LC-MS): 448.27 [M+1]⁺. |
| FDAB-81 | 11.01 (s, 1H), 9.16 (t, *J* = 5.8 Hz, 1H), 7.93-7.87 (m, 2H), 7.60-7.51 (m, 3H), 7.49 (dd, *J* = 8.2, 6.5 Hz, 2H), 5.12 (dd, *J* = 13.3, 5.1 Hz, 1H), 4.66-4.60 (m, 2H), 4.56 (d, *J* = 17.4 Hz, 1H), 4.40 (d, *J* = 17.4 Hz, 1H), 2.98-2.85 (m, 1H), 2.64-2.56 (m, 1H), 2.45-2.41 (m, 1H), 2.04-1.96 (m, 1H). Molecular formula: C21H18FN3O4, calculated molecular weight: 395.39; found (LC-MS): 396.39 [M+1]⁺. |
| FDAB-82 | 11.02 (s, 1H), 9.39 (d, *J =* 5.8 Hz, 1H), 8.45 (d, *J* = 2.2 Hz, 1H), 8.19 (dd, *J =* 8.5, 2.2 Hz, 1H), 7.90 (d, *J =* 8.5 Hz, 1H), 7.58 (d, *J =* 3.0 Hz, 2H), 5.12 (dd, *J =* 13.3, 5.1 Hz, 1H), 4.63 (d, *J* = 5.6 Hz, 2H), 4.58 (d, *J =* 17.4 Hz, 1H), 4.40 (d, *J* = 17.4 Hz, 1H), 2.98-2.85 (m, 1H), 2.62-2.57 (m, 1H), 2.45-2.41 (m, 1H), 2.04-1.96 (m, 1H). Molecular formula: C22H16ClFN4O4, calculated molecular weight: 454.84; found (LC-MS): 455.84 [M+1]⁺. |
| FDAB-83 | 11.02 (s, 1H), 10.3 (s, 1H), 9.11 (d, *J* = 7.6 Hz, 1H), 7.65-7.39 (m, 4H), 7.32 (d, *J* = 7.8 Hz, 1H), 5.12 (dd, *J* = 13.0, 5.0 Hz, 1H), 4.65-4.52 (m, 3H), 4.39 (d, *J =* 17.3 Hz, 1H), 2.97-2.87 (m, 1H), 2.60-2.57 (m, 1H), 2.46-2.42 (m, 1H), 2.04-1.97 (m, 1H). Molecular formula: C21H17ClFN3O5, calculated molecular weight: 445.83; found (LC-MS): 446.83 [M+1]⁺. |
| FDAB-84 | 11.02 (s, 1H), 9.22 (t, *J =* 5.8 Hz, 1H), 8.04-7.97 (m, 2H), 7.83-7.77 (m, 2H), 7.77-7.71 (m, 2H), 7.61-7.53 (m, 2H), 7.50 (dd, *J* = 8.4, 6.9 Hz, 2H), 7.44-7.39 (m, 1H), 5.12 (dd, *J* = 13.3, 5.1 Hz, 1H), 4.64 (d, *J =* 5.5 Hz, 2H), 4.59 (d, *J =* 17.4 Hz, 1H), 4.40 (d, *J* = 17.4 Hz, 1H), 2.97-2.87 (m, 1H), 2.62-2.57 (m, 1H), 2.46-2.42 (m, 1H), 2.04-1.97 (m, 1H). Molecular formula: C27H22FN304, calculated molecular weight: 471.49; found (LC-MS): 472.49 [M+1]⁺. |
| FDAB-85 | 11.02 (s, 1H), 9.00 (t, *J =* 5.9 Hz, 1H), 7.93-7.82 (m, 2H), 7.57 (d, *J* = 7.7 Hz, 1H), 7.52 (t, *J =* 7.1 Hz, 1H), 7.05-6.98 (m, 2H), 5.12 (dd, *J* = 13.3, 5.1 Hz, 1H), 4.62-4.53 (m, 3H), 4.39 (d, *J =* 17.4 Hz, 1H), 3.81 (s, 3H), 2.96-2.87 (m, 1H), 2.62-2.58 (m, 1H), 2.46-2.42 (m, 1H), 2.04-1.96 (m, 1H). Molecular formula: C22H20FN305, calculated molecular weight: 425.42; found (LC-MS): 426.42 [M+1]⁺. |
| FDAB-86 | 11.02 (s, 1H), 8.78 (s, 1H), 7.77 (d, *J* = 8.6 Hz, 2H), 7.56 (d, *J* = 7.7 Hz, 1H), 7.53-7.48 (m, 1H), 6.71 (d, *J =* 8.6 Hz, 2H), 5.12 (dd, *J =* 13.3, 5.1 Hz, 1H), 4.65-4.51 (m, 3H), 4.39 (d, *J =* 17.4 Hz, 1H), 2.97 (s, 6H), 2.94-2.88 (m, 1H), 2.62-2.57 (m, 1H), 2.46-2.42 (m, 1H), 2.06-1.95 (m, 1H). Molecular formula: C23H23FN404, calculated molecular weight: 438.46; found (LC-MS): 439.46 [M+1]⁺. |
| FDAB-87 | 11.03 (s, 1H), 9.38 (t, *J =* 5.8 Hz, 1H), 8.72 (d, *J =* 8.3 Hz, 2H), 8.64 (s, 1H), 8.21-8.10 (m, 3H), 7.95 (dd, *J* = 8.9, 1.7 Hz, 1H), 7.67-7.52 (m, 4H), 5.13 (dd, *J =* 13.3, 5.0 Hz, 1H), 4.70 (d, *J =* 4.5 Hz, 2H), 4.60 (d, *J* = 17.4 Hz, 1H), 4.42 (d, *J* = 17.4 Hz, 1H), 2.99-2.89 (m, 1H), 2.62-2.57 (m, 1H), 2.46-2.42 (m, 1H), 2.05-1.98 (m, 1H). Molecular formula: C29H22FN3O4, calculated molecular weight: 495.51; found (LC-MS): 496.51 [M+1]⁺. |
| FDAB-88 | 11.02 (s, 1H), 9.15 (t, *J* = 5.8 Hz, 1H), 7.61-7.46 (m, 2H), 5.12 (dd, *J* = 13.3, 5.1 Hz, 1H), 4.65-4.53 (m, 3H), 4.40 (d, *J* = 17.4 Hz, 1H), 2.96-2.87 (m, 1H), 2.63-2.57 (m, 1H), 2.46-2.42 (m, 1H), 2.05-1.96 (m, 1H). Molecular formula: C21H13D5FN3O4, calculated molecular weight: 400.42; found (LC-MS): 401.42 [M+1]⁺. |
| FDAB-89 | 11.02 (s, 1H), 9.09 (t, *J* = 5.8 Hz, 1H), 7.72 (s, 1H), 7.71-7.64 (m, 1H), 7.58 (d, *J* = 7.7 Hz, 1H), 7.55-7.49 (m, 1H), 7.42-7.29 (m, 2H), 5.12 (dd, *J =* 13.4, 5.1 Hz, 1H), 4.65-4.51 (m, 3H), 4.40 (d, *J =* 17.4 Hz, 1H), 2.96-2.87 (m, 1H), 2.63-2.57 (m, 1H), 2.46-2.42 (m, 1H), 2.36 (s, 3H), 2.04-1.97 (m, 1H). Molecular formula: C22H20FN3O4, calculated molecular weight: 409.42; found (LC-MS): 410.42 [M+1]⁺. |
| FDAB-90 | 11.02 (s, 1H), 9.07 (t, *J* = 5.8 Hz, 1H), 7.86-7.80 (m, 2H), 7.57 (d, *J =* 7.7 Hz, 1H), 7.54-7.48 (m, 1H), 7.32 (d, *J =* 8.1 Hz, 2H), 5.12 (dd, *J* = 13.4, 5.1 Hz, 1H), 4.63-4.53 (m, 3H), 4.39 (d, *J* = 17.4 Hz, 1H), 2.96-2.87 (m, 1H), 2.69-2.57 (m, 3H), 2.46-2.41 (m, 1H), 2.04-1.96 (m, 1H), 1.19 (t, *J =* 7.6 Hz, 3H). Molecular formula: C23H22FN3O4, calculated molecular weight: 423.44; found (LC-MS): 424.44 [M+1]⁺. |
| FDAB-91 | 11.02 (s, 1H), 9.07 (t, *J* = 5.8 Hz, 1H), 7.86-7.78 (m, 2H), 7.57 (d, *J =* 7.7 Hz, 1H), 7.54-7.48 (m, 1H), 7.30 (d, *J =* 8.1 Hz, 2H), 5.12 (dd, *J* = 13.3, 5.0 Hz, 1H), 4.57 (d, *J =* 17.3 Hz, 3H), 4.39 (d, *J* = 17.4 Hz, 1H), 2.97-2.87 (m, 1H), 2.65-2.55 (m, 3H), 2.46-2.41 (m, 1H), 2.04-1.96 (m, 1H), 1.65-1.56 (m, 2H), 0.89 (t, *J* = 7.3 Hz, 3H). Molecular formula: C24H24FN3O4, calculated molecular weight: 437.47; found (LC-MS): 438.47 [M+1]⁺. |
| FDAB-92 | 10.83 (s, 1H), 8.96 (t, *J* = 5.8 Hz, 1H), 7.57 (d, *J* = 7.7 Hz, 1H), 7.54 (s, 2H), 7.51 (t, *J* = 7.0 Hz, 1H), 5.12 (dd, *J =* 13.3, 5.1 Hz, 1H), 4.57 (t, *J* = 8.7 Hz, 3H), 4.39 (d, *J* = 17.4 Hz, 1H), 2.96-2.86 (m, 1H), 2.65-2.55 (m, 1H), 2.46-2.40 (m, 1H), 2.28 (s, 6H), 2.17 (s, 3H), 2.04-1.96 (m, 1H). Molecular formula: C24H24FN304, calculated molecular weight: 437.47; found (LC-MS): 438.47 [M+1]⁺. |
| FDAB-93 | 11.02 (s, 1H), 9.07 (t, *J =* 5.8 Hz, 1H), 7.87-7.80 (m, 2H), 7.57 (d, *J* = 7.7 Hz, 1H), 7.52 (t, *J =* 7.0 Hz, 1H), 7.38-7.32 (m, 2H), 5.12 (dd, *J* = 13.3, 5.1 Hz, 1H), 4.65-4.53 (m, 3H), 4.39 (d, *J* = 17.4 Hz, 1H), 2.98-2.85 (m, 2H), 2.62-2.57 (m, 1H), 2.46-2.41 (m, 1H), 2.04-1.97 (m, 1H), 1.21 (d, *J* = 6.9 Hz, 6H). Molecular formula: C24H24FN3O4, calculated molecular weight: 437.47; found (LC-MS): 438.47 [M+1]⁺. |
| FDAB-94 | 11.02 (s, 1H), 9.08 (t, *J* = 5.9 Hz, 1H), 7.88-7.81 (m, 2H), 7.57 (d, *J =* 7.7 Hz, 1H), 7.54-7.46 (m, 3H), 5.12 (dd, *J* = 13.3, 5.1 Hz, 1H), 4.63-4.53 (m, 3H), 4.39 (d, *J* = 17.3 Hz, 1H), 2.96-2.87 (m, 1H), 2.64-2.55 (m, 1H), 2.46-2.40 (m, 1H), 2.04-1.98 (m, 1H), 1.30 (s, 9H). Molecular formula: C25H26FN3O4, calculated molecular weight: 451.50; found (LC-MS): 452.50 [M+1]⁺. |
| FDAB-95 | 11.02 (s, 1H), 9.14 (t, *J =* 5.8 Hz, 1H), 7.92-7.85 (m, 2H), 7.63-7.56 (m, 3H), 7.56-7.51 (m, 1H), 6.80 (dd, *J* = 17.7, 11.0 Hz, 1H), 5.97 (d, *J =* 17.7 Hz, 1H), 5.38 (d, *J =* 11.0 Hz, 1H), 5.12 (dd, *J =* 13.4, 5.0 Hz, 1H), 4.65-4.53 (m, 3H), 4.40 (d, *J =* 17.4 Hz, 1H), 2.97-2.86 (m, 1H), 2.62-2.56 (m, 1H), 2.45-2.41 (m, 1H), 2.04-1.97 (m, 1H). Molecular formula: C23H20FN3O4, calculated molecular weight: 421.43; found (LC-MS): 422.43 [M+1]⁺. |
| FDAB-96 | 9.24 (t, *J =* 5.8 Hz, 1H), 7.93-7.87 (m, 2H), 7.62-7.57 (m, 2H), 7.57-7.48 (m, 2H), 5.12 (dd, *J =* 13.3, 5.1 Hz, 1H), 4.63-4.55 (m, 3H), 4.44-4.35 (m, 2H), 2.96-2.87 (m, 1H), 2.62-2.57 (m, 1H), 2.46-2.42 (m, 1H), 2.04-1.97 (m, 1H). Molecular formula: C23H18FN3O4, calculated molecular weight: 419.41; found (LC-MS): 420.41 [M+1]⁺. |
| FDAB-97 | 11.01 (s, 1H), 9.04 (t, *J* = 5.8 Hz, 1H), 7.82-7.75 (m, 2H), 7.57 (d, *J* = 7.7 Hz, 1H), 7.51 (t, *J* = 6.9 Hz, 1H), 7.20-7.13 (m, 2H), 5.12 (dd, *J* = 13.3, 5.0 Hz, 1H), 4.65-4.53 (m, 3H), 4.39 (d, *J* = 17.4 Hz, 1H), 2.96-2.87 (m, 1H), 2.62-2.57 (m, 1H), 2.45-2.41 (m, 1H), 2.08-1.94 (m, 2H), 1.05-0.96 (m, 2H), 0.78-0.69 (m, 2H). Molecular formula: C24H22FN3O4, calculated molecular weight: 435.46; found (LC-MS): 436.46 [M+1]⁺. |
| FDAB-98 | 11.02 (s, 1H), 9.03 (t, *J* = 5.8 Hz, 1H), 7.76 (dd, *J* = 7.6, 1.5 Hz, 1H), 7.61 (s, 1H), 7.57 (d, *J* = 7.7 Hz, 1H), 7.52 (t, *J* = 7.0 Hz, 1H), 7.18 (d, *J =* 7.7 Hz, 1H), 5.12 (dd, *J =* 13.3, 5.0 Hz, 1H), 4.65-4.53 (m, 3H), 4.39 (d, *J =* 17.4 Hz, 1H), 3.17 (s, 4H), 2.97-2.86 (m, 1H), 2.62-2.57 (m, 1H), 2.45-2.41 (m, 1H), 2.04-1.96 (m, 1H). Molecular formula: C23H20FN3O4, calculated molecular weight: 421.43; found (LC-MS): 422.43 [M+1]⁺. |
| FDAB-99 | 11.02 (s, 1H), 9.33 (t, *J =* 5.7 Hz, 1H), 8.08-7.98 (m, 4H), 7.62-7.51 (m, 2H), 5.12 (dd, *J =* 13.4, 5.0 Hz, 1H), 4.63 (d, *J* = 5.7 Hz, 2H), 4.58 (d, *J* = 17.4 Hz, 1H), 4.40 (d, *J* = 17.4 Hz, 1H), 2.96-2.86 (m, 1H), 2.62 (s, 3H), 2.61-2.57 (m, 1H), 2.45-2.41 (m, 1H), 2.04-1.98 (m, 1H). Molecular formula: C23H20FN3O5, calculated molecular weight: 437.43; found (LC-MS): 438.43 [M+1]⁺. |
| FDAB-100 | 11.02 (s, 1H), 9.36 (t, *J =* 5.7 Hz, 1H), 8.09-8.03 (m, 2H), 7.86-7.67 (m, 5H), 7.63-7.53 (m, 4H), 5.13 (dd, *J =* 13.3, 5.1 Hz, 1H), 4.65 (d, *J =* 4.3 Hz, 2H), 4.59 (d, *J =* 17.4 Hz, 1H), 4.41 (d, *J =* 17.4 Hz, 1H), 2.97-2.85 (m, 1H), 2.65-2.56 (m, 1H), 2.45-2.41 (m, 1H), 2.05-1.97 (m, 1H). Molecular formula: C28H22FN3O5, calculated molecular weight: 499.50; found (LC-MS): 500.50 [M+1]⁺. |
| FDAB-101 | 11.02 (s, 1H), 9.10 (t, *J =* 5.8 Hz, 1H), 7.97-7.89 (m, 2H), 7.61-7.49 (m, 2H), 7.49-7.39 (m, 2H), 7.26-7.17 (m, 1H), 7.13-6.99 (m, 4H), 5.12 (dd, *J =* 13.3, 5.1 Hz, 1H), 4.63-4.53 (m, 3H), 4.39 (d, *J =* 17.4 Hz, 1H), 2.97-2.87 (m, 1H), 2.64-2.54 (m, 1H), 2.47-2.42 (m, 1H), 2.04-1.96 (m, 1H). Molecular formula: C27H22FN3O5, calculated molecular weight: 487.49; found (LC-MS): 488.49 [M+1]⁺. |
| FDAB-102 | 11.02 (s, 1H), 9.01 (t, *J =* 5.8 Hz, 1H), 7.58 (d, *J =* 7.7 Hz, 1H), 7.56-7.50 (m, 2H), 7.49 (d, *J =* 2.1 Hz, 1H), 7.04 (d, *J =* 8.5 Hz, 1H), 5.12 (dd, *J =* 13.3, 5.1 Hz, 1H), 4.58 (d, *J =* 16.7 Hz, 3H), 4.40 (d, *J =* 17.3 Hz, 1H), 3.80 (d, *J =* 3.0 Hz, 6H), 2.97-2.87 (m, 1H), 2.65-2.55 (m, 1H), 2.46-2.40 (m, 1H), 2.04-1.96 (m, 1H). Molecular formula: C23H22FN3O6, calculated molecular weight: 455.44; found (LC-MS): 456.44 [M+1]⁺. |
| FDAB-103 | 11.02 (s, 1H), 9.04 (t, *J =* 5.8 Hz, 1H), 7.88-7.80 (m, 2H), 7.57 (d, *J =* 7.7 Hz, 1H), 7.56-7.48 (m, 1H), 7.09-7.01 (m, 2H), 5.12 (dd, *J =* 13.3, 5.1 Hz, 1H), 4.65-4.53 (m, 3H), 4.39 (d, *J =* 17.4 Hz, 1H), 2.96-2.87 (m, 1H), 2.64-2.55 (m, 1H), 2.48-2.37 (m, 1H), 2.04-1.96 (m, 1H), 1.34 (s, 9H). Molecular formula: C25H26FN3O5, calculated molecular weight: 467.50; found (LC-MS): 468.50 [M+1]⁺. |
| FDAB-104 | 11.02 (s, 1H), 8.76 (t, *J* = 6.0 Hz, 1H), 7.69 (d, *J* = 7.8 Hz, 1H), 7.64-7.48 (m, 2H), 6.99 (s, 1H), 6.86 (d, *J =* 7.9 Hz, 1H), 5.12 (dd, *J =* 13.4, 5.1 Hz, 1H), 4.63 (d, *J =* 4.7 Hz, 2H), 4.58 (d, *J =* 17.4 Hz, 1H), 4.39 (d, *J* = 17.3 Hz, 1H), 3.91 (s, 3H), 2.97-2.87 (m, 1H), 2.64-2.55 (m, 1H), 2.46-2.41 (m, 1H), 2.35 (s, 3H), 2.05-1.97 (m, 1H). Molecular formula: C23H22FN3O5, calculated molecular weight: 439.44; found (LC-MS): 440.44 [M+1]⁺. |
| FDAB-105 | 8.87 (t, *J =* 5.9 Hz, 1H), 7.63-7.53 (m, 2H), 7.35 (d, *J =* 7.8 Hz, 1H), 7.12 (d, *J =* 8.1 Hz, 2H), 5.12 (dd, *J* = 13.3, 5.1 Hz, 1H), 4.63-4.48 (m, 3H), 4.40 (d, *J* = 17.4 Hz, 1H), 2.97-2.86(m, 1H), 2.64-2.56 (m, 1H), 2.48 (s, 3H), 2.46-2.41 (m, 1H), 2.32 (s, 3H), 2.07-1.96 (m, 1H). Molecular formula: C23H22FN3O4S, calculated molecular weight: 455.50; found (LC-MS): 456.50 [M+1]⁺. |
| FDAB-106 | 10.95 (s, 1H), 9.35 (t, *J =* 5.9 Hz, 1H), 8.23-8.15 (m, 2H), 7.70 (d, *J =* 8.0 Hz, 1H), 7.62-7.52 (m, 2H), 5.12 (dd, *J =* 13.4, 5.1 Hz, 1H), 4.62 (d, *J =* 5.7 Hz, 2H), 4.58 (d, *J =* 17.5 Hz, 1H), 4.40 (d, *J =* 17.4 Hz, 1H), 3.20-3.13 (m, 2H), 2.97-2.86 (m, 1H), 2.75-2.67 (m, 2H), 2.62-2.57 (m, 1H), 2.04-1.96 (m, 1H). Molecular formula: C24H20FN3O5, calculated molecular weight: 449.44; found (LC-MS): 450.44 [M+1]⁺. |
| FDAB-107 | 11.03 (s, 1H), 9.42 (t, *J =* 5.8 Hz, 1H), 8.12 (d, *J =* 8.5 Hz, 2H), 8.04 (d, *J =* 8.5 Hz, 2H), 7.62-7.51 (m, 2H), 5.12 (dd, *J =* 13.4, 5.1 Hz, 1H), 4.64 (d, *J =* 5.0 Hz, 2H), 4.58 (d, *J =* 17.4 Hz, 1H), 4.40 (d, *J =* 17.4 Hz, 1H), 3.27 (s, 3H), 2.99-2.85 (m, 1H), 2.62-2.57 (m, 1H), 2.45-2.42 (m, 1H), 2.04-1.97 (m, 1H). Molecular formula: C22H20FN3O6S, calculated molecular weight: 473.48; found (LC-MS): 474.48 [M+1]⁺. |
| FDAB-108 | 11.02 (s, 1H), 9.10 (t, *J =* 5.9 Hz, 1H), 7.86 (d, *J =* 8.1 Hz, 2H), 7.61-7.49 (m, 2H), 7.41 (d, *J =* 8.1 Hz, 2H), 5.32 (t, *J =* 5.7 Hz, 1H), 5.12 (dd, *J =* 13.3, 5.1 Hz, 1H), 4.61 (d, *J =* 5.7 Hz, 2H), 4.59-4.51 (m, 3H), 4.39 (d, *J =* 17.4 Hz, 1H), 2.97-2.87 (m, 1H), 2.62-2.57 (m, 1H), 2.46-2.43 (m, 1H), 2.04-1.96 (m, 1H). Molecular formula: C22H20FN3O5, calculated molecular weight: 425.42; found (LC-MS): 426.42 [M+1]⁺. |
| FDAB-109 | 12.34 (d, *J =* 4.9 Hz, 1H), 11.02 (s, 1H), 9.33 (d, *J =* 3.5 Hz, 1H), 7.79 (d, *J =* 8.5 Hz, 1H), 7.58 (d, *J =* 7.7 Hz, 1H), 7.56-7.50 (m, 1H), 6.73 (d, *J =* 5.9 Hz, 2H), 5.12 (dd, *J =* 13.3, 5.1 Hz, 1H), 4.63 (s, 2H), 4.58 (d, *J =* 17.3 Hz, 1H), 4.40 (d, *J =* 17.4 Hz, 1H), 2.98-2.85 (m, 1H), 2.64-2.55 (m, 1H), 2.46-2.40 (m, 1H), 2.28 (s, 3H), 2.04-1.95 (m, 1H). Molecular formula: C22H20FN3O5, calculated molecular weight: 425.42; found (LC-MS): 426.42 [M+1]⁺. |
| FDAB-110 | 11.02 (s, 1H), 8.74 (t, *J =* 5.9 Hz, 1H), 7.84-7.68 (m, 2H), 7.56 (d, *J =* 7.7 Hz, 1H), 7.50 (t, *J =* 7.0 Hz, 1H), 6.54 (d, *J=* 8.8 Hz, 2H), 5.12 (dd, *J =* 13.4, 5.1 Hz, 1H), 4.57 (dd, *J=* 11.5, 6.0 Hz, 3H), 4.39 (d, *J* = 17.4 Hz, 1H), 3.29-3.26 (m, 4H), 2.96-2.87 (m, 1H), 2.64-2.55 (m, 1H), 2.46-2.41 (m, 1H), 2.04-1.89 (m, 5H). Molecular formula: C25H25FN4O4, calculated molecular weight: 464.50; found (LC-MS): 465.50 [M+1]⁺. |
| FDAB-111 | 10.99 (s, 1H), 8.78 (t, *J =* 5.8 Hz, 1H), 7.57 (d, *J =* 7.7 Hz, 1H), 7.50 (t, *J =* 7.9 Hz, 2H), 6.50 (s, 1H), 6.44 (d, *J=* 7.8 Hz, 2H), 6.38-6.32 (m, 1H), 5.12 (dd, *J =* 13.3, 5.1 Hz, 1H), 4.65-4.51 (m, 3H), 4.39 (d, *J =* 17.3 Hz, 1H), 2.98-2.85 (m, 1H), 2.62-2.57 (m, 1H), 2.45-2.42 (m, 1H), 2.17 (s, 3H), 2.04-1.96 (m, 1H). Molecular formula: C22H21FN4O4, calculated molecular weight: 424.43; found (LC-MS): 425.43 [M+1]⁺. |
| FDAB-112 | 11.02 (s, 1H), 8.88 (t, *J =* 6.1 Hz, 1H), 7.57 (d, *J =* 7.7 Hz, 1H), 7.49 (t, *J =* 7.0 Hz, 1H), 7.19 (s, 1H), 7.09 (d, *J=* 8.2 Hz, 1H), 7.03 (d, *J =* 7.7 Hz, 1H), 5.50 (brs, 2H), 5.12 (dd, *J =* 13.2, 5.0 Hz, 1H), 4.62-4.53 (m, 3H), 4.39 (d, *J =* 17.4 Hz, 1H), 2.96-2.87 (m, 1H), 2.62-2.57 (m, 1H), 2.45-2.42 (m, 1H), 2.11 (s, 3H), 2.04-1.96 (m, 1H). Molecular formula: C22H21FN4O4, calculated molecular weight: 424.43; found (LC-MS): 425.43 [M+1]⁺. |
| FDAB-113 | 11.02 (s, 1H), 9.01 (t, *J =* 5.8 Hz, 1H), 7.57 (d, *J =* 7.8 Hz, 1H), 7.50 (t, *J =* 7.0 Hz, 1H), 7.32-7.24 (m, 2H), 7.04 (dd, *J =* 8.3, 2.1 Hz, 1H), 5.56 (brs, 2H), 5.12 (dd, *J =* 13.3, 5.1 Hz, 1H), 4.62-4.53 (m, 3H), 4.39 (d, *J =* 17.4 Hz, 1H), 2.96-2.87 (m, 1H), 2.62-2.57 (m, 1H), 2.46-2.41 (m, 1H), 2.04-1.96 (m, 1H). Molecular formula: C21H18ClFN4O4, calculated molecular weight: 444.85; found (LC-MS): 445.85 [M+1]⁺. |
| FDAB-114 | 11.02 (s, 1H), 9.14 (t, *J =* 5.7 Hz, 1H), 7.78 (d, *J =* 8.3 Hz, 1H), 7.64-7.50 (m, 2H), 7.30-7.22 (m, 1H), 7.01 (dd, *J =* 8.3, 1.9 Hz, 1H), 6.85 (s, 2H), 5.12 (dd, *J =* 13.3, 5.0 Hz, 1H), 4.60-4.56 (m, 3H), 4.40 (d, *J* = 17.4 Hz, 1H), 3.17 (s, 3H), 2.99-2.86 (m, 1H), 2.63-2.57 (m, 1H), 2.46-2.41 (m, 1H), 2.04-1.96 (m, 1H). Molecular formula: C22H21FN4O6S, calculated molecular weight: 488.49; found (LC-MS): 489.49 [M+1]⁺. |
| FDAB-115 | 11.02 (s, 1H), 9.46 (*t, J =* 5.7 Hz, 1H), 8.50 (d, *J =* 2.1 Hz, 1H), 8.10 (dd, *J* = 8.3, 2.2 Hz, 1H), 7.79 (d, *J* = 8.3 Hz, 1H), 7.72 (s, 2H), 7.62-7.50 (m, 2H), 5.12 (dd, *J =* 13.3, 5.1 Hz, 1H), 4.63 (d, *J =* 5.6 Hz, 2H), 4.58 (d, *J =* 17.5 Hz, 1H), 4.40 (d, *J =* 17.4 Hz, 1H), 2.97-2.85 (m, 1H), 2.64-2.57 (m, 1H), 2.46-2.42 (m, 1H), 2.04-1.98 (m, 1H). Molecular formula: C21H18ClFN4O6S, calculated molecular weight: 508.91; found (LC-MS): 509.91 [M+1]⁺. |
| FDAB-116 | 11.02 (s, 1H), 9.30 (t, *J =* 5.7 Hz, 1H), 7.90 (dd, *J =* 10.2, 1.8 Hz, 1H), 7.81-7.70 (m, 2H), 7.61-7.51 (m, 2H), 5.12 (dd, *J =* 13.3, 5.0 Hz, 1H), 4.66-4.51 (m, 3H), 4.40 (d, *J =* 17.4 Hz, 1H), 2.97-2.87(m, 1H), 2.64-2.56 (m, 1H), 2.46-2.41 (m, 1H), 2.04-1.95 (m, 1H). Molecular formula: C21H16ClF2N3O4, calculated molecular weight: 447.82; found (LC-MS): 448.82 [M+1]⁺. |
| FDAB-117 | 11.03 (s, 1H), 9.06 (t, *J =* 5.7 Hz, 1H), 7.68 (t, *J =* 8.1 Hz, 1H), 7.63-7.52 (m, 3H), 7.40 (dd, *J =* 8.3, 2.0 Hz, 1H), 5.12 (dd, *J =* 13.2, 5.1 Hz, 1H), 4.58 (d, *J =* 15.8 Hz, 3H), 4.40 (d, *J =* 17.4 Hz, 1H), 2.97-2.86 (m, 1H), 2.64-2.56 (m, 1H), 2.45-2.41(m, 1H), 2.04-1.96 (m, 1H). Molecular formula: C21H16ClF2N3O4, calculated molecular weight: 447.82; found (LC-MS): 448.82 [M+1]⁺. |
| FDAB-118 | 11.03 (s, 1H), 9.33 (t, *J =* 5.7 Hz, 1H), 7.93 (dd, *J =* 10.2, 1.9 Hz, 1H), 7.84-7.68 (m, 3H), 7.56 (d, *J =* 7.8 Hz, 1H), 5.14 (dd, *J =* 13.3, 5.0 Hz, 1H), 4.66 (d, *J =* 4.1 Hz, 2H), 4.51 (d, *J =* 17.7 Hz, 1H), 4.34 (d, *J* = 17.7 Hz, 1H), 2.97-2.87 (m, 1H), 2.64-2.55 (m, 1H), 2.45-2.41 (m, 1H), 2.05-1.97 (m, 1H). Molecular formula: C21H16Cl2FN3O4, calculated molecular weight: 464.27; found (LC-MS): 465.27 [M+1]⁺. |
| FDAB-119 | 11.03 (s, 1H), 9.20-8.97 (m, 1H), 7.79-7.67 (m, 2H), 7.65-7.55 (m, 2H), 7.41 (dd, *J =* 8.3, 2.0 Hz, 1H), 5.14 (dd, *J =* 13.3, 5.1 Hz, 1H), 4.64 (d, *J =* 4.0 Hz, 2H), 4.51 (d, *J* = 17.7 Hz, 1H), 4.34 (d, *J =* 17.7 Hz, 1H), 2.98-2.88 (m, 1H), 2.64-2.55 (m, 1H), 2.45-2.41 (m, 1H), 2.05-1.98 (m, 1H). Molecular formula: C21H16Cl2FN3O4, calculated molecular weight: 464.27; found (LC-MS): 465.27 [M+1]⁺. |
| FDAB-120 | 11.02 (s, 1H), 9.16 (t, *J =* 5.9 Hz, 1H), 7.69-7.63(m, 2H), 7.61-7.49 (m, 2H), 7.41 (t, *J =* 8.0 Hz, 1H), 5.12 (dd, *J =* 13.3, 5.1 Hz, 1H), 4.57 (*d, J =* 17.4 Hz, 3H), 4.39 (d, *J =* 17.4 Hz, 1H), 2.96-2.86 (m, 1H), 2.63-2.56 (m, 1H), 2.45-2.41 (m, 1H), 2.28 (s, 3H), 2.04-1.96 (m, 1H). Molecular formula: C22H19F2N3O4, calculated molecular weight: 427.41; found (LC-MS): 428.41 [M+1]⁺. |
| FDAB-121 | 11.02 (s, 1H), 8.87 (s, 1H), 7.60-7.53 (m, 3H), 7.13 (dd, *J =* 15.8, 10.0 Hz, 2H), 5.12 (dd, *J =* 13.3, 5.1 Hz, 1H), 4.65-4.53 (m, 3H), 4.40 (d, *J =* 17.4 Hz, 1H), 2.98-2.87 (m, 1H), 2.63-2.57 (m, 1H), 2.45-2.41 (m, 1H), 2.36 (s, 3H), 2.05-1.97 (m, 1H). Molecular formula: C22H19F2N3O4, calculated molecular weight: 427.41; found (LC-MS): 428.41 [M+1]⁺. |
| FDAB-122 | 11.03 (s, 1H), 9.19 (t, *J =* 5.8 Hz, 1H), 7.75-7.64 (m, 3H), 7.54 (d, *J =* 7.9 Hz, 1H), 7.43 (t, *J =* 7.9 Hz, 1H), 5.14 (dd, *J =* 13.3, 5.1 Hz, 1H), 4.65 (d, *J =* 4.6 Hz, 2H), 4.51 (d, *J =* 17.7 Hz, 1H), 4.33 (d, *J =* 17.6 Hz, 1H), 2.96-2.86 (m, 1H), 2.63-2.57 (m, 1H), 2.45-2.41 (m,1H), 2.29 (s, 3H), 2.04-1.95 (m, 1H). Molecular formula: C22H19ClFN3O4, calculated molecular weight: 443.86; found (LC-MS): 444.86 [M+1]⁺. |
| FDAB-123 | 11.03 (s, 1H), 8.91-8.90 (m, 1H), 7.73 (d, *J =* 7.8 Hz, 1H), 7.64-7.53 (m, 2H), 7.14 (dd, *J =* 16.9, 10.0 Hz, 2H), 5.14 (dd, *J* = 13.3, 5.0 Hz, 1H), 4.63 (d, *J =* 4.3 Hz, 2H), 4.51 (d, *J =* 17.6 Hz, 1H), 4.33 (d, *J =* 17.6 Hz, 1H), 2.96-2.86 (m, 1H), 2.64-2.55 (m, 1H), 2.45-2.42 (m, 1H), 2.36 (s, 3H), 2.05-1.98 (m, 1H). Molecular formula: C22H19ClFN3O4, calculated molecular weight: 443.86; found (LC-MS): 444.86 [M+1]⁺. |
| FDAB-124 | 11.02 (s, 1H), 9.70 (t, *J =* 5.9 Hz, 1H), 7.65-7.58 (m, 4H), 7.56 (d, *J =* 7.7 Hz, 1H), 7.44-7.38 (m, 1H), 5.11 (dd, *J=* 13.3, 5.1 Hz, 1H), 4.56 (d, *J =* 17.4 Hz, 1H), 4.49 (d, *J =* 4.8 Hz, 2H), 4.38 (d, *J =* 17.4 Hz, 1H), 2.96-2.86 (m, 1H), 2.64-2.57 (m, 1H), 2.45-2.39 (m, 1H), 2.04-1.96 (m, 1H). Molecular formula: C22H17ClF3N3O4, calculated molecular weight: 479.84; found (LC-MS): 480.84 [M+1]⁺. |
| FDAB-125 | 9.23 (t, *J =* 5.8 Hz, 1H), 7.93 (d, *J =* 2.0 Hz, 1H), 7.91 (d, *J =* 2.1 Hz, 1H), 7.59-7.52 (m, 4H), 5.18 (dd, *J =* 13.4, 5.0 Hz, 1H), 4.59 (d, *J =* 17.7 Hz, 3H), 4.39 (d, *J =* 17.3 Hz, 1H), 3.75-3.59 (m, 2H), 3.05-2.96 (m, 1H), 2.78-2.72 (m, 1H), 2.46-2.37 (m, 1H), 2.07-1.97 (m, 1H), 1.02 (t, *J =* 7.0 Hz, 3H). Molecular formula: C23H21ClFN3O4, calculated molecular weight: 457.89; found (LC-MS): 458.89 [M+1]⁺. |
| FDAB-126 | 9.23 (t, *J =* 5.7 Hz, 1H), 7.96-7.88 (m, 2H), 7.62-7.51 (m, 4H), 5.30 (dd, *J =* 13.4, 5.1 Hz, 1H), 4.69-4.58 (m, 3H), 4.44 (d, *J =* 16.9 Hz, 1H), 4.40-4.26 (m, 2H), 4.16-4.03 (m, 2H), 3.17-3.08 (m, 1H), 2.90-2.80 (m, 1H), 2.46-2.38 (m, 1H), 2.14-2.07 (m, 1H), 1.19 (t, *J* = 7.1 Hz, 3H). Molecular formula: C25H23ClFN3O6, calculated molecular weight: 515.92; found (LC-MS): 516.92 [M+1]⁺. |
| FDAB-127 | 9.23 (s, 1H), 7.92 (d, *J =* 8.5 Hz, 1H), 7.61 (d, *J =* 7.8 Hz, 1H), 7.57 (d, *J =* 3.2 Hz, 2H), 7.56-7.50 (m, 2H), 5.17-5.10 (m, 1H), 4.69 (d, *J =* 17.3 Hz, 1H), 4.62-4.55 (m, 3H), 4.42 (t, *J =* 15.9 Hz, 1H), 3.05-2.96 (m, 1H), 2.78-2.72 (m, 1H), 2.46-2.37 (m, 1H), 2.07-1.97 (m, 1H). Molecular formula: C25H23ClFN3O6, calculated molecular weight: 515.92; found (LC-MS): 516.92 [M+1]⁺. |
| FDAB-128 | 9.23 (brs, 1H), 7.94-7.90 (m, 2H), 7.61-7.53 (m, 4H), 5.67 (d, *J =* 9.4 Hz, 1H), 5.61 (*d, J =* 9.4 Hz, 1H), 5.30 (dd, *J =* 13.4, 5.1 Hz, 1H), 4.80-4.73 (m, 1H), 4.65-4.57 (m, 3H), 4.40 (d, *J =* 17.4 Hz, 1H), 3.15-3.06 (m, 1H), 2.88-2.79 (m, 1H), 2.50-2.47 (m, 1H), 2.10-2.03 (m, 1H), 1.21 (dd, *J =* 6.3, 1.4 Hz, 6H). Molecular formula: C26H25ClFN3O7, calculated molecular weight: 545.95; found (LC-MS): 546.95 [M+1]⁺. |
| FDAB-129 | 9.23 (t, *J =* 5.6 Hz, 1H), 7.95-7.88 (m, 2H), 7.61-7.52 (m, 4H), 5.62 (q, *J =* 9.6 Hz, 2H), 5.31 (dd, *J =* 13.4, 5.1 Hz, 1H), 4.61 (dd, *J =* 10.1, 6.9 Hz, 3H), 4.36 (d, *J =* 17.3 Hz, 1H), 3.17-3.05 (m, 1H), 2.85-2.81 (m, 1H), 2.47-2.42 (m, 1H), 2.11-2.03 (m, 1H), 1.10 (s, 9H). Molecular formula: C27H27ClFN3O6, calculated molecular weight: 543.98; found (LC-MS): 544.98 [M+1]⁺. |
| FDAB-130 | ¹H NMR (400 MHz, DMSO - *d*₆) δ 9.23 (brs, 1H), 7.94-7.90 (m, 2H), 7.65(s, 1H), 7.49-7.45 (m, 2H), 5.67 (d, *J =* 9.4 Hz, 1H), 5.61 (d, *J =* 9.4 Hz, 1H), 5.30 (dd, *J =* 13.4, 5.1 Hz, 1H), 4.80-4.73 (m, 1H), 4.65-4.57 (m, 3H), 4.40 (d, *J =* 17.4 Hz, 1H), 3.15-3.06 (m, 1H), 2.88-2.79 (m, 1H), 2.50 (s, 3H), 2.50-2.48 (m, 1 H), 2.10-2.03 (m, 1H), 1.21 (dd, *J =* 6.3, 1.4 Hz, 6H). Molecular formula: C27H27F2N3O7, calculated molecular weight: 543.52; found (LC-MS): 544.52 [M+1]. |
| FDAB-131 | 11.02 (s, 1H), 8.70 (t, *J =* 5.7 Hz, 1H), 7.64-7.53 (m, 2H), 7.53-7.47 (m, 1H), 7.44-7.34 (m, 2H), 5.12 (dd, *J =* 13.3, 5.1 Hz, 1H), 4.56 (d, *J =* 17.5 Hz, 1H), 4.42 (d, *J =* 4.1 Hz, 2H), 4.38 (d, *J =* 17.4 Hz, 1H), 3.67 (s, 2H), 2.97-2.86 (m, 1H), 2.65-2.55 (m, 1H), 2.45-2.41 (m, 1H), 2.04-1.97 (m, 1H). Molecular formula: C22H18Cl2FN3O4, calculated molecular weight: 478.30; found (LC-MS): 479.30 [M+1]⁺. |
| FDAB-132 | 11.02 (s, 1H), 8.59 (t, *J =* 5.9 Hz, 1H), 7.54 (d, *J =* 7.7 Hz, 1H), 7.43 (t, *J =* 7.0 Hz, 1H), 7.20-7.12 (m, 2H), 6.89-6.81 (m, 2H), 5.11 (dd, *J =* 13.3, 5.1 Hz, 1H), 4.55 (d, *J =* 17.4 Hz, 1H), 4.43-4.32 (m, 3H), 3.92 (t, *J =* 6.5 Hz, 2H), 2.97-2.86 (m, 1H), 2.63-2.56 (m, 1H), 2.46-2.38 (m, 1H), 2.06-1.96 (m, 1H), 1.71-1.64 (m, 2H), 1.46-1.36 (m, 2H), 0.92 (t, *J* = 7.4 Hz, 3H). Molecular formula: C26H28FN3O5, calculated molecular weight: 481.52; found (LC-MS): 482.52 [M+1]⁺. |
| FDAB-133 | 11.02 (s, 1H), 8.73 (t, *J =* 5.9 Hz, 1H), 7.64-7.57 (m, 2H), 7.55 (t, *J =* 1.8 Hz, 1H), 7.52 (d, *J =* 7.7 Hz, 2H), 7.49-7.44 (m, 3H), 7.40 *(d, J =* 7.6 Hz, 1H), 7.39-7.34 (m, 1H), 7.26 (d, *J =* 7.6 Hz, 1H), 5.11 (dd, *J* = 13.3, 5.1 Hz, 1H), 4.53 (d, *J =* 17.4 Hz, 1H), 4.42 (d, *J =* 4.9 Hz, 2H), 4.36 (d, *J =* 17.3 Hz, 1H), 3.57 (s, 2H), 2.97-2.86 (m, 1H), 2.64-2.55 (m, 1H), 2.45-2.41 (m, 1H), 2.03-1.96 (m, 1H). Molecular formula: C28H24FN3O4, calculated molecular weight: 485.52; found (LC-MS): 486.52 [M+1]⁺. |
| FDAB-134 | 11.01 (d, *J =* 8.7 Hz, 1H), 8.56 (t, *J =* 5.9 Hz, 1H), 7.49 (d, *J =* 7.7 Hz, 1H), 7.43 (t, *J =* 6.8 Hz, 1H), 7.33 (dd, *J =* 8.8, 4.5 Hz, 1H), 7.30-7.25 (m, 2H), 6.90 (td, *J =* 9.2, 2.5 Hz, 1H), 5.11 (dd, *J =* 13.3, 5.0 Hz, 1H), 4.54 (d, *J =* 17.3 Hz, 1H), 4.43-4.32 (m, 3H), 3.55 (s, 2H), 2.98-2.85 (m, 1H), 2.64-2.55 (m, 1H), 2.45-2.41 (m, 1H), 2.03-1.96 (m, 1H). Molecular formula: C24H20F2N4O4, calculated molecular weight: 466.44; found (LC-MS): 467.44 [M+1]⁺. |
| FDAB-135 | 11.29 (s, 1H), 11.02 (s, 1H), 8.26 (t, *J =* 5.8 Hz, 1H), 7.57-7.45 (m, 2H), 7.37 (d, *J =* 8.0 Hz, 1H), 7.30 (d, *J =* 2.3 Hz, 1H), 7.14 *(d, J* = 7.5 Hz, 1H), 6.96 (t, *J =* 7.8 Hz, 1H), 5.11 (dd, *J =* 13.2, 5.1 Hz, 1H), 4.54 (d, *J =* 17.4 Hz, 1H), 4.46-4.29 (m, 3H), 3.84 (s, 2H), 2.96-2.87 (m, 1H), 2.62-2.51 (m, 1H), 2.46-2.39 (m, 1H), 2.05-1.97 (m, 1H). Molecular formula: C24H20BrFN4O4, calculated molecular weight: 527.35; found (LC-MS): 528.35 [M+1]⁺. |
| FDAB-136 | 11.01 (s, 1H), 8.80 (t, *J* = 5.8 Hz, 1H), 7.89 (d, *J* = 7.7 Hz, 1H), 7.76 (dd, *J* = 7.5, 1.6 Hz, 1H), 7.56 (d, *J* = 7.7 Hz, 1H), 7.50 (t, *J =* 7.0 Hz, 1H), 7.37-7.26 (m, 2H), 7.24 (s, 1H), 5.11 (dd, *J =* 13.4, 5.1 Hz, 1H), 4.56 (d, *J =* 17.4 Hz, 1H), 4.44 (d, *J =* 4.2 Hz, 2H), 4.38 (d, *J =* 17.3 Hz, 1H), 3.84 (s, 2H), 2.97-2.87 (m, 1H), 2.64-2.55 (m, 1H), 2.45-2.40 (m, 1H), 2.03-1.97 (m, 1H). Molecular formula: C24H20FN3O4S, calculated molecular weight: 465.50; found (LC-MS): 466.50 [M+1]⁺. |
| FDAB-137 | 11.02 (s, 1H), 8.46 (t, *J =* 6.0 Hz, 1H), 7.48 (d, *J =* 7.7 Hz, 1H), 7.34-7.22 (m, 2H), 7.07-6.99 (m, 3H), 5.12 (dd, *J =* 13.3, 5.1 Hz, 1H), 4.54 (d, *J =* 17.4 Hz, 1H), 4.36 (t, *J =* 8.7 Hz, 3H), 3.03-2.89 (m, 1H), 2.86 (t, *J =* 7.4 Hz, 2H), 2.64-2.56 (m, 1H), 2.45-2.40 (m, 1H), 2.06-1.95 (m, 1H). Molecular formula: C23H21F2N3O4, calculated molecular weight: 441.43; found (LC-MS): 442.43 [M+1]⁺. |
| FDAB-138 | 11.02 (s, 1H), 8.49 (t, *J =* 5.9 Hz, 1H), 8.10 (d, *J* = 8.1 Hz, 1H), 7.93 (dd, *J* = 7.7, 1.7 Hz, 1H), 7.79 (d, *J* = 8.0 Hz, 1H), 7.59-7.51 (m, 2H), 7.51-7.48 (m, 1H), 7.43-7.37 (m, 1H), 7.36-7.33 (m, 1H), 7.30 (t, *J =* 7.0 Hz, 1H), 5.12 (dd, *J =* 13.4, 5.1 Hz, 1H), 4.55 (d, *J =* 17.4 Hz, 1H), 4.42-4.33 (m, 3H), 3.31 (d, *J =* 7.6 Hz, 2H), 2.97-2.86 (m, 1H), 2.64-2.56 (m, 3H), 2.46-2.42 (m, 1H), 2.05-1.96 (m, 1H). Molecular formula: C27H24FN304, calculated molecular weight: 473.50; found (LC-MS): 474.50 [M+1]⁺. |
| FDAB-139 | 11.02 (s, 1H), 8.43 (t, *J =* 5.9 Hz, 1H), 7.63 (d, *J =* 1.9 Hz, 1H), 7.62-7.53 (m, 2H), 7.46 (t, *J =* 7.0 Hz, 1H), 7.05-6.97 (m, 2H), 5.11 (dd, *J* = 13.2, 5.1 Hz, 1H), 4.55 (d, *J =* 17.4 Hz, 1H), 4.42-4.33 (m, 3H), 2.97-2.87 (m, 1H), 2.64-2.56 (m, 1H), 2.53-2.50 (m, 2H), 2.45-2.40 (m, 1H), 2.14 (t, *J =* 7.5 Hz, 2H), 2.04-1.96 (m, 1H), 1.83-1.75 (m, 2H). Molecular formula: C24H23FIN3O4, calculated molecular weight: 563.37; found (LC-MS): 564.37 [M+1]⁺. |
| FDAB-140 | ¹H NMR (400 MHz, DMSO-*d*₆) δ 11.1 (s, 1H), 8.27 (dd, *J =* 7.6, 3.4 Hz, 2H), 8.22 (d, *J =* 7.8 Hz, 1H), 8.19 (d, *J =* 9.3 Hz, 1H), 8.13 (d, *J =* 2.4 Hz, 2H), 8.06 (t, *J =* 7.6 Hz, 1H), 7.93 (d, *J =* 7.8 Hz, 1H), 7.56 (d, *J =* 7.7 Hz, 1H), 7.50 (t, *J =* 6.9 Hz, 1H), 5.11 (dd, *J* = 13.3, 5.1 Hz, 1H), 4.55 (d, *J =* 17.4 Hz, 1H), 4.43 (d, *J =* 4.4 Hz, 2H), 4.37 (d, *J =* 17.4 Hz, 1H), 3.33 (s, 4H), 2.97-2.87 m, 1H), 2.63-2.54 (m, 1H), 2.53-2.51 (m, 2H), 2.46-2.36 (m, 1H), 2.33 (t, *J =* 7.3 Hz, 2H), 2.08-1.94 (m, 3H). Molecular formula: C34H30FN3O4, calculated molecular weight: 563.63; found (LC-MS): 564.63 [M+1]⁺. |
| FDAB-141 | 11.01 (s, 1H), 7.76 (t, *J =* 5.9 Hz, 1H), 7.63-7.47 (m, 2H), 7.46-7.35 (m, 2H), 7.33 (d, *J =* 7.3 Hz, 1H), 5.11 (dd, *J =* 13.4, 5.1 Hz, 1H), 4.53 (d, *J =* 17.4 Hz, 1H), 4.42-4.22 (m, 3H), 2.98-2.85 (m, 1H), 2.63-2.54 (m, 1H), 2.46-2.38 (m, 1H), 2.08-1.91 (m, 1H), 1.42 (q, *J =* 3.9 Hz, 2H), 1.02 (q, *J =* 4.0 Hz, 2H). Molecular formula: C24H20BrF2N3O4, calculated molecular weight: 532.34; found (LC-MS): 533.34 [M+1]⁺. |
| FDAB-142 | 11.02 (s, 1H), 8.77 (t, *J =* 5.9 Hz, 1H), 7.60 (t, *J =* 8.2 Hz, 3H), 7.53 (d, *J =* 6.7 Hz, 1H), 7.51-7.42 (m, 3H), 6.70 *(d, J =* 15.9 Hz, 1H), 5.12 (dd, *J =* 13.3, 5.1 Hz, 1H), 4.62-4.51 (m, 3H), 4.39 (d, *J =* 17.4 Hz, 1H), 2.97-2.87 (m, 1H), 2.64-2.55 (m, 1H), 2.45-2.41 (m, 1H), 2.04-1.97 (m, 1H). Molecular formula: C23H19ClFN3O4, calculated molecular weight: 455.87; found (LC-MS): 456.87 [M+1]⁺. |
| FDAB-143 | 11.01 (s, 1H), 8.64 (t, *J* = 5.9 Hz, 1H), 7.58 (d, *J* = 7.7 Hz, 1H), 7.51 (dd, *J* = 7.8, 5.8 Hz, 3H), 7.42 (d, *J* = 15.8 Hz, 1H), 7.03-6.92 (m, 2H), 6.54 *(d, J =* 15.8 Hz, 1H), 5.12 (dd, *J =* 13.3, 5.1 Hz, 1H), 4.68-4.45 (m, 3H), 4.39 (d, *J =* 17.4 Hz, 1H), 3.96 (t, *J =* 6.5 Hz, 2H), 2.97-2.87 (m, 1H), 2.65-2.55 (m, 1H), 2.45-2.41 (m, 1H), 2.04-1.97 (m, 1H), 1.77-1.68 (m, 2H), 0.97 (t, *J* = 7.4 Hz, 3H). Molecular formula: C26H26FN3O5, calculated molecular weight: 479.51; found (LC-MS): 480.51 [M+1]⁺. |
| FDAB-144 | 11.02 (s, 1H), 9.44 (t, *J =* 5.9 Hz, 1H), 7.66-7.55 (m, 3H), 7.55-7.44 (m, 4H), 5.12 (dd, *J =* 13.3, 5.0 Hz, 1H), 4.58 (d, *J =* 17.5 Hz, 1H), 4.50 (d, *J =* 4.2 Hz, 2H), 4.40 (d, *J =* 17.4 Hz, 1H), 2.97-2.89 (m, 1H), 2.63-2.55 (m, 1H), 2.47-2.42 (m, 1H), 2.05-1.97 (m, 1H). Molecular formula: C23H18FN3O4, calculated molecular weight: 419.41; found (LC-MS): 420.41 [M+1]⁺. |
| FDAB-145 | 11.02 (s, 1H), 9.47 (t, *J =* 5.9 Hz, 1H), 7.67-7.57 (m, 3H), 7.58-7.46 (m, 3H), 5.12 (dd, *J =* 13.3, 5.1 Hz, 1H), 4.58 (d, *J =* 17.5 Hz, 1H), 4.50 (d, *J =* 4.2 Hz, 2H), 4.40 (d, *J =* 17.4 Hz, 1H), 2.97-2.87 (m, 1H), 2.65-2.56 (m, 1H), 2.46-2.41 (m, 1H), 2.05-1.96 (m, 1H). Molecular formula: C23H17ClFN3O4, calculated molecular weight: 453.85; found (LC-MS): 454.85 [M+1]⁺. |
| FDAB-146 | 11.00 (s, 1H), 9.92 (s, 1H), 9.63-9.28 (m, 1H), 7.60 (d, *J =* 7.7 Hz, 1H), 7.53 (q, *J =* 7.3, 6.2 Hz, 1H), 7.25 (t, *J =* 7.8 Hz, 1H), 7.00 (dt, *J =* 7.6, 1.3 Hz, 1H), 6.95-6.87 (m, 2H), 5.12 (dd, *J =* 13.3, 5.1 Hz, 1H), 4.58 (d, *J =* 17.5 Hz, 1H), 4.49 (d, *J =* 4.4 Hz, 2H), 4.40 (d, *J =* 17.4 Hz, 1H), 2.97-2.87 (m, 1H), 2.64-2.55 (m, 1H), 2.45-2.41 (m, 1H), 2.05-1.97 (m, 1H). Molecular formula: C23H18FN3O5, calculated molecular weight: 435.41; found (LC-MS): 436.41 [M+1]⁺. |
| FDAB-147 | 11.02 (s, 1H), 9.46 (t, *J* = 5.8 Hz, 1H), 7.83-7.75 (m, 2H), 7.73 (dd, *J* = 7.4, 1.7 Hz, 2H), 7.68 (d, *J* = 8.3 Hz, 2H), 7.61 (d, *J =* 7.7 Hz, 1H), 7.52 (dt, *J =* 17.3, 7.3 Hz, 3H), 7.44-7.37 (m, 1H), 5.13 (dd, *J =* 13.3, 5.0 Hz, 1H), 4.58 *(d, J =* 17.4 Hz, 1H), 4.51 (d, *J =* 4.0 Hz, 2H), 4.40 (d, *J =* 17.5 Hz, 1H), 2.98-2.87 (m, 1H), 2.64-2.55 (m, 1H), 2.45-2.41 (m, 1H), 2.05-1.97 (m, 1H). Molecular formula: C29H22FN3O4, calculated molecular weight: 495.51; found (LC-MS): 496.51 [M+1]⁺. |
| FDAB-148 | 11.02 (s, 1H), 9.62 (t, *J =* 5.9 Hz, 1H), 8.74-8.55 (m, 1H), 7.90 (td, *J* = 7.8, 1.8 Hz, 1H), 7.70 (d, *J =* 7.8 Hz, 1H), 7.61 (d, *J =* 7.7 Hz, 1H), 7.58-7.46 (m, 2H), 5.12 (dd, *J =* 13.4, 5.1 Hz, 1H), 4.58 (d, *J =* 17.5 Hz, 1H), 4.51 (d, *J =* 4.3 Hz, 2H), 4.40 (d, *J =* 17.4 Hz, 1H), 3.51 (s, 1H), 3.01-2.92 (m, 1H), 2.65-2.55 (m, 1H), 2.45-2.41 (m, 1H), 2.04-1.97 (m, 1H). Molecular formula: C22H17FN4O4, calculated molecular weight: 420.40; found (LC-MS): 421.40 [M+1]⁺. |
| FDAB-149 | 11.01 (s, 1H), 8.65 (t, *J =* 5.9 Hz, 1H), 7.55 (d, *J =* 7.7 Hz, 1H), 7.49 (d, *J =* 8.5 Hz, 2H), 7.42 (dt, *J =* 13.7, 7.3 Hz, 1H), 7.02 (t, *J =* 6.0 Hz, 1H), 6.66-6.58 (m, 2H), 5.12 (dd, *J =* 13.3, 5.1 Hz, 1H), 4.55 (d, *J* = 17.5 Hz, 1H), 4.43 (d, *J =* 5.1 Hz, 2H), 4.37 (d, *J =* 17.4 Hz, 1H), 3.86-3.80 (m, 2H), 2.97-2.86 (m, 1H), 2.64-2.55 (m, 1H), 2.45-2.41 (m, 1H), 2.04-1.97 (m, 1H). Molecular formula: C23H20FN5O4, calculated molecular weight: 449.44; found (LC-MS): 450.44 [M+1]⁺. |
| FDAB-150 | 11.02 (s, 1H), 8.92 (t, *J =* 6.0 Hz, 1H), 8.59 (t, *J =* 5.9 Hz, 1H), 7.96-7.88 (m, 2H), 7.57 (d, *J =* 8.0 Hz, 3H), 7.51 (t, *J =* 6.9 Hz, 1H), 5.12 (dd, *J =* 13.3, 5.1 Hz, 1H), 4.56 (d, *J =* 17.4 Hz, 1H), 4.43 (d, *J =* 5.0 Hz, 2H), 4.38 (d, *J =* 17.4 Hz, 1H), 3.97-3.88 (m, 2H), 2.97-2.86 (m, 1H), 2.64-2.55 (m, 1H), 2.46-2.39 (m, 1H), 2.05-1.96 (m, 1H). Molecular formula: C23H20ClFN4O5, calculated molecular weight: 486.88; found (LC-MS): 487.88 [M+1]⁺. |
| FDAB-151 | 11.02 (s, 1H), 8.44 (t, *J =* 5.9 Hz, 1H), 8.08 (t, *J =* 5.9 Hz, 1H), 7.55 (d, *J =* 7.7 Hz, 1H), 7.47 (t, *J =* 7.0 Hz, 1H), 5.12 (dd, *J =* 13.3, 5.1 Hz, 1H), 4.56 (d, *J =* 17.5 Hz, 1H), 4.41 (d, *J =* 4.5 Hz, 2H), 4.38 (d, *J =* 17.5 Hz, 1H), 3.72 (t, *J =* 3.0 Hz, 2H), 2.97-2.87 (m, 1H), 2.64-2.55 (m, 1H), 2.46-2.39 (m, 1H), 2.12 (t, *J* = 7.5 Hz, 2H), 2.05-1.96 (m, 1H), 1.54-1.46 (m, 2H), 1.34-1.15 (m, 8H), 0.85 (t, *J* = 6.9 Hz, 3H). Molecular formula: C24H31FN4O5, calculated molecular weight: 474.53; found (LC-MS): 475.53 [M+1]⁺. |
| FDAB-152 | 11.13 (s, 1H), 8.86 (t, *J =* 6.0 Hz, 1H), 7.75 (s, 2H), 7.59 (d, *J =* 7.7 Hz, 1H), 7.57-7.49 (m, 1H), 5.12 (dd, *J =* 13.3, 5.1 Hz, 1H), 4.57 *(d, J =* 17.4 Hz, 1H), 4.54-4.51 (m, 4H), 4.39 (d, *J =* 17.4 Hz, 1H), 2.97-2.87 (m, 1H), 2.64-2.57 (m, 1H), 2.45-2.37 (m, 1H), 2.05-1.96 (m, 1H). Molecular formula: C22H17Cl3FN3O5, calculated molecular weight: 528.74; found (LC-MS): 529.74 [M+1]⁺. |
| FDAB-153 | 11.02 (s, 1H), 8.80 (t, *J =* 6.0 Hz, 1H), 7.73 (d, *J =* 8.1 Hz, 1H), 7.56 (d, *J =* 8.6 Hz, 1H), 7.48 (d, *J =* 3.0 Hz, 2H), 7.41-7.34 (m, 1H), 7.29-7.22 (m, 3H), 7.17 (dd, *J =* 8.0, 1.7 Hz, 2H), 7.08 (t, *J =* 7.5 Hz, 1H), 5.47 (s, 2H), 5.11 (dd, *J =* 13.3, 5.0 Hz, 1H), 4.86 (s, 2H), 4.54 (d, *J =* 17.3 Hz, 1H), 4.48 (d, *J =* 4.9 Hz, 2H), 4.37 (d, *J =* 17.3 Hz, 1H), 2.97-2.87 (m, 1H), 2.65-2.55 (m, 1H), 2.45-2.39 (m, 1H), 2.03-1.96 (m, 1H). Molecular formula: C30H26FN5O5, calculated molecular weight: 555.57; found (LC-MS): 556.57 [M+1]⁺. |
| FDAB-154 | 11.02 (s, 1H), 8.65 (t, *J =* 5.9 Hz, 1H), 7.57-7.46 (m, 2H), 7.37-7.23 (m, 2H), 6.93 (dd, *J =* 8.2, 7.0 Hz, 3H), 5.12 (dd, *J =* 13.3, 5.0 Hz, 1H), 4.56 (d, *J =* 17.4 Hz, 1H), 4.44 (d, *J =* 4.1 Hz, 2H), 4.38 (d, *J =* 17.3 Hz, 1H), 4.20 (t, *J =* 6.0 Hz, 2H), 2.97-2.86 (m, 5.4 Hz, 1H), 2.63 (t, *J =* 6.0 Hz, 2H), 2.62-2.55 (m, 1H), 2.45-2.39 (m, 1H), 2.06-1.93 (m, 1H). Molecular formula: C23H22FN3O5, calculated molecular weight: 439.44; found (LC-MS): 440.44 [M+1]⁺. |
| FDAB-155 | 11.02 (s, 1H), 8.87 (t, *J =* 5.9 Hz, 1H), 8.40-8.34 (m, 2H), 7.52 (d, *J =* 7.7 Hz, 1H), 7.41 (t, *J =* 7.0 Hz, 1H), 7.34-7.28 (m, 2H), 5.12 (dd, *J =* 13.3, 5.1 Hz, 1H), 4.55 (d, *J =* 17.4 Hz, 1H), 4.43 (d, *J =* 4.1 Hz, 2H), 4.37 (d, *J =* 17.4 Hz, 1H), 3.89 (s, 2H), 2.96-2.86 (m, 1H), 2.63-2.57 (m, 1H), 2.45-2.40 (m, 1H), 2.04-1.97 (m, 1H). Molecular formula: C21H19FN4O4S, calculated molecular weight: 442.47; found (LC-MS): 443.47 [M+1]⁺. |
| FDAB-156 | 11.02 (s, 1H), 8.29 (t, *J =* 5.9 Hz, 1H), 7.56 (d, *J =* 7.7 Hz, 1H), 7.42 (t, *J =* 7.0 Hz, 1H), 5.11 (dd, *J =* 13.3, 5.1 Hz, 1H), 4.55 (d, *J =* 17.4 Hz, 1H), 4.44-4.29 (m, 3H), 2.97-2.90 (m, 1H), 2.64-2.55 (m, 1H), 2.46-2.38 (m, 1H), 2.08 (dt, *J =* 8.3, 2.5 Hz, 4H), 2.02-1.97 (m, 3H), 1.87-1.82 (m, 2H), 1.80-1.68 (m, 2H). Molecular formula: C22H24FN304, calculated molecular weight: 413.45; found (LC-MS): 414.45 [M+1]⁺. |
| FDAB-157 | 11.02 (s, 1H), 8.41 (t, *J =* 6.0 Hz, 1H), 7.56 (d, *J =* 7.7 Hz, 1H), 7.46 (t, *J =* 7.0 Hz, 1H), 5.12 (dd, *J =* 13.2, 5.1 Hz, 1H), 4.55 (d, *J =* 17.4 Hz, 1H), 4.38 (t, *J =* 8.7 Hz, 3H), 2.97-2.87 (m, 1H), 2.63-2.57 (m, 1H), 2.46-2.39 (m, 1H), 2.13 (t, *J =* 7.4 Hz, 2H), 2.04-1.96 (m, 1H), 1.58-1.43 (m, 2H), 1.23 (s, 14H), 0.85 (t, *J* = 6.6 Hz, 3H). Molecular formula: C25H34FN3O4, calculated molecular weight: 459.56; found (LC-MS): 460.56 [M+1]⁺. |
| FDAB-158 | 11.02 (s, 1H), 8.43 (t, *J =* 5.9 Hz, 1H), 7.54 (dd, *J =* 16.9, 7.6 Hz, 1H), 7.48-7.42 (m, 1H), 5.11 (dd, *J =* 13.3, 5.1 Hz, 1H), 4.55 (d, *J =* 17.6 Hz, 1H), 4.46-4.32 (m, 3H), 2.97-2.87 (m, 1H), 2.63-2.57 (m, 1H), 2.46-2.39 (m, 1H), 2.30-2.26 (m, 2H), 2.17-2.14 (m, 6H), 2.03-1.96 (m, 1H), 1.63-1.41 (m, 3H), 1.45-1.28 (m, 3H). Molecular formula: C21H27FN4O4, calculated molecular weight: 418.47; found (LC-MS): 419.47 [M+1]⁺. |
| FDAB-159 | 10.99 (s, 1H), 8.40 (t, *J =* 5.9 Hz, 1H), 7.55 (d, *J =* 7.8 Hz, 1H), 7.47 (t, *J =* 7.0 Hz, 1H), 5.11 (dd, *J =* 13.3, 5.0 Hz, 1H), 4.55 (d, *J =* 17.4 Hz, 1H), 4.44-4.31 (m, 3H), 2.95-2.86 (m, 1H), 2.63-2.57 (m, 1H), 2.47-2.41 (m, 1H), 2.36-2.09 (m, 8H), 2.04-1.96 (m, 1H), 1.59-1.43 (m, 6H), 1.41-1.33 (m, 4H). Molecular formula: C24H31FN4O4, calculated molecular weight: 458.53; found (LC-MS): 459.53 [M+1]⁺. |
| FDAB-160 | 11.00 (s, 1H), 8.46 (t, *J =* 5.8 Hz, 1H), 7.70-7.51 (m, 2H), 7.47 (t, *J =* 7.1 Hz, 1H), 7.45-7.22 (m, 9H), 5.11 (dd, *J* = 13.3, 5.1 Hz, 1H), 4.55 (d, *J =* 17.3 Hz, 1H), 4.44-4.31 (m, 3H), 2.97-2.86 (m, 1H), 2.63-2.56 (m, 1H), 2.46-2.40 (m, 1H), 2.35-2.28 (m, 2H), 2.27-2.19 (m, 2H), 2.04-1.96 (m, 1H). Molecular formula: C29H27FN3O4P, calculated molecular weight: 531.52; found (LC-MS): 532.52 [M+1]⁺. |
| FDAB-161 | 11.05 (s, 1 H), 8.62-8.56 (m, 1H), 7.56 (dd, *J =* 7.8, 2.7 Hz, 1H), 7.54-7.39 (m, 2H), 7.39-7.16 (m, 4H), 5.11 (dd, *J =* 13.3, 5.1 Hz, 1H), 4.68 (d, *J =* 3.8 Hz, 2H), 4.56 (d, *J =* 17.2 Hz, 1H), 4.43-4.35 (m, 3H), 3.64-3.56 (m, 2H), 2.97-2.87 (m, 1H), 2.70-2.55 (m, 3H), 2.46-2.41 (m, 1H), 2.04-1.96 (m, 1H). Molecular formula: C25H24FN3O4S3, calculated molecular weight: 545.66; found (LC-MS): 546.66 [M+1]⁺. |
| FDAB-162 | 11.02 (s, 1H), 8.59 (d, *J =* 5.9 Hz, 1H), 7.57 (dd, *J =* 7.8, 2.7 Hz, 1H), 7.52-7.45 (m, 1H), 6.97 (dd, *J =* 15.1, 11.4 Hz, 1H), 6.74-6.64 (m, 2H), 6.41-6.23 (m, 3H), 5.90 (*d, J =* 7.8 Hz, 1H), 5.12 (dd, *J* = 13.2, 5.1 Hz, 1H), 4.56 (d, *J =* 17.7 Hz, 1H), 4.47-4.36 (m, 3H), 3.75 (s, *J =* 1.3 Hz, 3H), 2.94-2.85 (m, 1H), 2.62-2.57 (d, *J =* 17.5 Hz, 1H), 2.45-2.41 (m, 1H), 2.30 (s, 3H), 2.24 (d, *J =* 7.0 Hz, 3H), 2.19 (d, *J =* 7.3 Hz, 3H), 2.07 (d, *J =* 7.7 Hz, 6H), 2.04-1.96 (m, 1H). Molecular formula: C35H38FN3O5, calculated molecular weight: 599.70; found (LC-MS): 600.70 [M+1]⁺. |
| FDAB-163 | 11.02 (s, 1H), 8.61 (t, *J =* 5.9 Hz, 1H), 7.50 (d, *J =* 7.7 Hz, 1H), 7.45 (t, *J =* 6.9 Hz, 1H), 5.11 (dd, *J =* 13.3, 5.1 Hz, 1H), 4.53 (d, *J =* 17.4 Hz, 1H), 4.36 (dd, *J =* 11.6, 5.8 Hz, 3H), 3.25 (t, *J =* 7.3 Hz, 2H), 2.97-2.87 (m, 1H), 2.62-2.57 (m, 1H), 2.46-2.39 (m, 1H), 2.03-1.95 (m, 1H), 1.64 (s, 6H), 1.59-1.52 (m, 2H), 1.35-1.15 (m, 18H), 0.90-0.80 (m, 3H). Molecular formula: C31H44FN3O4S3, calculated molecular weight: 637.89; found (LC-MS): 638.89 [M+1]⁺. |
| FDAB-164 | 11.02 (s, 1H), 8.65 (s, 1H), 7.56 (d, *J =* 7.8 Hz, 1H), 7.54-7.47 (m, 1H), 5.17-5.07 (m, 1H), 4.56 (d, *J =* 17.6 Hz, 1H), 4.38 (d, *J =* 18.9 Hz, 3H), 3.51-3.48 (m, 2H), 2.94-2.88 (m, 1H), 2.64-2.57 (m, 3H), 2.35-2.32 (m, 1H), 2.07-1.94 (m, 2H), 1.90-1.85 (m, 4H), 1.69-1.63 (m, 2H), 1.35-1.15 (m, 18H), 0.85-0.83 (m, 3H). Molecular formula: C33H45FN4O4S3, calculated molecular weight: 676.93; found (LC-MS): 677.93 [M+1]⁺. |
| FDAB-165 | 11.00 (s, 1H), 7.73-7.63 (m, 2H), 7.60-7.46 (m, 2H), 7.27 (d, *J =* 9.2 Hz, 1H), 6.77 (s, 1H), 5.76 (s, 1H), 5.11 (dd, *J =* 13.3, 5.1 Hz, 1H), 4.67 (s, 1H), 4.53 (d, *J =* 17.3 Hz, 1H), 4.48-4.29 (m, 2H), 3.88 (d, *J =* 9.0 Hz, 2H), 2.97-2.86 (m, 1H), 2.63-2.58 (m, 1H), 2.46-2.39 (m, 1H), 2.05-1.97 (m, 1H). Molecular formula: C23H19ClFN3O4, calculated molecular weight: 455.87; found (LC-MS): 456.87 [M+1]⁺. |
| FDAB-166 | 11.01 (s, 1H), 7.79-7.68 (m, 1H), 7.68-7.60 (m, 1H), 7.56 (d, *J* = 7.6 Hz, 1H), 7.41 (dd, *J* = 15.7, 8.8 Hz, 3H), 7.26 (dd, *J =* 9.8, 5.4 Hz, 1H), 7.20-7.11 (m, 1H), 7.06-6.94 (m, 3H), 5.75 (s, 1 H), 5.13 (dd, *J =* 13.4, 4.7 Hz, 1H), 4.68-4.49 (m, 2H), 4.49-4.30 (m, 2H), 4.09-3.92 (m, 2H), 2.97-2.86 (m, 1H), 2.65-2.58 (m, 1H), 2.45-2.38 (m, 1H), 2.05-1.96 (m, 1H). Molecular formula: C27H24FN3O4, calculated molecular weight: 473.50; found (LC-MS): 474.50 [M+1]⁺. |
| FDAB-167 | 11.00 (s, 1H), 7.68 (t, *J =* 6.9 Hz, 1H), 7.60-7.54 (m, 1H), 7.25 (d, *J =* 7.7 Hz, 2H), 7.13 (d, *J =* 7.6 Hz, 2H), 5.76 (s, 1H), 5.11 (dd, *J =* 13.2, 5.2 Hz, 1H), 4.54 (d, *J =* 17.4 Hz, 1H), 4.37 (d, *J =* 17.4 Hz, 1H), 3.81 (s, 2H), 3.67 (s, 2H), 2.97-2.86 (m, 1H), 2.63-2.58 (m, 1H), 2.54 (s, 2H), 2.46-2.39 (m, 1H), 2.04-1.96 (m, 1H), 1.56 (p, *J* = 7.4 Hz, 2H), 0.88 (td, *J* = 7.3, 1.7 Hz, 3H).Molecular formula: C24H26FN3O3, calculated molecular weight: 423.49; found (LC-MS): 424.49 [M+1]⁺. |
| FDAB-168 | 11.00 (s, 1H), 7.67 (t, *J =* 6.9 Hz, 1H), 7.58 (d, *J =* 7.7 Hz, 1H), 7.38 (s, 4H), 5.76 (s, 1H), 5.11 (dd, *J =* 13.3, 5.1 Hz, 1H), 4.54 (d, *J =* 17.3 Hz, 1H), 4.37 (d, *J =* 17.3 Hz, 1H), 3.82 (s, 2H), 3.69 (s, 2H), 2.97-2.86 (m, 1H), 2.64-2.57 (m, 1H), 2.45-2.38 (m, 1H), 2.04-1.97 (m, 1H). Molecular formula: C21H19ClFN3O3, calculated molecular weight: 415.85; found (LC-MS): 416.85 [M+1]⁺. |
| FDAB-169 | 11.02 (s, 1H), 8.76 (s, 1H), 7.88 (d, *J =* 7.7 Hz, 1H), 7.76 (d, *J =* 7.7 Hz, 1H), 7.43-7.40 (m, 1H), 7.38-7.30 (m, 2H), 7.31-7.26 (m, 1H), 7.25 (s, 1H), 5.13-5.04 (m, 1H), 4.51-4.36 (m, 3H), 4.32 (d, *J =* 17.5 Hz, 1H), 3.85 (s, 2H), 2.92-2.82 (m, 1H), 2.61-2.54 (m, 1H), 2.27-2.16 (m, 1H), 1.97-1.88 (m, 1H). Molecular formula: C24H20FN3O4S, calculated molecular weight: 465.50; found (LC-MS): 466.50 [M+1]⁺. |
| FDAB-170 | 11.02 (s, 1H), 8.46 (t, *J* = 6.2 Hz, 1H), 8.10 (d, *J* = 8.2 Hz, 1H), 7.92 (d, *J* = 7.9 Hz, 1H), 7.77 (d,*J* = 79 Hz, 1H), 7.60-7.47 (m, 2H), 7.44-7.31 (m, 3H), 7.19 (dd, *J =* 10.2, 2.2 Hz, 1H), 5.13 (dd, *J* = 13.4, 5.1 Hz, 1H), 4.46 (d, *J =* 17.3 Hz, 1H), 4.40-4.23 (m, 3H), 3.35 (s, 2H), 2.97-2.86 (m, 1H), 2.64-2.55 (m, 3H), 2.39-2.31 (m, 1H), 2.05-1.97 (m, 1H). Molecular formula: C27H24FN3O4, calculated molecular weight: 473.50; found (LC-MS): 474.50 [M+1]. |
| FDAB-171 | 11.04 (s, 1H), 9.67 (s, 1H), 7.64-7.60 (m, 4H), 7.45 (dd, *J =* 7.6, 2.3 Hz, 1H), 7.23-7.15 (m, 1H), 5.13 (dd, *J =* 13.1, 5.1 Hz, 1H), 4.41 (d, *J =* 6.4 Hz, 3H), 4.33 (d, *J =* 17.2 Hz, 1H), 2.97-2.86 (m, 1H), 2.65-2.59 (m, 1H), 2.33-2.27 (m, 1H), 2.03-1.97 (m, 1H). Molecular formula: C22H17ClF3N3O4, calculated molecular weight: 479.84; found (LC-MS): 480.84 [M+1]⁺. |
| FDAB-172 | 11.01 (s, 1H), 8.78 (t, *J =* 5.9 Hz, 1H), 7.63-7.42 (m, 3H), 6.71 (d, *J =* 2.0 Hz, 1H), 6.58 (dd, *J =* 8.4, 2.0 Hz, 1H), 6.43 (s, 2H), 5.12 (dd, *J* = 13.3, 5.1 Hz, 1H), 4.63-4.50 (m, 3H), 4.39 (d, *J* = 17.3 Hz, 1H), 2.96-2.87 (m, 1H), 2.64-2.55 (m, 1H), 2.46-2.41 (m, 1H), 2.04-1.96 (m, 1H), 1.23 (s, 9H). Molecular formula: C25H27FN4O4, calculated molecular weight: 466.51; found: 467.51 [M+1]. |
| FDAB-173 | 11.02 (s, 1H), 9.63 (s, 1H), 8.96 (t, *J =* 5.9 Hz, 1H), 7.57 (d, *J =* 7.8 Hz, 1H), 7.49 (t, *J =* 7.0 Hz, 1H), 7.33-7.24 (m, 2H), 7.21 (d, *J =* 8.0 Hz, 1H), 5.12 (dd, *J =* 13.3, 5.1 Hz, 1H), 4.59 (d, *J =* 4.3 Hz, 1H), 4.56 (d, *J =* 7.4 Hz, 2H), 4.39 (d, *J =* 17.4 Hz, 1H), 2.96-2.86 (m, 1H), 2.64-2.56 (m, 1H), 2.46-2.41 (m, 1H), 2.04-1.96 (m, 1H), 1.35 (s, 9H). Molecular formula: C25H26FN3O5, calculated molecular weight: 467.50; found: 468.50 [M+1]. |
| FDAB-174 | 11.02 (s, 1H), 9.08 (s, 1H), 7.88-7.81 (m, 2H), 7.57 (d, *J =* 7.7 Hz, 1H), 7.54-7.46 (m, 3H), 5.12 (dd, *J =* 13.3, 5.1 Hz, 1H), 4.63-4.53 (m, 3H), 4.39 (d, *J* = 17.3 Hz, 1H), 2.96-2.87 (m, 1H), 2.78-2.73 (m, 2H), 2.64-2.55 (m, 1H), 2.46-2.40 (m, 1H), 2.04-1.98 (m, 1H), 1.56 (s, 6 H), 1.54 (s, 2H). Molecular formula: C25H27FN4O4, calculated molecular weight: 466.51; found: 467.51 [M+1]. |
| FDAB-175 | 11.02 (s, 1H), 9.24 (t, *J =* 5.8 Hz, 1H), 8.33 (s, 2H), 8.06-7.98 (m, 2H), 7.82 (dd, *J =* 8.4, 3.4 Hz, 4H), 7.57 (dd, *J =* 11.6, 7.0 Hz, 4H), 5.12 (dd, *J =* 13.2, 5.1 Hz, 1H), 4.64 (d, *J* = 5.7 Hz, 2H), 4.59 (d, *J =* 17.4 Hz, 1H), 4.40 (d, *J =* 17.4 Hz, 1H), 4.09 (s, 2H), 2.98-2.88 (m, 1H), 2.65-2.56 (m, 1H), 2.46-2.41 (m, 1H), 2.04-1.96 (m, 1H). Molecular formula: C28H25FN4O4, calculated molecular weight: 500.53; found: 501.53 [M+1]. |
| FDAB-176 | 11.02 (s, 1H), 8.94 (t, *J =* 5.8 Hz, 1H), 7.70 (d, *J =* 8.4 Hz, 1H), 7.66 -7.57 (m, 3H), 7.57-7.51 (m, 1H), 7.46 (dd, *J =* 8.4, 6.8 Hz, 2H), 7.42-7.33 (m, 1H), 7.00 (d, *J =* 1.8 Hz, 1H), 6.84 (dd, *J =* 8.2, 1.9 Hz, 1H), 6.59 (s, 2H), 5.12 (dd, *J =* 13.2, 5.1 Hz, 1H), 4.59 (d, *J =* 7.9 Hz, 1H), 4.57 (d, *J =* 4.5 Hz, 2H), 4.40 (d, *J =* 17.3 Hz, 1H), 2.96-2.86 (m, 1H), 2.65-2.56 (m, 1H), 2.46-2.42 (m, 1H), 2.04-1.96 (m, 1H). Molecular formula: C27H23FN4O4, calculated molecular weight: 486.50; found: 487.50 [M+1]. |
| FDAB-177 | 11.01 (s, 1H), 9.10 (t, *J =* 5.8 Hz, 1H), 7.96-7.84 (m, 2H), 7.68- 7.61 (m, 2H), 7.61-7.50 (m, 2H), 7.48-7.41 (m, 2H), 6.69-6.62 (m, 2H), 5.35 (br, s, 2H), 5.12 (dd, *J =* 13.3, 5.1 Hz, 1H), 4.66-4.52 (m, 3H), 4.40 (d, *J =* 17.4 Hz, 1H), 2.96-2.86 (m, 1H), 2.64-2.57 (m, 1H), 2.46-2.42 (m, 1H), 2.06-1.95 (m, 1H). Molecular formula: C27H23FN4O4, calculated molecular weight: 486.50; found: 487.50 [M+1]. |
| FDAB-178 | 11.01 (s, 1H), 8.69 (t, *J =* 5.9 Hz, 1H), 7.57 (d, *J =* 7.8 Hz, 1H), 7.56 -7.48 (m, 1H), 7.47-7.43 (m, 2H), 7.41-7.38 (m, 2H), 7.37-7.32 (m, 1H), 7.22 (d, *J =* 1.0 Hz, 1H), 6.42 (s, 1H), 6.38 (s, 2H), 5.12 (dd, *J =* 13.3, 5.1 Hz, 1H), 5.04 (s, 2H), 4.62-4.52 (m, 3H), 4.39 (d, *J =* 17.3 Hz, 1H), 3.70 (s, 3H), 2.96-2.86 (m, 1H), 2.64-2.57 (m, 1H), 2.46-2.42 (m, 1H), 2.06-1.95 (m, 1H). Molecular formula: C29H27FN4O6, calculated molecular weight: 546.56; found: 547.56 [M+1]. |
| FDAB-179 | 11.01 (s, 1H), 9.30 (s, 1H), 8.90 (t, *J =* 5.8 Hz, 1H), 7.56 (d, *J =* 7.7 Hz, 1H), 7.53-7.43 (m, 3H), 7.43-7.35 (m, 3H), 7.35-7.25 (m, 2H), 7.05 (d, *J =* 8.5 Hz, 1H), 5.18 (s, 2H), 5.12 (dd, *J =* 13.3, 5.1 Hz, 1H), 4.63-4.49 (m, 3H), 4.39 (d, *J* = 17.4 Hz, 1H), 2.96-2.86 (m, 1H), 263-257 (m, 1H), 2.46-2.41 (m, 1H), 2.04-1.97 (m, 1H). Molecular formula: C28H24FN3O6, calculated molecular weight: 517.51; found: 518.51 [M+1]. |
| FDAB-180 | 11.01 (s, 1H), 9.18 (s, 2H), 8.37 (t, *J =* 5.8 Hz, 1H), 7.55 (d, *J =* 7.8 Hz, 1H), 7.42 (t, *J =* 7.0 Hz, 1H), 6.98-6.90 (m, 4H), 6.68-6.60 (m, 4H), 5.11 (dd, *J =* 13.3, 5.1 Hz, 1H), 4.54 (d, *J =* 17.4 Hz, 1H), 4.41-4.28 (m, 3H), 2.95-2.86 (m, 1H), 2.64-2.55 (m, 1H), 2.46-2.38 (m, 1H), 2.25-1.98 (m, 2H), 2.04-1.95 (m, 1H), 1.95-1.87 (m, 2H), 1.46 (s, 3H). Molecular formula: C31H30FN3O6, calculated molecular weight: 559.59; found: 560.59 [M+1]. |
| FDAB-181 | 11.01 (s, 1H), 8.57 (t, *J =* 5.8 Hz, 1H), 7.55 (d, *J =* 7.7 Hz, 1H), 7.47 (t, *J =* 7.0 Hz, 1H), 7.46-7.39 (m, 2H), 7.36-7.335 (m, 2H), 7.30 (t, *J =* 6.8 Hz, 2H), 7.27 (t, *J =* 6.7 Hz, 2H), 7.23-7.12 (m, 2H), 5.11 (dd, *J =* 13.3, 5.1 Hz, 1H), 4.55 (d, *J =* 17.4 Hz, 1H), 4.43-4.28 (m, 3H), 4.08 (t, *J* = 10.1 Hz, 1H), 3.83 (d, *J* = 10.2 Hz, 1H), 2.96-2.86 (m, 1H), 2.63-2.57 (m, 1H), 2.46-2.41 (m, 1H), 2.3 (s, 2H), 2.26-2.17 (m, 1H), 2.13-2.07 (m, 1H), 2.04-1.97 (m, 1H). Molecular formula: C30H29FN404, calculated molecular weight: 528.58; found: 529.58 [M+1]. |
| FDAB-182 | 11.02 (s, 1H), 9.09 (s, 1H), 7.90 (d, *J =* 12.7 Hz, 1H), 7.69 (d, *J =* 1.3 Hz, 1H), 7.59 (d, *J =* 7.7 Hz, 1H), 7.55-7.46 (m, 1H), 7.21 (d, *J =* 1.3 Hz, 1H), 7.13-6.94 (m, 1H), 5.12 (dd, *J* = 13.3, 5.1 Hz, 1H), 4.57 (d, *J* = 17.4 Hz, 1H), 4.50-4.20 (m, 3H), 3.12-3.04 (m, 1H), 2.97-2.95 (m, 2H), 2.93-2.86 (m, 2H), 2.63-2.57 (m, 1H), 2.47-2.45 (m, 1H), 2.05-1.97 (m, 1H), 2.0 (s, 2H). Molecular formula: C23H23ClFN5O4, calculated molecular weight: 487.92; found: 488.92 [M+1]. |
| FDAB-183 | 11.02 (s, 1H), 8.84 (s, 1H), 7.64 (d, *J =* 2.1 Hz, 1H), 7.58 (d, *J =* 7.7 Hz, 1H), 7.52 (t, *J =* 7.0 Hz, 1H), 7.18 (d, *J =* 8.4 Hz, 1H), 7.13 (dd, *J =* 8.3, 2.2 Hz, 1H), 6.84 (t, *J =* 6.0 Hz, 1H), 5.12 (dd, *J* = 13.3, 5.1 Hz, 1H), 4.70 (t, *J =* 5.3 Hz, 1H), 4.57 (d, *J =* 17.4 Hz, 1H), 4.44 (d, *J =* 5.8 Hz, 2H), 4.39 (d, *J =* 17.4 Hz, 1H), 3.53 (q, *J =* 6.8 Hz, 2H), 2.96-2.86 (m, 1H), 2.75 (t, *J =* 7.2 Hz, 2H), 2.64-2.55 (m, 1H), 2.46-2.41 (m, 1H), 2.04-1.97 (m, 1H). Molecular formula: C23H22ClFN4O5, calculated molecular weight: 488.90; found: 489.90 [M+1]. |
| FDAB-184 | 11.01 (s, 1H), 9.30 (s, 1H), 7.81 (d, *J* = 7.9 Hz, 1H), 7.64-7.59 (m, 2H), 7.60-7.49 (m, 3H), 7.50-7.38 (m, 2H), 6.64 (d, *J =* 8.6 Hz, 2H), 5.39 (s, 2H), 5.11 (dd, *J =* 13.2, 5.0 Hz, 1H), 4.50-4.42 (m, 3H), 4.32 (d, *J* = 17.4 Hz, 1H), 2.94-2.90 (m, 1H), 2.63-2.61 (m, 1H), 2.43-2.35 (m, 1H), 2.04-1.95 (m, 1H). Molecular formula: C29H23FN4O4, calculated molecular weight: 510.53; found: 511.53 [M+1]. |
| FDAB-185 | 11.03 (s, 1H), 9.02 (s, 1H), 7.74 (d, *J =* 2.1 Hz, 1H), 7.58 (d, *J =* 7.7 Hz, 1H), 7.54-7.49 (m, 2H), 7.18 (d, *J =* 8.4 Hz, 1H), 7.13 (dd, *J* = 8.3, 2.2 Hz, 1H), 6.84 (t, *J =* 6.0 Hz, 1H), 5.12 (dd, *J* = 13.3, 5.1 Hz, 1H), 4.70 (t, *J* = 5.3 Hz, 1H), 4.57 (d, *J =* 17.4 Hz, 1H), 4.44 (d, *J =* 5.8 Hz, 2H), 4.39 (d, *J =* 17.4 Hz, 1H), 3.58 (q, *J =* 6.8 Hz, 2H), 2.96-2.86 (m, 1H), 2.80 (t, *J =* 7.2 Hz, 2H), 2.64-2.55 (m, 1H), 2.46-2.41 (m, 1H), 2.04-1.97 (m, 1H). Molecular formula: C23H23ClN4O5, calculated molecular weight: 470.91; found: 471.91 [M+1]. |
| FDAB-186 | 11.03 (s, 1H), 9.30 (s, 1H), 8.96 (s, 1H), 7.81 (d, *J =* 7.9 Hz, 1H), 7.64-7.59 (m, 2H), 7.60-7.49 (m, 3H), 7.50-7.38 (m, 2H), 6.64 (d, *J =* 8.6 Hz, 2H), 5.11 (dd, *J =* 13.2, 5.0 Hz, 1H), 4.50-4.42 (m, 3H), 4.32 (d, *J* = 17.4 Hz, 1H), 2.94-2.90 (m, 1H), 2.63-2.61 (m, 1H), 2.43-2.35 (m, 1H), 2.04-1.95 (m, 1H). Molecular formula: C29H22FN3O5, calculated molecular weight: 511.51; found: 512.51 [M+1]. |
| FDAB-187 | 11.03 (s, 1H), 9.30 (s, 1H), 7.83-7.80 (m, 1H), 7.68-7.62 (m, 2H), 7.60-7.49 (m, 3H), 7.50-7.38 (m, 2H), 6.64 (d, *J =* 8.6 Hz, 2H), 5.11 (dd, *J =* 13.2, 5.0 Hz, 1H), 4.85 (s, 1H), 4.50-4.42 (m, 3H), 4.32 (d, *J =* 17.4 Hz, 1H), 3.54 (q, *J =* 6.8 Hz, 2H), 2.94-2.90 (m, 1H), 2.73 (t, *J =* 7.2 Hz, 2H), 2.63-2.61 (m, 1H), 2.43-2.35 (m, 1H), 2.04-1.95 (m, 1H). Molecular formula: C31H26FN3O5, calculated molecular weight: 539.56; found: 540.56 [M+1]. |
| FDAB-188 | 11.01 (s, 1H), 8.94 (s, 1H), 7.81 (d, *J =* 7.9 Hz, 1H), 7.64-7.59 (m, 2H), 7.60-7.49 (m, 3H), 7.50-7.38 (m, 2H), 6.64 (d, *J =* 8.6 Hz, 2H), 5.11 (dd, *J =* 13.2, 5.0 Hz, 1H), 4.50-4.42 (m, 3H), 4.32 (d, *J =* 17.4 Hz, 1H), 3.15 (q, *J =* 6.8 Hz, 2H), 2.94-2.90 (m, 1H), 2.67 (t, *J =* 7.2 Hz, 2H), 263-261 (m, 1H), 2.43-2.35 (m, 1H), 2.2 (s, 2H), 2.04-1.95 (m, 1H). Molecular formula: C31H27FN4O4, calculated molecular weight: 538.58; found: 539.58 [M+1]. |

### Example 14: Antiproliferative activity of compound against MV-4-11 cells

MV411 cells in the logarithmic growth phase were diluted in a medium (RPMI + 10% FBS) and plated at 5,000 cells/well in 96-well whole black plates (WHB) with 50 µL/well. After plating, the plates were incubated in an incubator at 5% CO₂ and 37 °C for 24 h before the compounds were added. The compounds were prepared into a 10 mM stock solution in DMSO and diluted in a culture medium to the desired concentrations (a final DMSO concentration of 0.2%). 50 µL of the compound dilutions at final concentrations of 10,000, 3,333.33, 1,111.11, 370.37, 123.46, 41.15, 13.72, 4.57, 1.52, and 0 nM were added to the 96-well plates containing the cells, that is, each compound was serially diluted to 10 concentrations in duplicate. The plates were incubated at 5% CO₂ and 37 °C for 72 h. After the incubation was complete, the plates were equilibrated at room temperature for 30 min. CellTiter-Glo^{®} Reagent (Promega G7573) was added at 100 µL/well. The plates were shaken for 12 min on a microplate shaker. The luminescence value was measured at room temperature by the GloMax navigator (Promega GM2010). The IC₅₀ values were calculated using the GraphPad Prism 7 software with the 0 nM control group as 0% inhibition.

The antiproliferative activity of the compounds against MV-4-11 cells is shown in Table 2, where A denotes IC₅₀ < 1 nM, B denotes 1 nM ≤ IC₅₀ < 10 nM, C denotes 10 nM ≤ IC₅₀ < 100 nM, D denotes 100 nM ≤ IC₅₀ < 1,000 nM, and E denotes IC₅₀ ≥ 1,000 nM.

**Table 2. Antiproliferative activity**

| **Compound** | **IC₅₀** | **Compound** | **IC₅₀** | **Compound** | **IC₅₀** |
|---|---|---|---|---|---|
| FDAB-1 | E | FDAB-64 | D | FDAB-127 | E |
| FDAB-2 | E | FDAB-65 | C | FDAB-128 | C |
| FDAB-3 | E | FDAB-66 | C | FDAB-129 | C |
| FDAB-4 | E | FDAB-67 | B | FDAB-130 | C |
| FDAB-5 | E | FDAB-68 | B | FDAB-131 | A |
| FDAB-6 | C | FDAB-69 | B | FDAB-132 | A |
| FDAB-7 | E | FDAB-70 | D | FDAB-133 | A |
| FDAB-8 | E | FDAB-71 | E | FDAB-134 | B |
| FDAB-9 | E | FDAB-72 | E | FDAB-135 | B |
| FDAB-10 | E | FDAB-73 | E | FDAB-136 | B |
| FDAB-11 | E | FDAB-74 | E | FDAB-137 | B |
| FDAB-12 | E | FDAB-75 | B | FDAB-138 | A |
| FDAB-13 | E | FDAB-76 | E | FDAB-139 | B |
| FDAB-14 | E | FDAB-77 | E | FDAB-140 | A |
| FDAB-15 | E | FDAB-78 | E | FDAB-141 | E |
| FDAB-16 | C | FDAB-79 | C | FDAB-142 | A |
| FDAB-17 | D | FDAB-80 | E | FDAB-143 | A |
| FDAB-18 | C | FDAB-81 | E | FDAB-144 | A |
| FDAB-19 | B | FDAB-82 | E | FDAB-145 | A |
| FDAB-20 | B | FDAB-83 | C | FDAB-146 | A |
| FDAB-21 | E | FDAB-84 | A | FDAB-147 | A |
| FDAB-22 | E | FDAB-85 | C | FDAB-148 | B |
| FDAB-23 | C | FDAB-86 | B | FDAB-149 | E |
| FDAB-24 | B | FDAB-87 | A | FDAB-150 | C |
| FDAB-25 | E | FDAB-88 | E | FDAB-151 | D |
| FDAB-26 | E | FDAB-89 | E | FDAB-152 | A |
| FDAB-27 | E | FDAB-90 | B | FDAB-153 | A |
| FDAB-28 | E | FDAB-91 | A | FDAB-154 | B |
| FDAB-29 | E | FDAB-92 | B | FDAB-155 | E |
| FDAB-30 | E | FDAB-93 | A | FDAB-156 | B |
| FDAB-31 | E | FDAB-94 | A | FDAB-157 | A |
| FDAB-32 | C | FDAB-95 | B | FDAB-158 | E |
| FDAB-33 | E | FDAB-96 | B | FDAB-159 | E |
| FDAB-34 | E | FDAB-97 | B | FDAB-160 | A |
| FDAB-35 | C | FDAB-98 | B | FDAB-161 | A |
| FDAB-36 | E | FDAB-99 | C | FDAB-162 | A |
| FDAB-37 | C | FDAB-100 | A | FDAB-163 | E |
| FDAB-38 | E | FDAB-101 | A | FDAB-164 | A |
| FDAB-39 | E | FDAB-102 | E | FDAB-165 | B |
| FDAB-40 | E | FDAB-103 | A | FDAB-166 | B |
| FDAB-41 | E | FDAB-104 | E | FDAB-167 | B |
| FDAB-42 | E | FDAB-105 | B | FDAB-168 | C |
| FDAB-43 | E | FDAB-106 | C | FDAB-169 | C |
| FDAB-44 | E | FDAB-107 | E | FDAB-170 | C |
| FDAB-45 | B | FDAB-108 | E | FDAB-171 | D |
| FDAB-46 | B | FDAB-109 | B | FDAB-172 | A |
| FDAB-47 | B | FDAB-110 | A | FDAB-173 | A |
| FDAB-48 | C | FDAB-111 | E | FDAB-174 | C |
| FDAB-49 | E | FDAB-112 | C | FDAB-175 | A |
| FDAB-50 | B | FDAB-113 | C | FDAB-176 | B |
| FDAB-51 | C | FDAB-114 | E | FDAB-177 | A |
| FDAB-52 | B | FDAB-115 | E | FDAB-178 | A |
| FDAB-53 | C | FDAB-116 | B | FDAB-179 | A |
| FDAB-54 | C | FDAB-117 | B | FDAB-180 | C |
| FDAB-55 | B | FDAB-118 | C | FDAB-181 | B |
| FDAB-56 | E | FDAB-119 | C | FDAB-182 | B |
| FDAB-57 | E | FDAB-120 | C | FDAB-183 | A |
| FDAB-58 | C | FDAB-121 | B | FDAB-184 | A |
| FDAB-59 | E | FDAB-122 | D | FDAB-185 | B |
| FDAB-60 | C | FDAB-123 | C | FDAB-186 | A |
| FDAB-61 | B | FDAB-124 | C | FDAB-187 | A |
| FDAB-62 | E | FDAB-125 | E | FDAB-188 | B |
| FDAB-63 | E | FDAB-126 | E | | |

### Example 15: Antiproliferative activity of compounds against various tumor cells

U937, NCI-H929, AML-2, and MOLM-13 cells in the logarithmic growth phase were diluted in media RPMI + 10% FBS, RPMI + 10% FBS, MEMα + 20% FBS, RPMI + 20% FBS, respectively, and plated at 4,000 cells/well in 96-well whole black plates (WHB) with 50 µL/well. After plating, the plates were incubated in an incubator at 5% CO₂ and 37 °C for 24 h before the compounds were added. The compounds were prepared into a 10 mM stock solution in DMSO and diluted in a culture medium to the desired concentrations (a final DMSO concentration of 0.2%). 50 µL of the compound dilutions at final concentrations of 10,000, 3,333.33, 1,111.11, 370.37, 123.46, 41.15, 13.72, 4.57, 1.52, and 0 nM were added to the 96-well plates containing the cells, that is, each compound was serially diluted to 10 concentrations in duplicate. The plates were incubated at 5% CO₂ and 37 °C for 72 h. After the incubation was complete, the plates were equilibrated at room temperature for 30 min. CellTiter-Glo^{®} Reagent (Promega G7573) was added at 100 µL/well. The plates were shaken for 12 min on a microplate shaker. The luminescence value was measured at room temperature by the GloMax navigator (Promega GM2010). The IC₅₀ values were calculated using the GraphPad Prism 7 software with the 0 nM control group as 0% inhibition.

The antiproliferative activity of the compounds against various tumor cells is shown in Table 3, where A denotes IC₅₀ < 1 nM, B denotes 1 nM ≤ IC₅₀ < 10 nM, C denotes 10 nM ≤ IC₅₀ < 100 nM, D denotes 100 nM ≤ IC₅₀ < 1,000 nM, and E denotes IC₅₀ ≥ 1,000 nM.

**Table 3. Antiproliferative activity IC₅₀**

| **Compound** | **NCI-H929** | **U937** | **MOLM-13** | **AML-2** |
|---|---|---|---|---|
| FDAB-46 | B | C | D | D |
| FDAB-48 | B | C | C | D |
| FDAB-65 | B | C | D | D |
| FDAB-68 | B | C | C | C |
| FDAB-92 | C | B | B | B |
| FDAB-94 | B | A | A | A |
| FDAB-100 | A | A | A | B |
| FDAB-121 | B | C | C | C |
| FDAB-124 | D | C | C | C |
| FDAB-137 | B | B | B | B |
| FDAB-142 | A | B | A | B |
| FDAB-144 | A | B | A | B |
| FDAB-147 | A | A | A | A |
| FDAB-156 | B | B | C | C |
| FDAB-157 | B | B | A | B |

### Example 16: Apoptotic effect of compounds on AML cells

MV-4-11 cells in the logarithmic growth phase were diluted in a growth medium and plated at 600,000 cells/well in 6-well plates with 2 mL/well and the compounds were added. The cells were collected, washed twice with pre-cooled PBS, and resuspended in 100 µL of 1X annexin-binding buffer. 5 µL of FITC and 1 µL of 100 µg/mL PI were added. The cells were incubated at room temperature in the dark for 15 min and transferred on ice, and 400 µL of 1X annexin-binding buffer was added. The mixture was gently mixed and subjected to flow cytometry.

Results: after the cells were treated with compounds FDAB-46 and FDAB-65 for 7 h, no apoptotic effect on MV-4-11 cells was observed on the compounds at concentrations of 10 µM, 1 µM, and 100 nM. Whereas, after treating with compounds FDAB-46 and FDAB-65 for 16 h, apoptosis was induced by the compounds at concentrations of 1 µM and 100 nM.

### Example 17: Regulatory effect of compounds on GSPT1 protein

NB-4 cells were cultured in an RPMI 1640 medium (containing 10% FBS), centrifuged, counted, adjusted to a proper cell concentration, and plated on 12-well plates at 10⁶ cells/well with 1350 µL/well. 150 µL of DMSO and the compounds (1 µM) were added, and the mixture was incubated in an incubator at 5% CO₂ and 37 °C for 6 h. The cells were centrifuged, and the culture medium was discarded. PBS was added for washing and then discarded. The whole cell lysate was prepared using a RIPA lysis buffer containing a protease inhibitor cocktail and a phosphatase inhibitor, and incubated on ice for 30 min. The mixture was centrifuged, and the cell debris was discarded. The whole cell lysate supernatant was collected and transferred to a fresh EP tube. Samples were prepared using a loading buffer after a BCA protein assay. The samples were incubated in a metal bath at 100 °C for 10 min.

The samples were electrophoretically separated from proteins on a 4-20% precast gel (SDS-PAGE gel), transferred to a PVDF membrane, blocked with 5% NFDM/TBST for 1 h at room temperature, co-incubated overnight at 4 °C with the primary antibody, and co-incubated for 2 h at room temperature with the secondary antibody on the next day. The signals were detected on the MINICHEMI^{™} imaging system.

The following are antibodies used in this example:
Anti-eRF3/GSPT1: Cell Signaling Technology #14980s
Anti-GAPDH: Huabio ET601-4
Goatanti-Rabbit IgG-HRP antibody: Huabio HA1001

The protein degradation effect on GSPT1 is shown in Table 4, where A denotes a percentage of GSPT1 protein degradation not less than 80%, B denotes a percentage of degradation less than 80% but not less than 50%, C denotes a percentage of degradation less than 50% but not less than 25%, and D denotes a percentage of degradation less than 25%.

**Table 4. Degradation of GSPT1 protein induced by compounds**

| Compound | GSPT1 degradation | Compound | GSPT1 degradation | Compound | GSPT1 degradation |
|---|---|---|---|---|---|
| FDAB-1 | D | FDAB-58 | A | FDAB-115 | D |
| FDAB-2 | D | FDAB-59 | A | FDAB-116 | A |
| FDAB-3 | D | FDAB-60 | A | FDAB-117 | A |
| FDAB-4 | D | FDAB-61 | A | FDAB-118 | B |
| FDAB-5 | D | FDAB-62 | D | FDAB-119 | A |
| FDAB-6 | B | FDAB-63 | D | FDAB-120 | A |
| FDAB-7 | D | FDAB-64 | B | FDAB-121 | A |
| FDAB-8 | D | FDAB-65 | A | FDAB-122 | D |
| FDAB-9 | D | FDAB-66 | A | FDAB-123 | B |
| FDAB-10 | D | FDAB-67 | A | FDAB-124 | A |
| FDAB-11 | D | FDAB-68 | B | FDAB-125 | D |
| FDAB-12 | D | FDAB-69 | B | FDAB-126 | D |
| FDAB-13 | D | FDAB-70 | C | FDAB-127 | D |
| FDAB-14 | D | FDAB-71 | D | FDAB-128 | B |
| FDAB-15 | D | FDAB-72 | D | FDAB-129 | B |
| FDAB-16 | D | FDAB-73 | D | FDAB-130 | B |
| FDAB-17 | D | FDAB-74 | D | FDAB-131 | A |
| FDAB-18 | A | FDAB-75 | B | FDAB-132 | A |
| FDAB-19 | A | FDAB-76 | D | FDAB-133 | A |
| FDAB-20 | A | FDAB-77 | D | FDAB-134 | B |
| FDAB-21 | D | FDAB-78 | D | FDAB-135 | A |
| FDAB-22 | D | FDAB-79 | A | FDAB-136 | A |
| FDAB-23 | A | FDAB-80 | D | FDAB-137 | A |
| FDAB-24 | A | FDAB-81 | D | FDAB-138 | A |
| FDAB-25 | D | FDAB-82 | D | FDAB-139 | A |
| FDAB-26 | D | FDAB-83 | A | FDAB-140 | A |
| FDAB-27 | D | FDAB-84 | A | FDAB-141 | D |
| FDAB-28 | D | FDAB-85 | A | FDAB-142 | A |
| FDAB-29 | D | FDAB-86 | A | FDAB-143 | A |
| FDAB-30 | D | FDAB-87 | D | FDAB-144 | A |
| FDAB-31 | D | FDAB-88 | D | FDAB-145 | A |
| FDAB-32 | A | FDAB-89 | D | FDAB-146 | A |
| FDAB-33 | D | FDAB-90 | A | FDAB-147 | A |
| FDAB-34 | D | FDAB-91 | A | FDAB-148 | A |
| FDAB-35 | B | FDAB-92 | A | FDAB-149 | D |
| FDAB-36 | D | FDAB-93 | A | FDAB-150 | C |
| FDAB-37 | B | FDAB-94 | A | FDAB-151 | D |
| FDAB-38 | D | FDAB-95 | A | FDAB-152 | A |
| FDAB-39 | D | FDAB-96 | A | FDAB-153 | A |
| FDAB-40 | D | FDAB-97 | A | FDAB-154 | A |
| FDAB-41 | D | FDAB-98 | A | FDAB-155 | D |
| FDAB-42 | D | FDAB-99 | B | FDAB-156 | A |
| FDAB-43 | D | FDAB-100 | A | FDAB-157 | A |
| FDAB-44 | D | FDAB-101 | A | FDAB-158 | D |
| FDAB-45 | A | FDAB-102 | D | FDAB-159 | D |
| FDAB-46 | A | FDAB-103 | A | FDAB-160 | A |
| FDAB-47 | A | FDAB-104 | D | FDAB-161 | A |
| FDAB-48 | A | FDAB-105 | A | FDAB-162 | A |
| FDAB-49 | D | FDAB-106 | A | FDAB-163 | D |
| FDAB-50 | A | FDAB-107 | D | FDAB-164 | A |
| FDAB-51 | A | FDAB-108 | D | FDAB-165 | A |
| FDAB-52 | A | FDAB-109 | A | FDAB-166 | A |
| FDAB-53 | A | FDAB-110 | A | FDAB-167 | A |
| FDAB-54 | B | FDAB-111 | D | FDAB-168 | A |
| FDAB-55 | A | FDAB-112 | A | FDAB-169 | B |
| FDAB-56 | D | FDAB-113 | A | FDAB-170 | A |
| FDAB-57 | D | FDAB-114 | D | FDAB-171 | D |

In addition, after test, compound FDAB-48 of the present disclosure demonstrated an IC₅₀ of 10.8 nM against MV-4-11 cell proliferation and an 88% degradation rate of GSPT1 protein when being co-incubated for 6 h with NB-4 cells, while the corresponding unsubstituted compound 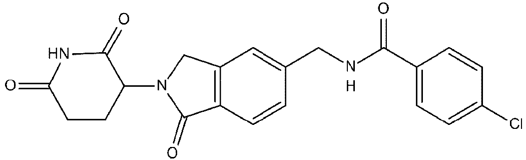 demonstrated an IC₅₀ of 102.6 nM against MV-4-11 cell proliferation and only a 65% degradation rate of GSPT1 protein when being co-incubated for 6 h with NB-4 cells.

### Example 18: Regulatory effect of compounds on IKZF1, IKZF3, CK1α, and c-MYC proteins

HL-60 cells were cultured in an IMDM medium (containing 20% FBS), centrifuged, counted, adjusted to a proper cell concentration, and plated on 12-well plates at 1.2E6 cells/well with 1350 µL/cell. 150 µL of DMSO and the compounds were added, and the mixture was incubated in an incubator at 5% CO₂ and 37 °C for 4 h. The cells were centrifuged, and the culture medium was discarded. PBS was added for washing and then discarded. The whole cell lysate was prepared using a RIPA lysis buffer containing a protease inhibitor cocktail and a phosphatase inhibitor, and incubated on ice for 30 min. The mixture was centrifuged, and the cell debris was discarded. The whole cell lysate supernatant was collected and transferred to a fresh EP tube. Samples were prepared using a loading buffer after a BCA protein assay. The samples were incubated in a metal bath at 100 °C for 10 min. The samples were electrophoretically separated from proteins on a 4-20% precast gel (SDS-PAGE gel), transferred to a PVDF membrane, blocked with 5% NFDM/TBST for 1 h at room temperature, co-incubated overnight at 4 °C with the primary antibody, and co-incubated for 2 h at room temperature with the secondary antibody on the next day. The signals were detected on the MINICHEMI^{™} imaging system.

The following are antibodies used in this example:
Anti-eRF3/GSPT1: Cell Signaling Technology# 14980s
Anti-c-Myc: Cell Signaling Technology #9402s
Anti-Casein Kinase 1 alpha: Abcam ab206652
Anti-ikaros (IKZF1): Abcam ab191394
Anti-IKZF3: Abcam 139408
Anti-beta Tubulin: Huabio SR25-04
Goatanti-Rabbit IgG-HRP: Huabio HA1001

Table 5 shows the ability of compounds to degrade IKZF1, IKZF3, CK1α, and c-Myc proteins at different concentrations in HL-60 cells, where A denotes a percentage of GSPT1 protein degradation not less than 80%, B denotes a percentage of degradation less than 80% but not less than 50%, C denotes a percentage of degradation less than 50% but not less than 25%, and D denotes a percentage of degradation less than 25%.

**Table 5. Selective degradation of IKZF1, IKZF3, CK1α, and c-MYC proteins by compounds**

| Compound | Concentration (nM) | IKZF1 | IKZF3 | CK1α | c-Myc |
|---|---|---|---|---|---|
| FDAB-48 | 10 | B | C | D | D |
| | 100 | A | B | D | D |
| | 1000 | A | A | D | C |
| FDAB-121 | 10 | B | D | D | D |
| | 100 | A | B | D | D |
| | 1000 | A | B | C | D |
| FDAB-153 | 10 | / | / | D | D |
| | 100 | / | / | D | D |
| | 1000 | / | / | D | B |
| FDAB-164 | 10 | / | / | D | D |
| | 100 | / | / | C | C |
| | 1000 | / | / | C | C |

| | | | | | |
|---|---|---|---|---|---|
| Note: "/" denotes that no test was performed. | | | | | |

### Example 19: Antiproliferative effect of combinations of compounds with other medicaments

MOLM-13 cells in the logarithmic growth phase were diluted in a medium RPMI + 20% FBS, and NCI-H929 cells were diluted in a medium RPMI + 10% FBS. The cells were plated at 4,000 cells/well in 96-well whole black plates (WHB) with 50 µL/well. After plating, the plates were incubated in an incubator at 5% CO₂ and 37 °C for 24 h before the compounds were added. The compounds were prepared into a 10 mM stock solution in DMSO and diluted in a culture medium to the desired concentrations (a final DMSO concentration of 0.2%). 50 µL of the compound dilutions at final concentrations of 10,000, 3,333.33, 1,111.11, 370.37, 123.46, 41.15, 13.72, 4.57, 1.52, and 0 nM were added to the 96-well plates containing the cells, that is, each compound was serially diluted to 10 concentrations in duplicate. The plates were incubated at 5% CO₂ and 37 °C for 72 h. When pharmaceutical partners were used, the concentrations of azacitidine and dexamethasone were kept at 200 nM and 500 nM respectively, and the compounds were serially three-fold diluted from 10,000 nM. After the incubation was complete, the plates were equilibrated at room temperature for 30 min. CellTiter-Glo^{®} Reagent (Promega G7573) was added at 100 µL/well. The plates were shaken for 12 min on a microplate shaker. The luminescence value was measured at room temperature by the GloMax navigator (Promega GM2010). The IC₅₀ values were calculated using the GraphPad Prism 7 software with the 0 nM control group as 0% inhibition.

**Table 6. Antiproliferative effect of compounds in combination with azacitidine against MOLM-13 cells**

| **Compound** | **IC₅₀ (nM)** |
|---|---|
| FDAB-121 | 247.9 |
| CC-90009 | 1422 |
| Azacitidine | 751.1 |
| FDAB-121 (200 nM azacitidine) | 9.675 |
| CC-90009 (200 nM azacitidine) | 43.85 |

**Table 7. Antiproliferative effect of compounds in combination with dexamethasone against NCI-H929 cells**

| **Compound** | **IC₅₀ (nM)** |
|---|---|
| FDAB-48 | 14.26 |
| CC-90009 | 3197 |
| Lenalidomide | 2177 |
| Pomalidomide | 243.2 |
| Dexamethasone | >10000 |
| FDAB-48 (500 nM dexamethasone) | 2.447 |
| CC-90009 (500 nM dexamethasone) | 77.33 |
| Lenalidomide (500 nM dexamethasone) | 773.5 |
| Pomalidomide (500 nM dexamethasone) | 22.22 |

The test results are shown in Table 6. As can be seen from Table 6, the antiproliferative activity IC₅₀ of azacitidine against MOLM-13 cells was 751.1 nM, and no significant antiproliferative activity against MOLM-13 cells was found for 200 nM azacitidine. However, when FDAB-121 was combined with 200 nM azacitidine, the antiproliferative activity IC₅₀ of FDAB-121 against MOLM-13 cells was increased from 247.9 nM to 9.675 nM, showing an increase of about 25 folds. FDAB-121, either alone or in combination with azacitidine, exhibited an activity of about 5 folds greater than CC-90009.

As shown in Table 7, the antiproliferative activity IC₅₀ of dexamethasone against NCI-H929 cells was greater than 10,000 nM, while no significant antiproliferative activity against NCI-H929 cells was found for 500 nM dexamethasone. However, when FDAB-48 was combined with 500 nM dexamethasone, the antiproliferative activity IC₅₀ of FDAB-48 against NCI-H929 cells was increased from 14.26 nM to 2.447 nM, showing an increase of about 6 folds. In addition, the compound FDAB-48, either alone or in combination with dexamethasone, had a far higher antiproliferative activity against NCI-H929 cells than those of CC-90009 and first-line treatments for myeloma lenalidomide and pomalidomide.

### Example 20: hERG inhibition assay

The rapidly activating potassium channel encoded by human ether-a-go-go-related gene (hERG) is an important ion channel involved in the formation of cardiac action potential phase 3 repolarization. Blocking of the hERG channel by drugs can lead to the prolongation of cardiac repolarization and thus a prolonged QT interval in the electrocardiogram, known as long QT syndrome. Drug-induced delayed ventricular repolarization may in some cases trigger fatal arrhythmia-torsade de pointes-type ventricular tachycardia.

This assay was used to measure the effect of compounds on the hERG potassium channel clones expressed in human embryonic kidney (HEK293) cells. The assay procedures are as follows: the cells were transferred into a HEPES-buffered saline in glass-lined 96-well plates and exposed to an appropriate amount of test compound or control solution at various concentrations for 3 min. The test compounds were diluted in 0.3% DMSO. The automated parallel patch clamp system QPatch 48X (Sophion) was used to test the hERG inhibition rate by compounds at 3 µM.

**Result:** The hERG inhibition rates of compounds FDAB-48, FDAB-65, FDAB-120, and FDAB-121 at 3 µM were 25%, 11%, 13% and 9%, respectively, indicating that compounds FDAB-65, FDAB-120, and FDAB-121 have barely observable hERG inhibitory activity (< 20% inhibition) at 3 µM, while compound FDAB-48 has only minor hERG inhibitory activity (< 25% inhibition). Compound CC-90009 exhibited a significant hERG inhibition of 66% at 3 µM. It can be seen that the compounds of the present disclosure have surprisingly reduced inhibitory activity against the hERG channel.

### Example 21: Efficacy study in acute myeloid leukemia xenograft mouse model

In an *in vivo* efficacy study, female immunocompromised NSG mice aged 6 to 8 weeks were used and the experimental procedures started after the animals were acclimatized in the experimental environment for 7 days. The animals were housed in SPF animal rooms in individually ventilated cages (IVCs) each containing 6 mice. Human acute myeloid leukemia MV-4-11 cells expressing luciferase (MV-4-11-luc) were grafted into mice by tail vein injection (5 × 10⁶ cells/mouse). Seven days after the cell grafting, the mice were subjected to imaging on the IVIS Lumina III small animal imaging system for grouping and administration, and tumor-bearing mice were randomized to the vehicle control group or treatment groups (6 mice/group). The compound (10 mg/kg, twice daily) was administered to the animals in each treatment group by oral gavage. The body weight was measured daily, while mean luminescence was measured after the start of treatment with the compounds or vehicle.

Indicators: The indicators were to investigate whether the tumor growth was inhibited or delayed or the tumor was cured. The tumor size was measured twice weekly using the IVIS Lumina III small animal imaging system. The tumor signals were determined by exposure photons per second. The anti-tumor efficacy of the compounds was evaluated by the tumor proliferation rate T/C (%). T/C % = T_{RTV}/C_{RTV} × 100% (T_{RTV}: the RTV of the treatment group; C_{RTV}: the RTV of negative control group; RTV = Vₜ/V₀, where V₀ is the tumor signal measured at the time of grouping and administration (i.e., day 0), and Vₜ is the tumor signal at every measurement). According to the guidelines of the Center for Drug Evaluation, NMPA, a T/C (%) of > 40% denotes the absence of efficacy, while a T/C of ≤ 40% with P < 0.05 denotes the presence of efficacy.

**Results:** The tumor proliferation rates T/C (%) of the compounds at different time points are shown in Table 8. The compound CC-90009, when administered at 10 mg/kg orally twice daily, exhibited no tumor inhibitory effects within 14 days. The T/C (%) on day 14 for the compounds of the present disclosure, FDAB-48, FDAB-65, FDAB-120, and FDAB-121, when administered at 10 mg/kg orally twice daily, were 0.05%, 0.78%, 2.12%, and 0.005%, respectively. As can be seen, all the treatment groups of the compounds of the present disclosure demonstrated significant anti-tumor effects. Surprisingly, the compounds of the present disclosure exhibited significant tumor inhibitory effects on day 3 of administration, and the T/C (%) on day 3 for FDAB-48, FDAB-65, FDAB-120, and FDAB-121, when administered at 10 mg/kg orally twice daily, were 0.95%, 4.58%, 6.69%, and 0.32%, respectively.

**Table 8. Anti-tumor activity of compounds in MV-4-11-Luc xenograft model**

| **Group** | T/C (%)* | | | | |
|---|---|---|---|---|---|
| | **Day 0** | **Day 3** | **Day 7** | **Day 10** | **Day 14** |
| Blank group | - | - | - | - | - |
| CC-90009,10 mg/kg | | 100.80% | 133.08% | 127.05% | 132.90% |
| FDAB-48, 10 mg/kg | - | 0.95% | 0.22% | 0.10% | 0.05% |
| FDAB-65,10 mg/kg | - | 4.58% | 1.40% | 1.01% | 0.78% |
| FDAB-120,10 mg/kg | - | 6.69% | 1.58% | 2.00% | 2.12% |
| FDAB-121,10 mg/kg | - | 0.32% | 0.06% | 0.02% | 0.00% |

The embodiments of the present disclosure have been described above. However, the present disclosure is not limited to the embodiments described above. Any modification, equivalent substitution, improvement, and the like made without departing from the spirit and principle of the present disclosure shall fall within the claimed scope of the present disclosure.

## Claims

1. A compound of formula I, or a tautomer, a stereoisomer, a hydrate, a solvate, or a pharmaceutically acceptable salt thereof: 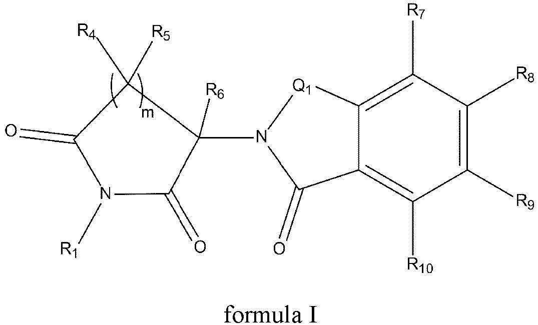
wherein R₁ is selected from hydrogen, deuterium, C₁₋₁₂ alkyl, 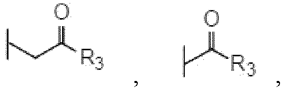 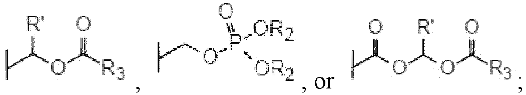
R₂ are identical or different and are each independently selected from hydrogen and the following groups unsubstituted or optionally substituted with one, two, or more Ra: C₁₋₁₂ alkyl, C₆₋₂₀ aryl, 5-to 20-membered heteroaryl, C₃₋₂₀ cycloalkyl, and 3- to 20-membered heterocyclyl;
R' and R₃ are identical or different and are each independently selected from hydrogen and the following groups unsubstituted or optionally substituted with one, two, or more Rb: C₁₋₁₂ alkyl, C₁₋₁₂ alkoxy, C₆₋₂₀ aryl, 5- to 20-membered heteroaryl, C₃₋₂₀ cycloalkyl, and 3- to 20-membered heterocyclyl;
R₄ and R₅ are identical or different and are each independently selected from hydrogen, deuterium, and halogen;
m is 1, 2, or 3;
R₆ is selected from hydrogen, deuterium, halogen, and the following groups unsubstituted or optionally substituted with one, two, or more Rc: C₁₋₁₂ alkyl and C₁₋₁₂ alkoxy;
Q₁ is -CH₂-, -CD₂-, -C(O)-, or -C(S)-;
R₇, R₈, R₉, and R₁₀ are identical or different and are each independently selected from hydrogen, halogen, deuterium, CN, C₁₋₁₂ alkyl, -CH₂-NHBoc, -CH₂-NH₂, -CH₂-NHCO-CF₂-R₁₁, -CH₂-NHCO-R₁₂, -CH₂-NHCO-Y-R₁₃, -CH₂-NH-Y-R₁₄, and -CH₂-NHCONH-R₁₂, wherein hydrogen(s) on methylene of -CH₂-NHBoc, -CH₂-NH₂, -CH₂-NHCO-CF₂-R₁₁, -CH₂-NHCO-R₁₂, -CH₂-NHCONH-R₁₂, -CH₂-NHCO-Y-R₁₃, and -CH₂-NH-Y-R₁₄ is(are) optionally substituted with 1 or 2 deuteriums;
R₁₁, R₁₂, R₁₃, and R₁₄ are identical or different and are each independently selected from the following groups unsubstituted or optionally substituted with one, two, or more Rs: C₆₋₂₀ aryl, 5- to 20-membered heteroaryl, C₃₋₂₀ cycloalkyl, 3- to 20-membered heterocyclyl, C₂₋₁₂ alkenyl, C₂₋₁₂ alkynyl, C₁₋₁₂ alkyl, -P(C₆₋₂₀ aryl)₂, -N(C₁₋₁₂ alkyl)₂, -COC₁₋₁₂ alkyl, -C₁₋₁₂ alkyl-N(C₁₋₁₂ alkyl)₂, C₆₋₂₀ aryl fused with C₃₋₂₀ cycloalkyl, and spirocyclyl formed by C₃₋₂₀ cycloalkyl and C₃₋₂₀ cycloalkyl;
Y is selected from C₁₋₁₂ alkylene, C₃₋₂₀ cycloalkylene, C₂₋₁₂ alkenylene, C₂₋₁₂ alkynylene, -CH₂NH-, -CH₂NHCO-, -CH₂NHCO-, -CH₂O-, -C₂H₄O-, -CH₂S-, 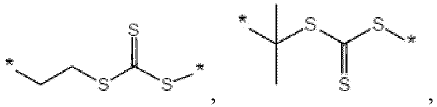 and 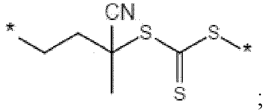
Ra, Rb, Rc, and Rs are identical or different and are each independently selected from halogen, amino, hydroxyl, C₁₋₁₂ alkyl, C₁₋₁₂ alkoxy, halogenated C₁₋₁₂ alkyl, CN, C₃₋₂₀ cycloalkyl, -N(C₁₋₁₂ alkyl)₂, deuterium, C₂₋₁₂ alkenyl, C₂₋₁₂ alkynyl, -COC₁₋₁₂ alkyl, -COC₆₋₂₀ aryl, C₁₋₁₂ alkylthio, sulfhydryl, =O, -C₁₋₁₂ alkylhydroxy, 3- to 20-membered heterocyclyl, -SO₂C₁₋₁₂ alkyl, -SO₂NH₂, C₆₋₂₀ aryl, -OC₆₋₂₀ aryl, -C₁₋₁₂ alkyl-C₆₋₂₀ aryl, -C₁₋₁₂ alkylamino, -C₆-₂₀ aryl-C₁₋₁₂ alkylamino, -C₆-₂₀ arylamino, -OC₁₋₁₂ alkyl-C₆-₂₀ aryl, -C₆-₂₀ arylhydroxy, and -C₆-₂₀ aryl-C₁₋₁₂ alkylhydroxy.

2. The compound according to claim 1, wherein R₁ is selected from hydrogen, C₁₋₆ alkyl, -C₁₋₆ alkyl-COOC₁₋₆ alkyl, -COOC₁₋₆ alkyl, and -C₁₋₆ alkyl-OCOOC₁₋₆ alkyl;
R₄, R₅, and R₆ are hydrogen; m is 1, 2, or 3; Q₁ is -CH₂- or -C(O)-;
R₇, R₈, R₉, and R₁₀ are identical or different and are each independently selected from hydrogen, deuterium, halogen, CN, C₁₋₆ alkyl, -CH₂-NHBoc, -CH₂-NH₂, -CH₂-NHCO-CF₂-R₁₁, -CH₂-NHCO-R₁₂, -CH₂-NHCONH-R₁₂, -CH₂-NHCO-Y-R₁₃, and -CH₂-NH-Y-R₁₄, wherein hydrogen(s) on CH₂ of -CH₂-NHBoc, -CH₂-NH₂, -CH₂-NHCO-CF₂-R₁₁, -CH₂-NHCO-R₁₂, -CH₂-NHCONH-R₁₂, -CH₂-NHCO-Y-R₁₃, and -CH₂-NH-Y-R₁₄ is(are) optionally substituted with 1 or 2 deuteriums;
R₁₁, R₁₂, R₁₃, and R₁₄ are identical or different and are each independently selected from the following groups unsubstituted or optionally substituted with one, two, or more Rs: C₆₋₁₂ aryl, 5- to 12-membered heteroaryl, C₃₋₁₂ cycloalkyl, 3- to 12-membered heterocyclyl, C₂₋₆ alkenyl, C₂₋₆ alkynyl, C₁₋₆ alkyl, -P(C₆₋₂₀ aryl)₂, -N(C₁₋₆ alkyl)₂, -COC₁₋₆ alkyl, -C₁₋₆ alkyl-N(C₁₋₆ alkyl)₂, C₆₋₁₂ aryl fused with C₃₋₁₂ cycloalkyl, and spirocyclyl formed by C₃₋₁₂ cycloalkyl and C₃₋₁₂ cycloalkyl;
Y is selected from C₁₋₆ alkylene, C₃₋₁₂ cycloalkylene, C₂₋₆ alkenylene, C₂₋₆ alkynylene, -CH₂NH-, - CH₂NHCO-, -CH₂NHCO-, -CH₂O-, -C₂H₄O-, -CH₂S-, 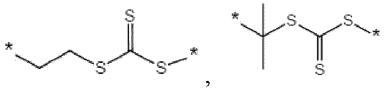 , and 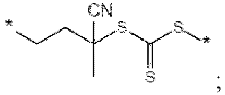
Rs are identical or different and are each independently selected from halogen, amino, hydroxyl, C₁₋₆ alkyl, C₁₋₆ alkoxy, halogenated C₁₋₆ alkyl, CN, C₃₋₁₂ cycloalkyl, -N(C₁₋₆ alkyl)₂, deuterium, C₂₋₆ alkenyl, C₂₋₆ alkynyl, -COC₁₋₆ alkyl, -COC₆₋₁₂ aryl, C₁₋₆ alkylthio, sulfhydryl, =O, -C₁₋₆ alkylhydroxy, 3- to 12-membered heterocyclyl, -SO₂C₁₋₆ alkyl, -SO₂NH₂, C₆₋₁₂ aryl, -OC₆₋₁₂ aryl, - C₁₋₆ alkyl-C₆-₁₂ aryl, -C₁₋₆ alkylamino, -C₆-₁₂ aryl-C₁₋₆ alkylamino, -C₆-₁₂ arylamino, -OC₁₋₆ alkyl-C₆₋₁₂ aryl, -C₆-₁₂ arylhydroxy, and -C₆-₁₂ aryl-C₁₋₆ alkylhydroxy.

3. The compound according to claim 1 or 2, wherein the compound of formula I has the structure of formula Ia: 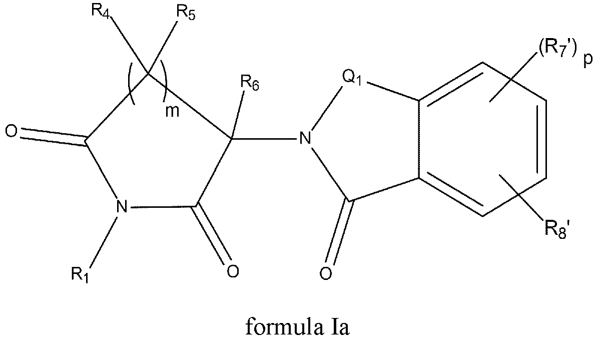
wherein, in formula Ia, R₁ is selected from hydrogen, C₁₋₆ alkyl, -C₁₋₆ alkyl-COOC₁₋₆ alkyl, - COOC₁₋₆ alkyl, and -C₁₋₆ alkyl-OCOOC₁₋₆ alkyl;
R₄, R₅, and R₆ are selected from hydrogen and deuterium; m is 1, 2, or 3; Q₁ is -CH₂-, -CD₂-, or - C(O)-;
R₇' is selected from hydrogen, deuterium, and halogen; p is 1, 2, or 3, with the proviso that at least one R₇' is halogen;
R₈' is selected from CN, -CH₂-NHBoc, -CH₂-NH₂, -CH₂-NHCO-CF₂-R₁₁, -CH₂-NHCO-R₁₂, -CH₂-NHCONH-R₁₂, -CH₂-NHCO-Y-R₁₃, and -CH₂-NH-Y-R₁₄, wherein H atom(s) on CH₂ of -CH₂-NHBoc, -CH₂-NH₂, -CH₂-NHCO-CF₂-R₁₁, -CH₂-NHCO-R₁₂, -CH₂-NHCONH-R₁₂, -CH₂-NHCO-Y-R₁₃, and -CH₂-NH-Y-R₁₄ is(are) optionally substituted with 1 or 2 deuteriums;
R₁₁, R₁₂, R₁₃, and R₁₄ are identical or different and are each independently selected from the following groups unsubstituted or optionally substituted with one, two, or more Rs: C₆₋₁₂ aryl, 5- to 12-membered heteroaryl, C₃₋₁₂ cycloalkyl, 3- to 12-membered heterocyclyl, C₂₋₆ alkenyl, C₂₋₆ alkynyl, C₁₋₆ alkyl, -P(C₆₋₂₀ aryl)₂, -N(C₁₋₆ alkyl)₂, -COC₁₋₆ alkyl, -C₁₋₆ alkyl-N(C₁₋₆ alkyl)₂, C₆₋₁₂ aryl fused with C₃₋₁₂ cycloalkyl, and spirocyclyl formed by C₃₋₁₂ cycloalkyl and C₃₋₁₂ cycloalkyl;
Y is selected from C₁₋₆ alkylene, C₃₋₁₂ cycloalkylene, C₂₋₆ alkenylene, C₂₋₆ alkynylene, -CH₂NH-, - CH₂NHCO-, -CH₂NHCO-, -CH₂O-, -C₂H₄O-, -CH₂S-, 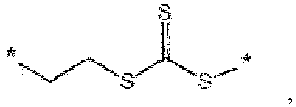 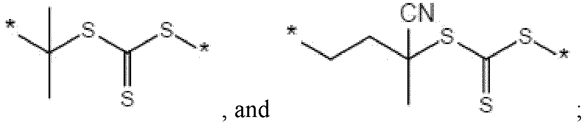
Rs are identical or different and are each independently selected from halogen, amino, hydroxyl, C₁₋₆ alkyl, C₁₋₆ alkoxy, halogenated C₁₋₆ alkyl, CN, C₃₋₁₂ cycloalkyl, -N(C₁₋₆ alkyl)₂, deuterium, C₂₋₆ alkenyl, C₂₋₆ alkynyl, -COC₁₋₆ alkyl, -COC₆₋₁₂ aryl, C₁₋₆ alkylthio, sulfhydryl, =O, -C₁₋₆ alkylhydroxy, 3- to 12-membered heterocyclyl, -SO₂C₁₋₆ alkyl, -SO₂NH₂, C₆₋₁₂ aryl, -OC₆₋₁₂ aryl, - C₁₋₆ alkyl-C₆-₁₂ aryl, -C₁₋₆ alkylamino, -C₆-₁₂ aryl-C₁₋₆ alkylamino, -C₆-₁₂ arylamino, -OC₁₋₆ alkyl-C₆₋₁₂ aryl, -C₆-₁₂ arylhydroxy, and -C₆-₁₂ aryl-C₁₋₆ alkylhydroxy.

4. The compound according to claim 1 or 2, wherein the compound of formula I has the structure of formula Ib: 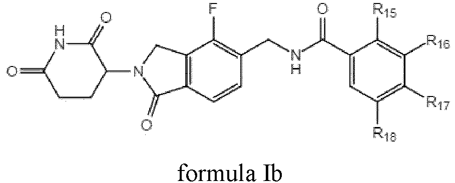
wherein, in formula Ib, R₁₅, R₁₆, R₁₇, and R₁₈ are identical or different and are each independently selected from hydrogen, deuterium, halogen, amino, hydroxyl, cyano, and the following groups unsubstituted or optionally substituted with one, two, or more Rx: C₁₋₆ alkyl, -SO₂C₁₋₆ alkyl, C₁₋₆ haloalkyl, C₆₋₁₂ aryl, C₁₋₆ alkoxy, -N(C₁₋₆ alkyl)₂, C₂₋₆ alkenyl, C₂₋₆ alkynyl, C₃₋₁₂ cycloalkyl, C₁₋₆ alkylthio, -COC₁₋₆ alkyl, -COC₆₋₁₂ aryl, -OC₆₋₁₂ aryl, -C₁₋₆ alkyl-hydroxyl, 3- to 12-membered heterocyclyl, and -SO₂NH₂; and
Rx are identical or different and are each independently selected from halogen, amino, hydroxyl, C₁₋₆ alkyl, C₁₋₆ alkoxy, halogenated C₁₋₆ alkyl, CN, C₃₋₁₂ cycloalkyl, -N(C₁₋₆ alkyl)₂, deuterium, C₂₋₆ alkenyl, C₂₋₆ alkynyl, -COC₁₋₆ alkyl, -COC₆₋₁₂ aryl, C₁₋₆ alkylthio, sulfhydryl, =O, -C₁₋₆ alkylhydroxy, -C₁₋₆ alkylamino, 3- to 12-membered heterocyclyl, -SO₂C₁₋₆ alkyl, -SO₂NH₂, C₆₋₁₂ aryl, -OC₆₋₁₂ aryl, and -C₁₋₆ alkyl-C₆-₁₂ aryl;
or, the compound of formula I has the structure of formula Ic: 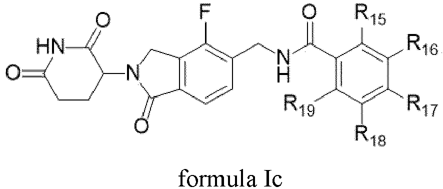
wherein, in formula Ic, R₁₅, R₁₆, R₁₇, R₁₈, and R₁₉ are identical or different and are each independently selected from hydrogen, deuterium, halogen, amino, hydroxyl, cyano, and the following groups unsubstituted or optionally substituted with one, two, or more Rx: C₁₋₆ alkyl, - SO₂C₁₋₆ alkyl, C₁₋₆ haloalkyl, C₆₋₁₂ aryl, C₁₋₆ alkoxy, -N(C₁₋₆ alkyl)₂, C₂₋₆ alkenyl, C₂₋₆ alkynyl, C₃₋₁₂ cycloalkyl, C₁₋₆ alkylthio, -COC₁₋₆ alkyl, -COC₆₋₁₂ aryl, -OC₆₋₁₂ aryl, -C₁₋₆ alkyl-hydroxyl, 3- to 12-membered heterocyclyl, and -SO₂NH₂;
Rx are identical or different and are each independently selected from halogen, amino, hydroxyl, C₁₋₆ alkyl, C₁₋₆ alkoxy, halogenated C₁₋₆ alkyl, CN, C₃₋₁₂ cycloalkyl, -N(C₁₋₆ alkyl)₂, deuterium, C₂₋₆ alkenyl, C₂₋₆ alkynyl, -COC₁₋₆ alkyl, -COC₆₋₁₂ aryl, C₁₋₆ alkylthio, sulfhydryl, =O, -C₁₋₆ alkylhydroxy, -C₁₋₆ alkylamino, 3- to 12-membered heterocyclyl, -SO₂C₁₋₆ alkyl, -SO₂NH₂, C₆₋₁₂ aryl, -OC₆₋₁₂ aryl, and -C₁₋₆ alkyl-C₆-₁₂ aryl, and
R₁₉ is not H;
or, the compound of formula I has the structure of formula Id: 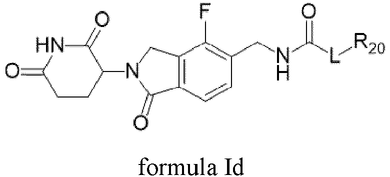
wherein, in formula Id, L is a chemical bond, C₂₋₁₂ alkynylene, C₂₋₁₂ alkenylene, NH, oxygen, sulfur, or C₁₋₁₂ alkylene, wherein the C₁₋₁₂ alkylene is optionally substituted with the following group: hydroxyl or amino;
R₂₀ is the following groups unsubstituted or optionally substituted with one, two, or more Ry: C₆₋₁₂ aryl, 3- to 12-membered cycloalkyl, 5- to 12-membered heterocyclyl, or 5- to 12-membered heteroaryl; and
Ry is selected from deuterium, halogen, hydroxyl, amino, carboxyl, CN, C₁₋₆ alkyl, C₁₋₆ alkoxy, halogenated C₁₋₆ alkyl, C₃₋₁₂ cycloalkyl, -N(C₁₋₆ alkyl)₂, -COC₁₋₆ alkyl, -COC₆₋₁₂ aryl, C₁₋₆ alkylthio, sulfhydryl, =O, -C₁₋₆ alkylhydroxy, -C₁₋₆ alkylamino, 3- to 12-membered heterocyclyl, -SO₂C₁₋₆ alkyl, -SO₂NH₂, C₆₋₁₂ aryl, -OC₆₋₁₂ aryl, -OC₁₋₆ alkyl-C₆-₁₂ aryl, -C₁₋₆ alkyl-C₆-₁₂ aryl, -C₁₋₆ alkyl-COOH, -C₆-₁₂ aryl-C₁₋₆ alkylamino, -C₆-₁₂ aryl-C₁₋₆ alkylhydroxy, -C₆-₁₂ arylamino, and -C₆-₁₂ arylhydroxy.

5. The compound according to claim 4, wherein in formula Ib, R₁₅, R₁₆, R₁₇, and R₁₈ are identical or different and are each independently selected from hydrogen, deuterium, F, Cl, methyl, amino, hydroxyl, methoxy, trifluoromethyl, cyano, phenyl, dimethylamino, ethyl, n-propyl, isopropyl, *tert-*butyl, vinyl, ethynyl, cyclopropyl, carbonylmethyl, carbonylphenyl, phenoxy, tert-butoxy, alkylthio, -SO₂NH₂, hydroxymethyl, *N*-tetrahydropyrrolyl, -SO₂CH₂, *p*-aminophenyl, 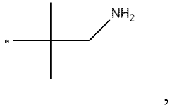 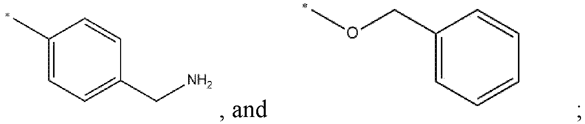 preferably, in formula Id, L is a chemical bond, alkynylene, NH, 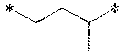 , or 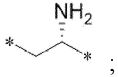
R₂₀ is phenyl; and
Ry is selected from Cl, hydroxyl, amino, *tert*-butyl, phenyl, *p*-aminophenyl, *p*-hydroxyphenyl, ethylamino, hydroxyethyl, *p*-hydroxyethylphenyl, *p*-ethylaminophenyl,

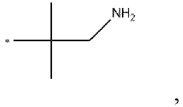

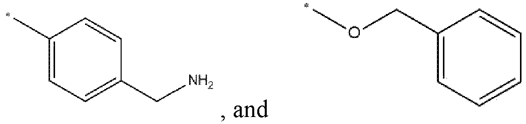

6. The compound according to any one of claims 1-5, wherein the compound of formula I is selected from:

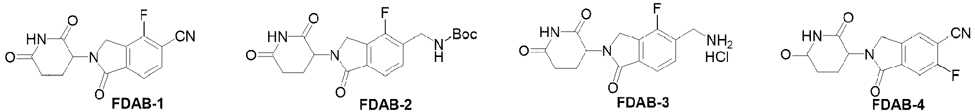

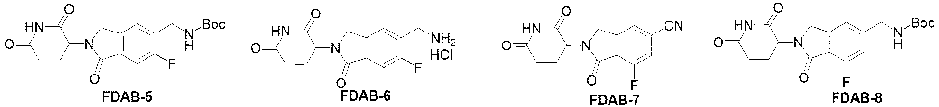

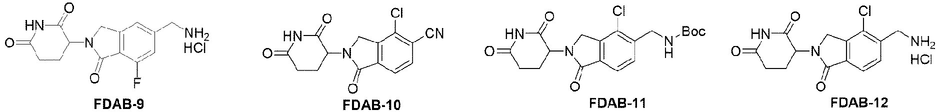

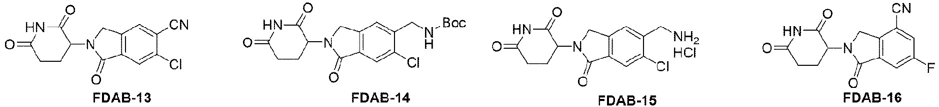

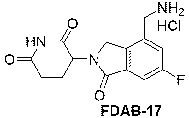

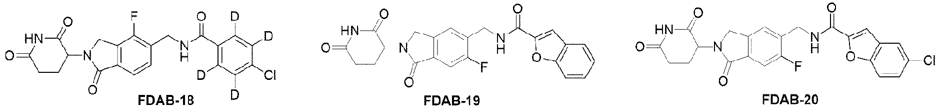

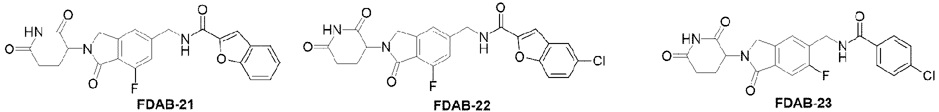

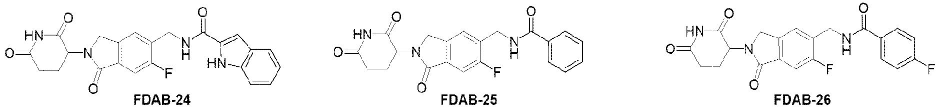

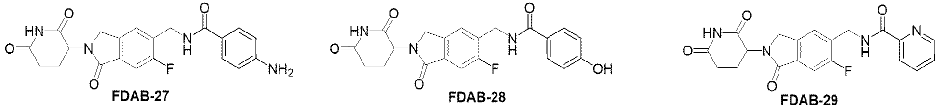

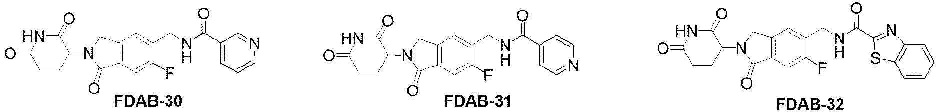

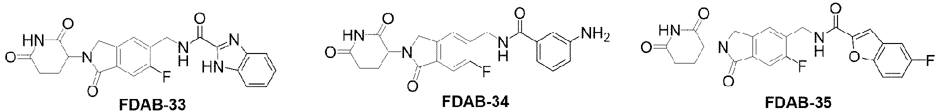

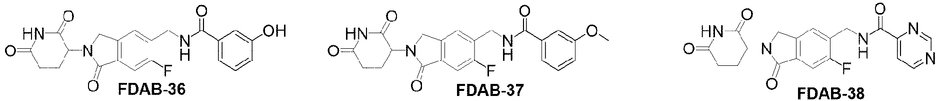

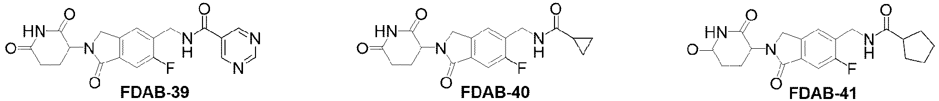

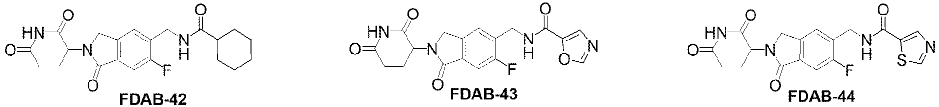

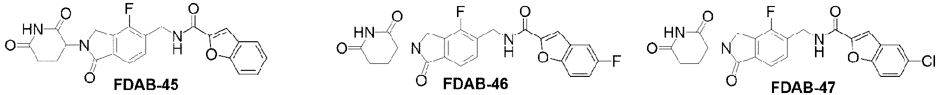

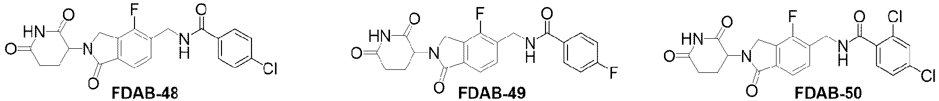

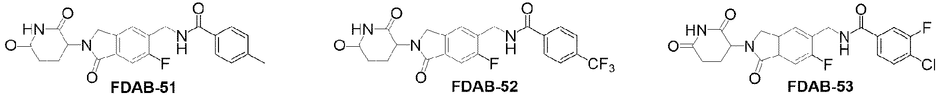

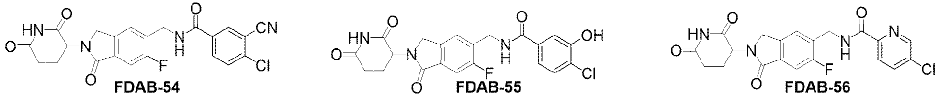

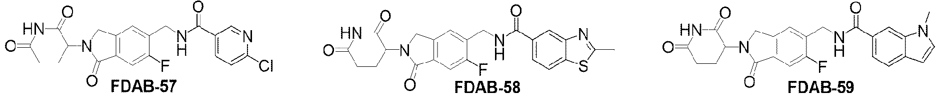

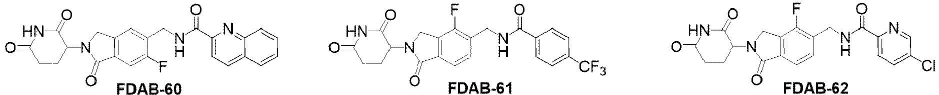

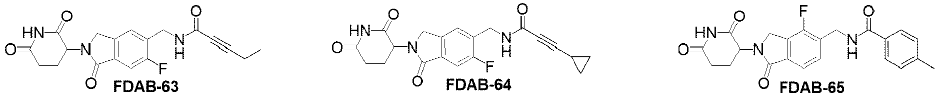

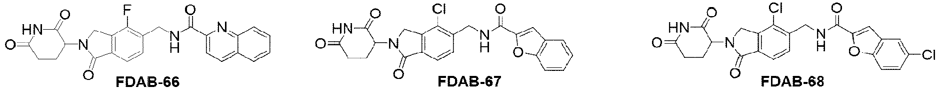

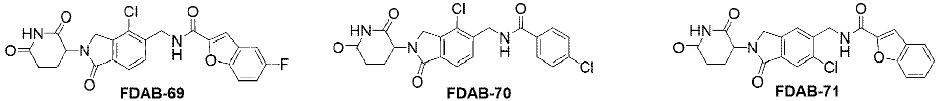

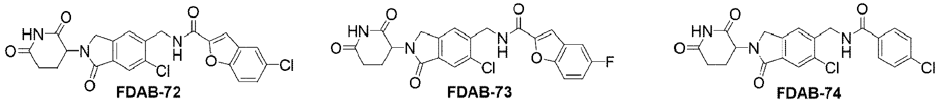

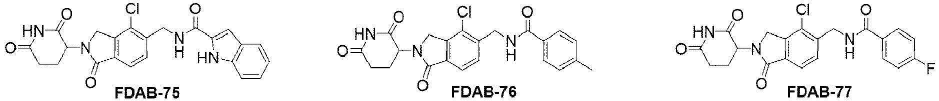

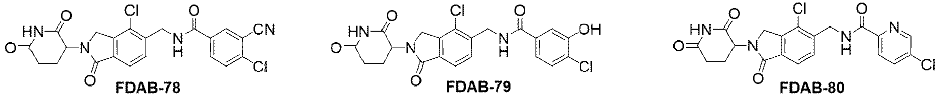

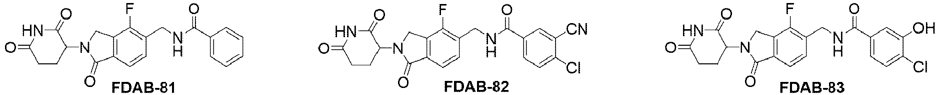

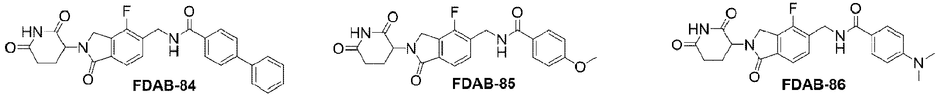

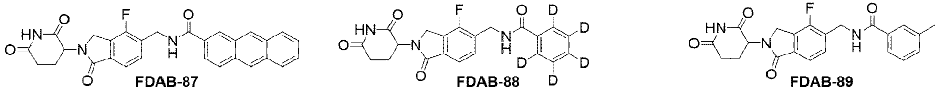

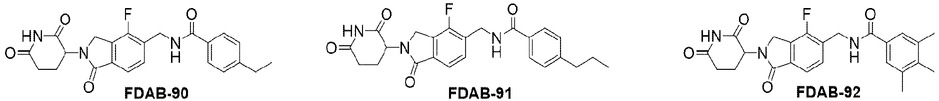

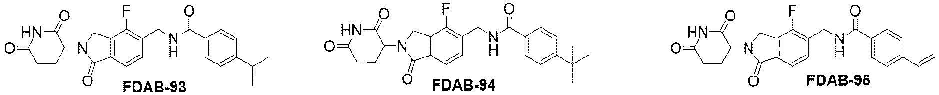

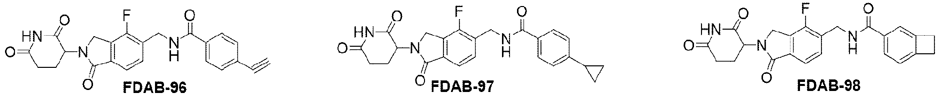

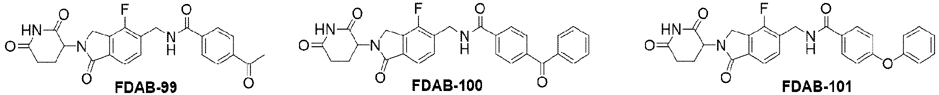

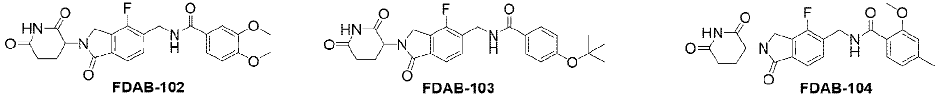

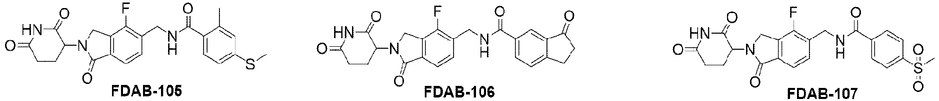

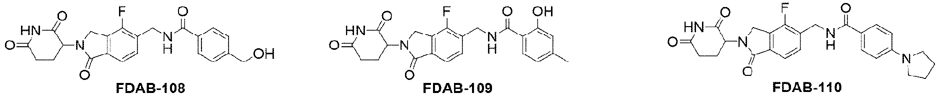

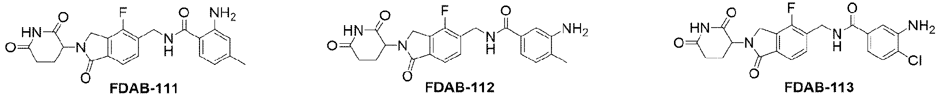

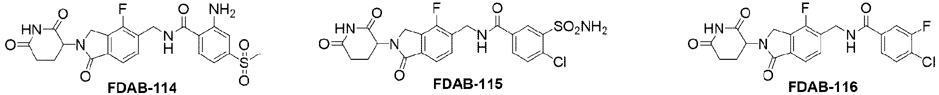

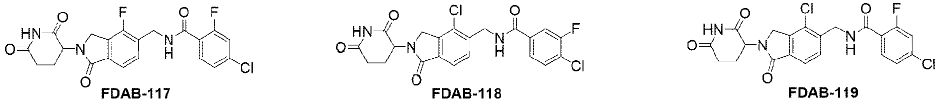

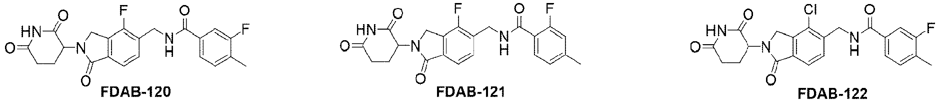

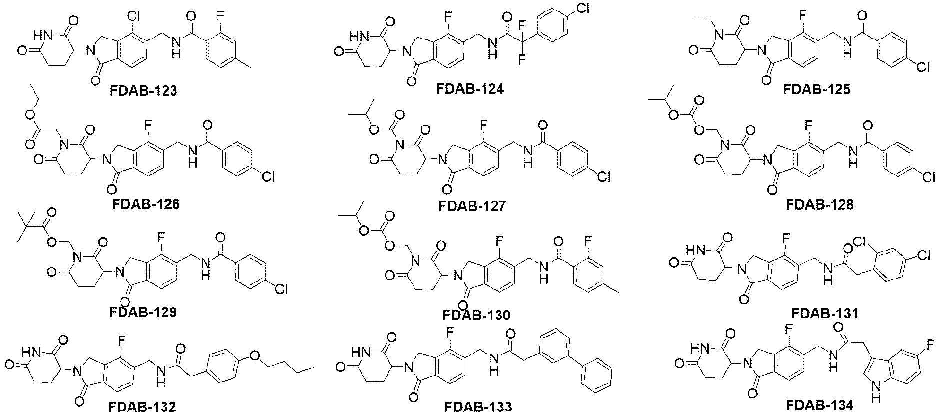

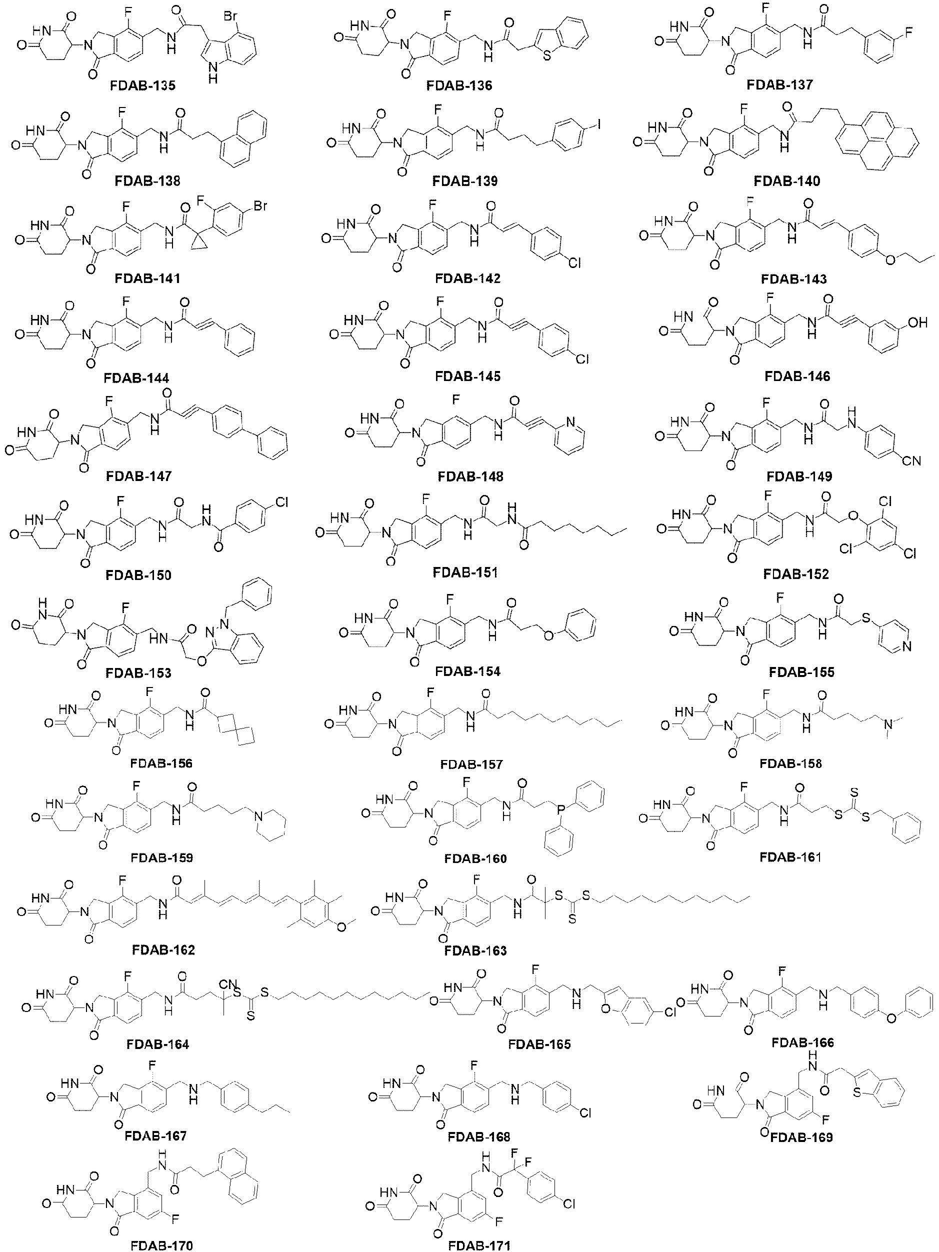

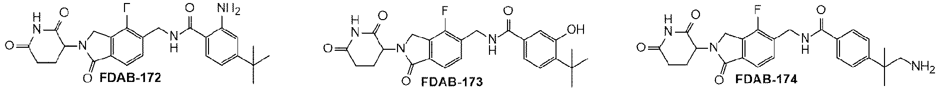

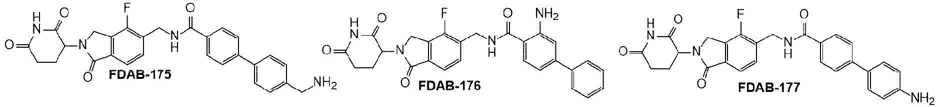

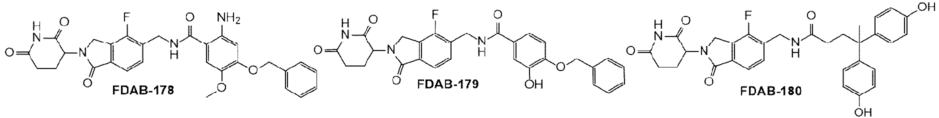

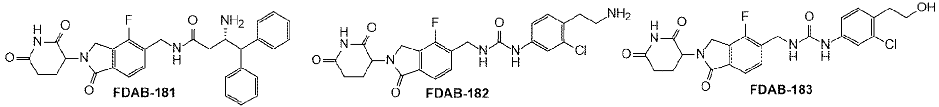

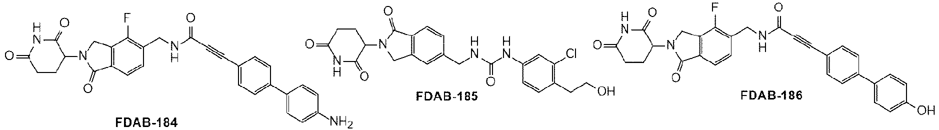

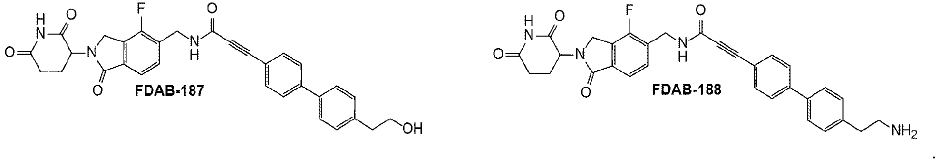

7. Use of the compound of formula I, or the tautomer, the stereoisomer, the hydrate, the solvate, or the pharmaceutically acceptable salt thereof according to any one of claims 1-6 in preparing a medicament for treating or preventing a disease, disorder, or condition associated with a mutation, improper expression, allosterism, and functional abnormality of a protein such as GSPT1, IKZF1, IKZF2, IKZF3, CK1α, N-MYC, or C-MYC.

8. The use according to claim 7, wherein the disease, disorder, or condition includes: myelodysplastic syndrome, multiple myeloma, mantle cell lymphoma, non-Hodgkin's lymphoma, papillary and follicular thyroid carcinoma, breast cancer, prostate cancer, chronic lymphocytic leukemia, amyloidosis, complex regional pain syndrome type I, malignant melanoma, radiculopathy, myelofibrosis, glioblastoma, gliosarcoma, malignant glioma, refractory plasmacytoma, chronic myelomonocytic leukemia, follicular lymphoma, ciliary body and chronic melanoma, iris melanoma, recurrent bilateral melanoma, extraocular extension melanoma, solid tumor, T-cell lymphoma, erythroid lymphoma, monoblastic and monocytic leukemia, myeloid leukemia, central nervous system lymphoma, brain tumor, meningioma, spinal cord tumor, thyroid cancer, non-small cell lung cancer, ovarian cancer, skin cancer, renal cell carcinoma, Burkitt's lymphoma, Hodgkin's lymphoma, large cell lymphoma, diffuse large B-cell lymphoma, astrocytoma, hepatocellular carcinoma, primary macroglobulinemia, and viral infection.

9. A pharmaceutical composition, comprising the compound of formula I, or the tautomer, the stereoisomer, the hydrate, the solvate, or the pharmaceutically acceptable salt thereof according to any one of claims 1-6.

10. The pharmaceutical composition according to claim 9, further comprising an additional therapeutic agent in addition to the active ingredient of the compound of formula I, wherein the additional therapeutic agent is selected from at least one of PD-1 inhibitor, PD-L1 inhibitor, rituximab, trastuzumab, elotuzumab, urotuximab, daratumumab, atezolizumab, ibritumomab, alemtuzumab, brentuximab, cytarabine, azacitidine, anthracycline, prednisone, dexamethasone, melphalan, cladribine, fludarabine, mitoxantrone, etoposide, methotrexate, pemetrexed, topotecan, doxorubicin, cyclophosphamide, gemcitabine, dacarbazine, clarithromycin, vincristine, docetaxel, clofarabine injection, HDAC inhibitor, FLT3 inhibitor, IDH1/2 inhibitor, BCL-2 inhibitor, proteasome inhibitor, PI3K inhibitor, BTK inhibitor, palbociclib, erythrocyte growth hormone, eltrombopag, minocycline, and CAR-T;
preferably, the pharmaceutical composition further comprises azacitidine or dexamethasone in addition to the active ingredient of the compound of formula I;
preferably, the pharmaceutical composition is administered by oral, rectal, topical, buccal, parenteral, intramuscular, intradermal, intravenous, or transdermal administration.
